# EUROPEAN PATENT APPLICATION

(11) **EP 4 844 008 A1**
(43) Date of publication of application: **25.11.2026**
(21) Application number: 25751156.8
(22) Date of filing: 20.01.2025
(51) Int. Cl.: C07K 19/00, C07K 14/54, C12N 15/62, A61K 38/20, A61K 47/68, A61P 35/00, A61P 37/00, A61P 31/00

(54) **ACTIVITY-REGULATABLE IL-12 FUSION PROTEIN AND USE THEREOF**

(30) Priority: 07.02.2024 CN 202410176044
(71) Applicant: Shanghai Jiao Tong University, Shanghai 200240 (CN)
(72) Inventor: LU, Huili, Shanghai 200240 (CN); SONG, Luyao, Shanghai 200240 (CN); QI, Gen, Shanghai 200240 (CN)
(74) Representative: Dehns
(86) International application number: PCT/CN2025/073376
(87) International publication number: WO 2025/167554

(57) **Abstract**

Provided are an IL-12 fusion protein with an adjustable activity and use thereof. The fusion protein includes a first structural unit, a second structural unit, and a third structural unit. The first structural unit is selected from a first Fc fragment, or a first antibody or antigen-binding fragment thereof. The first antibody or antigen-binding fragment thereof has a binding activity to a tumor antigen or an immune checkpoint. The second structural unit is selected from a cleavable linker or a non-cleavable linker. The third structural unit includes an IL-12 cytokine or a functional fragment thereof. The second structural unit mediates steric hindrance of the first structural unit to mask an activity of the IL-12 cytokine in the third structural unit, reducing toxic and side effects of the IL-12 cytokine in vivo. Compared with wild-type IL-12, it has an advantage of high safety, and can be further fused with antibodies Fab, scFv, and VHH, an antigen-binding peptide or a recombinant protein, a polypeptide, etc., to obtain a multifunctional fusion protein that can be conditionally released and activated.

## Description

### FIELD

The present disclosure belongs to the technical field of pharmaceuticals, specifically relates to an IL-12 fusion protein with an adjustable activity and use thereof, and more specifically relates to a fusion protein containing an IL-12 cytokine, a nucleic acid molecule, an expression vector, a recombinant cell, a fusion protein complex, an immunotherapy cell, a pharmaceutical composition, and use thereof in the manufacture of a medicament for treating or preventing a cancer, an infectious disease, or an autoimmune disease.

### BACKGROUND

Interleukin-12 (IL-12), which is a pleiotropic cytokine encoded by two independent genes, IL-12A (p35) and IL-12B (p40), forms an active p70 heterodimer bridged by two disulfide bonds. Activated dendritic cells (DCs), macrophages, and neutrophils locally produce IL-12 during T cell priming and directly act on cytotoxic immune effector cells including natural killer (NK) cells, natural killer T (NKT) cells, and CD8+T cells, to stimulate their proliferation and enhance their cytotoxic functions. IL-12 further induces the differentiation of naive T cells into the Th1 phenotype, leading to the production of cytokines (primarily IFNγ). IFNγ, in turn, exhibits cytostatic and cytotoxic activities, possesses anti-angiogenic properties, and can upregulate the expression of MHC class I and II molecules on tumor cells, thereby enhancing recognition and lysis thereof. Therefore, in clinical applications, even low concentrations of IL-12 still exhibit substantially potent stimulatory activity on immune cells in normal tissues, which may readily give rise to severe systemic toxic side effects, thus resulting in a relatively narrow therapeutic window. In addition, IL-12 has a serum half-life with rapid clearance, requiring frequent administration to maintain therapeutically relevant exposure levels.

Antibody-cytokine fusion proteins, also known as immunocytokines, can effectively enrich cytokines at tumor sites using the targeting ability of antibodies, thereby reducing the administration dose and minimizing drug toxic side effects. In previous studies, bifunctional antibody-cytokine fusion proteins have been constructed by fusing IL-12 with monoclonal antibodies targeting tumor-associated antigens or tumor necrosis. IL-12 can stimulate the proliferation of CD8+ T cells and NK cells to exert anti-tumor effects. The antibody can act as a "missile" carrying IL-12, achieving the enrichment of IL-12 at tumor sites. However, due to the significant biological activity of IL-12, even a small off-target effect can lead to a severe toxic side effect. Existing immune cytokines still fail to completely solve the problem of IL-12 systemic toxicity. Therefore, there is an urgent need to develop a drug product that can target delivery and release IL-12 to target tissues in vivo.

### SUMMARY

The present disclosure aims to solve at least one of the technical problems in the related art to a certain extent. To this end, the present disclosure provides a fusion protein containing an IL-12 cytokine.

In a first aspect of the present disclosure, the present disclosure provides a fusion protein. According to embodiments of the present disclosure, the fusion protein includes a first structural unit, a second structural unit, and a third structural unit. The first structural unit is selected from a first Fc fragment, or a first antibody antigen-binding fragment thereof. The first antibody or antigen-binding fragment thereof has a binding activity to a tumor antigen or an immune checkpoint. The second structural unit is selected from a cleavable linker or a non-cleavable linker. The cleavable linker is capable of being cleaved by a protease expressed in a tumor tissue. The third structural unit includes an IL-12 cytokine or functional fragment thereof. The first structural unit is linked to one or more third structural units, the third structural unit being linked to the C-terminus of the first structural unit via the second structural unit. The third structural unit of the fusion protein of the present disclosure contains the IL-12 cytokine or functional fragment thereof. The second structural unit mediates steric hindrance of the first structural unit, thereby masking an activity of the IL-12 cytokine in the third structural unit, in particular, without requiring a conventional affinity masking strategy. Therefore, the fusion protein can reduce toxic and side effects induced by the IL-12 cytokine without incorporating any antibody or receptor of the IL-12 cytokine and thus exhibiting the advantages of enhanced safety and structural simplicity.

In a second aspect of the present disclosure, the present disclosure provides a nucleic acid molecule. According to the embodiments of the present disclosure, the nucleic acid molecule encodes the fusion protein described in the first aspect. The nucleic acid molecules of the present disclosure can encode the fusion protein described in the first aspect.

In a third aspect of the present disclosure, the present disclosure provides an expression vector. According to the embodiments of the present disclosure, the expression vector carries the nucleic acid molecule described in the second aspect. In this way, the expression vector of the present disclosure can effectively achieve the expression of the fusion protein described in the first aspect, thereby obtaining a large amount of the fusion protein *in vitro.*

In a fourth aspect of the present disclosure, the present disclosure provides a recombinant cell. According to the embodiments of the present disclosure, the recombinant cell carries the nucleic acid described in the second aspect or the expression vector described in the third aspect, or expresses the fusion protein described in the first aspect. Under suitable conditions, the recombinant cell can be used to effectively express the fusion protein described in the first aspect.

In a fifth aspect of the present disclosure, the present disclosure provides a fusion protein complex. According to the embodiments of the present disclosure, the fusion protein complex includes the fusion protein described in the first aspect. As can be seen above, the third structural unit of the fusion protein described in the first aspect contains the IL-12 cytokine. The second structural unit mediates steric hindrance of the first structural unit, thereby masking an activity of the IL-12 cytokine in the third structural unit, in particular, without requiring a conventional affinity masking strategy. Therefore, the fusion protein can reduce toxic and side effects induced by the IL-12 cytokine without incorporating any antibody or receptor of the IL-12 cytokine, thus exhibiting the advantages of enhanced safety. In this way, the fusion protein complex of the present disclosure has advantages of enhanced safety and can be used for treating cancer, an infectious disease, or an autoimmune disease.

In a sixth aspect of the present disclosure, the present disclosure provides an immunotherapy cell. According to the embodiments of the present disclosure, the immunotherapy cell expresses the fusion protein described in the first aspect. As can be seen above, the third structural unit of the fusion protein described in the first aspect contains the IL-12 cytokine. The second structural unit mediates steric hindrance of the first structural unit, thus masking an activity of the IL-12 cytokine in the third structural unit, in particular, without requiring a conventional affinity masking strategy. Therefore, the fusion protein can reduce toxic and side effects induced by the IL-12 cytokine without incorporating any antibody or receptor of the IL-12 cytokine, thus exhibiting the advantages of enhanced safety. In this way, the immunotherapy cell containing the fusion protein described above has advantages of enhanced safety and can be used for treating cancer, an infectious disease, or an autoimmune disease.

In a seventh aspect of the present disclosure, the present disclosure provides a pharmaceutical composition. According to the embodiments of the present disclosure, the pharmaceutical composition includes the fusion protein described in the first aspect, the nucleic acid molecule described in the second aspect, the expression vector described in the third aspect, the recombinant cell described in the fourth aspect, the fusion protein complex described in the fifth aspect, or the immunotherapy cell described in the sixth aspect. As can be seen above, the third structural unit of the fusion protein described in the first aspect contains the IL-12 cytokine. The second structural unit mediates steric hindrance of the first structural unit, thus masking an activity of the IL-12 cytokine in the third structural unit, in particular without requiring a conventional affinity masking strategy. Therefore, the fusion protein can reduce toxic and side effects induced by the IL-12 cytokine without incorporating any antibody or receptor of the IL-12 cytokine, thus exhibiting the advantages of enhanced safety. In this way, the pharmaceutical composition containing the fusion protein described above has advantages of enhanced safety and can be used for treating cancer, an infectious disease, or an autoimmune disease.

In an eighth aspect of the present disclosure, the present disclosure provides use of the fusion protein described in the first aspect, the fusion protein complex described in the fifth aspect, the immunotherapy cell described in the sixth aspect, or the pharmaceutical composition described in the seventh aspect in the manufacture of a medicament for treating cancer, an infectious disease, or an autoimmune disease.

Additional aspects and advantages of the present disclosure will be provided in part in the following description or will become apparent in part from the following description, or can be learned from practicing of the present disclosure.

### BRIEF DESCRIPTION OF THE DRAWINGS

The above and/or additional aspects and advantages of the present disclosure will become more apparent and more understandable from the following description of embodiments taken in conjunction with the accompanying drawings.

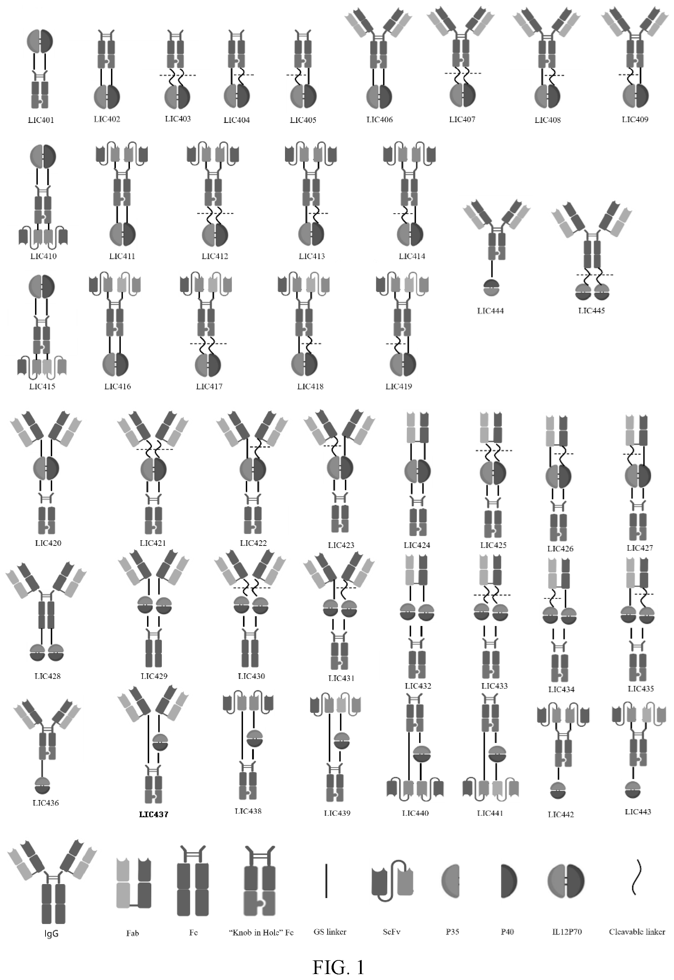

FIG. 1 is a schematic structural diagram of a fusion protein of the present disclosure, in which: LIC401 represents C-non-cleavable L-Fc where only Fc serves as the first structural unit; LIC402 represents Fc-non-cleavable L-C where only Fc serves as the first structural unit; LIC403 represents Fc-double-cleavable L-C where only Fc serves as the first structural unit; LIC404 and LIC405 each represent Fc-single-cleavable L-C where only Fc serves as the first structural unit; LIC406 represents IgG-non-cleavable L-C where the monoclonal antibody serves as the first structural unit; LIC407 represents IgG-double-cleavable L-C where the monoclonal antibody serves as the first structural unit; LIC408 and LIC409 each represent IgG-single-cleavable L-C where the monoclonal antibody serves as the first structural unit; LIC410 represents C-non-cleavable L-Fc-ScFv where two identical ScFv-Fcs serve as the first structural unit; LIC411 represents ScFv-Fc-non-cleavable L-C where two identical ScFv-Fcs serve as the first structural unit; LIC412 represents ScFv-Fc-double-cleavable L-C where two identical ScFv-Fcs serve as the first structural unit; LIC413, LIC414 each represent ScFv-Fc-single-cleavable L-C where two identical ScFv-Fcs serve as the first structural unit; LIC415 represents C-non-cleavable L-Fc-ScFv where two different ScFv-Fcs serve as the first structural unit; LIC416 represents ScFv-Fc-non-cleavable L-C where two different ScFv-Fcs serve as the first structural unit; LIC417 represents ScFv-Fc-double-cleavable L-C where two different ScFv-Fcs serve as the first structural unit; LIC418, LIC419 each represent ScFv-Fc-single-cleavable L-C where two different ScFv-Fcs serves as the first structural unit; LIC420 represents (Fab)2-non-cleavable L-C-L-Fc where two Fabs serve as the first structural unit and Fc serves as the fourth structural unit; LIC421 represents (Fab)2-double-cleavable L-C-L-Fc where two Fabs serve as the first structural unit and Fc serves as the fourth structural unit; LIC422, LIC423 each represent (Fab)2-single-cleavable L-C-L-Fc where two Fabs serve as the first structural unit and Fc serves as the fourth structural unit; LIC424 represents Fab-non-cleavable L-C-L-Fc where a single Fab serves as the first structural unit and Fc serves as the fourth structural unit; LIC425 represents Fab-double-cleavable L-C-L-Fc where a single Fab serves as the first structural unit and Fc serves as the fourth structural unit; LIC426 and LIC427 each represent Fab-single-cleavable L-C-L-Fc where a single Fab serves as the first structural unit and Fc serves as the fourth structural unit; LIC428 represents IgG-non-cleavable L-C2 where the monoclonal antibody serves as the first structural unit and two cytokine molecules are contained; LIC429 represents (Fab)2-non-cleavable L-C2-L-Fc where two Fabs serve as the first structural unit, Fc serves as the fourth structural unit, and two cytokine molecules are contained; LIC430 represent (Fab)2-double-cleavable L-C2-L-Fc where two Fabs serve as the first structural unit, Fc serves as the fourth structural unit, and two cytokine molecules are contained; LIC431 represents (Fab)2-single-cleavable L-C2-L-Fc where two Fabs serve as the first structural unit, Fc serves as the fourth structural unit, and two cytokine molecules are contained; LIC432 represents Fab-non-cleavable L-C2-L-Fc where a single Fab serves as the first structural unit, Fc serves as the fourth structural unit, and two cytokine molecules are contained; LIC433 represents Fab-double-cleavable L-C2-L-Fc where a single Fab serves as the first structural unit, Fc serves as the fourth structural unit, and two cytokine molecules are contained; LIC434 and LIC435 each represent Fab-single-cleavable L-C2-L-Fc where a single Fab serves as the first structural unit, Fc serves as the fourth structural unit, and two cytokine molecules are contained; LIC436 represents IgG-non-cleavable L-C where the monoclonal antibody serves as the first structural unit and subunits of the cytokine are tandemly linked thereto; LIC437 represents (Fab)2-non-cleavable L-C where two Fabs serve as the first structural unit, Fc serves as the fourth structural unit, and subunits of the cytokine are tandemly linked thereto; LIC438 represents ScFv-non-cleavable L-C-L-Fc where two identical ScFvs serve as the first structural unit, Fc serves as the fourth structural unit, and subunits of the cytokine are tandemly linked thereto; LIC439 represents ScFv-non-cleavable L-C-L-Fc where two different ScFvs serve as the first structural unit, Fc serves as the fourth structural unit, and subunits of the cytokine are tandemly linked thereto; LIC440 represents Fc-non-cleavable L-C-L-ScFv where two identical ScFvs serve as the first structural unit, Fc serves as the fourth structural unit, and subunits of the cytokine are tandemly linked thereto; LIC441 represents Fc-non-cleavable L-C-L-ScFv where two different ScFvs serve as the first structural unit, Fc serves as the fourth structural unit, and subunits of the cytokine are tandemly linked thereto; LIC442 represents ScFv-Fc-non-cleavable L-C where two identical ScFv-Fcs serve as the first structural unit and subunits of the cytokine are tandemly linked thereto; LIC443 represents ScFv-Fc-non-cleavable L-C where two different ScFv-Fcs serve as the first structural unit and subunits of the cytokine are tandemly linked thereto; LIC444 represents IgG- non-cleavable L-C where the monoclonal antibody serve as the first structural unit and subunits of the cytokine are tandemly linked thereto in an order different from that of LIC436; and LIC445 represents IgG-double-cleavable L-C2 where the monoclonal antibody serve as the first structural unit and two cytokine molecules are contained. Wherein: single-cleavable L represents that only one chain in a fusion protein contains a cleavable linker; double-cleavable L represents that two chains in a fusion protein contains a cleavable linker; C represents that one molecule of the cytokine is contained in a fusion protein, C2 represents that two molecules of the cytokine are contained in a fusion protein; and L represents a non-cleavable linker at the C-terminus of the IL12 subunit.
FIG. 2 is a diagram showing protein A affinity chromatography purification of the LIC401 fusion protein.
FIG. 3 is an SDS-PAGE electrophoretogram of the purified LIC401 fusion protein, in which: NR represents a non-reduced sample, R represents a reduced sample, the mother liquid represents a collected cell supernatant, NaOH represents impurities eluted with a NaOH solution after chromatography, the flow-through represents the flow-through liquid during protein A affinity chromatography, and eluate represents the LIC401 fusion protein.
FIG. 4 shows Western blot identification of the LIC401 fusion protein, in which plasmid was transfected at a ratio of 1:1, and R represents a reduced sample.
FIG. 5 is a diagram showing protein A affinity chromatography purification of the LIC402 fusion protein.
FIG. 6 shows Western blot identification of the LIC402 fusion protein, in which plasmid was transfected at a ratio of 1:1, and R represents a reduced sample.
FIG. 7 is a photograph showing stimulatory effects of LIC401 and LIC402 fusion proteins on spleens of mice.
FIG. 8 is a statistical diagram of spleen weights of mice treated with PBS, the LIC401 fusion protein, and the LIC402 fusion protein.
FIG. 9 is a diagram showing protein A affinity chromatography purification of the LIC403 fusion protein.
FIG. 10 is an SDS-PAGE electrophoretogram of the purified LIC403 fusion protein, in which: NR represents a non-reduced sample, R represents a reduced sample, the flow-through represents the flow-through liquid during protein A affinity chromatography, and eluate represents the LIC403 fusion protein.
FIG. 11 is a diagram showing detection of an *in vitro* activity of the purified LIC403 fusion protein, detection of *in vivo* activities of the LIC401, LIC402, LIC402-1, and LIC403 fusion proteins, and body weight changes of mice after administration of the LIC401, LIC402, LIC402-1, and LIC403 fusion proteins.
FIG. 12 is a diagram showing protein A affinity chromatography purification of the LIC404 fusion protein.
FIG. 13 is an SDS-PAGE electrophoretogram of the purified LIC404 fusion protein, in which: NR represents a non-reduced sample, R represents a reduced sample, the flow-through represents the flow-through liquid during protein A affinity chromatography, and eluate represents an LIC404 fusion protein.
FIG. 14 is a diagram showing protein A affinity chromatography purification of the LIC405 fusion protein.
FIG. 15 is a diagram showing the detection results of *in vitro* activities of the LIC404, LIC405, and control fusion proteins.
FIG. 16 is a diagram showing protein A affinity chromatography purification of the LIC407 fusion protein.
FIG. 17 is a diagram showing ELISA assay of affinity of the LIC407 fusion protein to human PDL1 antigen.
FIG. 18 is a diagram showing detection of *in vitro* activities of the LIC407, LIC408, and LIC409 fusion proteins, detection of *in vivo* activities of the LIC406 to LIC409 fusion proteins, and body weight changes of mice after administration of the LIC406 to LIC409 fusion proteins.
FIG. 19 is a diagram showing protein A affinity chromatography purification of the LIC412 fusion protein.
FIG. 20 is a diagram showing detection of *in vitro* activities of the LIC412 and control fusion proteins.
FIG. 21 is a diagram showing protein A affinity chromatography purification of the LIC417 fusion protein.
FIG. 22 is a diagram showing detection of *in vitro* activities of the LIC417 and control fusion proteins.
FIG. 23 is a diagram showing protein A affinity chromatography purification of the LIC421 fusion protein.
FIG. 24 is a diagram showing detection of an *in vitro* activity of the purified LIC421 fusion protein.
FIG. 25 is a diagram showing detection of *in vitro* activities of the single-cleavable LIC422 and LIC423 fusion proteins.
FIG. 26 is a diagram showing protein A affinity chromatography purification of the LIC425 fusion protein.
FIG. 27 is a schematic SDS-PAGE diagram after *in vitro* enzymatic cleavage of the LIC425 fusion protein.
FIG. 28 is a diagram showing detection of an *in vitro* activity of the LIC425 fusion protein.
FIG. 29 is a diagram showing ELISA assay of affinity of the LIC425 fusion protein to human PDL1 antigen.
FIG. 30 is a diagram showing ELISA assay of distribution of the LIC425 fusion protein in various tissues of tumor-bearing mice.
FIG. 31 is a diagram showing protein A affinity chromatography purification of the LIC425-1 fusion protein.
FIG. 32 is a diagram showing body weight changes of mice after administration of the LIC425-1 and control fusion proteins.
FIG. 33 is a statistical diagram showing spleen weights of mice at the endpoint after administration of the LIC425-1 and control fusion proteins.
FIG. 34 is a diagram showing results of CD8+ T cell and NK cell counts in the peripheral blood at the endpoint after administration of the LIC425-1 and control fusion proteins.
FIG. 35 is a schematic diagram showing a proportion of CD8+ T cells in the spleen at the endpoint after administration of the LIC425-1 and control fusion proteins.
FIG. 36 is a diagram showing detection of *in vitro* activities of the single-cleavable LIC426 and LIC427 fusion proteins.
FIG. 37 is a diagram showing protein A affinity chromatography purification of the LIC430 fusion protein.
FIG. 38 is a diagram showing the detection results of an activity of the LIC430 fusion protein in promoting IFN-γ secretion by mouse splenocytes, before and after cleavage by the uPA enzyme.
FIG. 39 is a diagram showing protein A affinity chromatography purification of the LIC433 fusion protein.
FIG. 40 is a diagram showing detection of *in vitro* activities of the LIC433 and control fusion proteins.
FIG. 41 is a diagram showing protein A affinity chromatography purification of the LIC436 fusion protein.
FIG. 42 is a diagram showing protein A affinity chromatography purification of the LIC444 fusion protein.
FIG. 43 is a diagram showing the detection results of activities of the LIC436 and LIC444 fusion proteins in promoting IFN-γ secretion by mouse spleen splenocytes, before and after cleavage by the uPA enzyme.
FIG. 44 is a diagram showing the results of *in vitro* stimulation of IFN-γ secretion by the LIC438 and LIC441 fusion proteins.
FIG. 45 is a diagram showing protein A affinity chromatography purification of the humanized IL12-form LIC445 fusion protein.
FIG. 46 is a diagram of the results of *in vitro* stimulation of IFN-γ secretion from human PBMCs by the LIC445 fusion protein.
FIG. 47 is a diagram showing the comparison results of masking effects of the fusion proteins with different structures in Example 37 of the present disclosure.
FIG. 48 is a diagram showing body weight changes of mice after administration of the fusion proteins with different structures in Example 38 of the present disclosure.

### DETAILED DESCRIPTION

Embodiments of the present disclosure will be described in detail below. The embodiments described below are exemplary and are only used to explain the present disclosure, and they should not be construed as limiting the present disclosure.

It should be noted that the terms "first" and "second" are only used for descriptive purposes, rather than indicating or implying relative importance or implicitly indicating the number of indicated technical features. Therefore, the features defined with "first" and "second" may include one or more of these features explicitly or implicitly. Further, in the description of the present disclosure, unless otherwise specified, "plurality of" means two or more.

### Detailed Description of the Present Disclosure

### Definitions and General Terms

It should be understood that, in the present disclosure, endpoints and any value of the ranges shall not be limited to the exact range or value, and those ranges or values should be understood to include values close to those ranges or values. For numerical ranges, endpoints of respective ranges, an endpoint and individual point value of respective ranges, and individual point values can be combined with each other to obtain one or more new numerical ranges, which should be deemed to be specifically disclosed herein. If specific technologies or conditions are not specified in the examples, they shall be carried out according to the technologies or conditions as described in the literature in the art or according to the product instructions. The reagents and instruments used without indicating the manufacturer are all conventional products that can be purchased commercially.

It should be noted that, unless otherwise indicated, scientific and technical terms used herein have the meanings commonly understood by those skilled in the art. In addition, the laboratory operating procedures for cell culture, molecular genetics, nucleic acid chemistry, and immunology used herein are conventional procedures widely used in the corresponding fields.

As used herein, the term "comprise", "include", "including" or "comprising" is an open expression, that is, including the contents indicated by the present disclosure, but not excluding other aspects.

As used herein, the terms "optionally", "optional" or "option" usually mean that the event or situation described subsequently may happen but not necessarily happen, and the description includes the situation in which the event or situation happens and the situation in which the event or situation does not happen.

As used herein, the term "fragment" refers to a target protein or polypeptide, and a target protein or polypeptide with N-terminal end (N-terminus) or C-terminal end (C-terminus) truncation and/or internal deletion.

To make the present disclosure more easily understood, certain technical and scientific terms are specifically defined below. Unless otherwise clearly defined, all other technical and scientific terms used herein have the meanings commonly understood by those skilled in the art to which the present disclosure belongs. The abbreviations for amino acid residues are standard 3-letter and/or 1-letter codes used in the art to refer to one of the 20 commonly used L-amino acids.

As used herein, the term "fusion protein" refers to a new type of protein formed by fusing at least two proteins or polypeptides (e.g., biologically active polypeptides and effector molecules). The above-described fusion operation can usually be performed by genetic engineering, chemical methods or other suitable technologies, for example, the expression product of two genes recombined by DNA recombination technology. If necessary, fusion molecules can be fused at one or several sites via amino acid linkers. The fusion protein can exist in the form of a monomer or polymer (e.g., dimer). As used herein, it means that the first structural unit, the second structural unit, and the third structural unit including the IL-12 cytokine or functional fragment thereof are in a fused state.

The fusion protein described in the present disclosure is generally prepared by biosynthesis methods. According to the nucleotide sequences in the present disclosure, the encoding nucleic acid according to the present disclosure may be conveniently prepared by those skilled in the art using various known methods. For example, these methods include, but not limited to: polymerase chain reaction (PCR), DNA artificial synthesis, etc. Specific methods may refer to J. Sambrook, Molecular Cloning: Laboratory Manual. As an implementation of the present disclosure, the encoding nucleic acid sequence may be constructed by a method for synthesizing nucleotide sequence fragments followed by overlap extension PCR.

In the present disclosure, the "antibody" refers to an immunoglobulin molecule typically composed of two pairs of polypeptide chains, each pair consisting of a light chain (L chain) and a heavy chain (H chain). In a general sense, the heavy chain can be understood as the polypeptide chain with a larger molecular weight in an antibody, while a light chain refers to the polypeptide chain with a smaller molecular weight in an antibody. Each heavy chain is composed of a heavy chain variable region (VH) and a heavy chain constant region (CH), and the heavy chain constant region consists of three domains (CH-1, CH-2, and CH-3). Each light chain is composed of a light chain variable region (VL) and a light chain constant region (CL), and the light chain constant region consists of one domain CL. The VH and VL regions can be further subdivided into hypervariable regions (referred to as complementary determining regions (CDRs)), and the variable regions (VH and VL) of heavy chain/light chain pairs form antibody binding sites. The term "antibody" is not limited by any specific method for preparing antibodies. For example, the antibody includes, in particular, recombinant antibodies, monoclonal antibodies, and polyclonal antibodies. Antibodies may be antibodies of different types, such as IgG or a mutant thereof, IgA1, IgA2, IgD, IgE, or IgM antibody. In some embodiments of the present disclosure, the antibody that binds to a tumor antigen or an immune checkpoint is a full-length immunoglobulin, including IgG1, IgG2, IgG3, and IgG4.

In the present disclosure, the "antigen-binding fragment of the antibody" refers to a fragment including a portion or all of a full-length antibody. It retains the ability to specifically bind to the same antigen that the full-length antibody binds to, and/or competes with the full-length antibody for specific binding to the antigen. It is also referred to as an antigen-binding portion. For example, this fragment may include a portion or all of the CDRs of the antibody. Such fragments are biologically active because they bind to the antigen and can compete with other antigen-binding molecules (including intact antibodies) for binding to a given epitope. Antigen-binding fragments of antibodies can be prepared by recombinant DNA technology or by enzymatic or chemical cleavage of intact antibodies. In some embodiments of the present disclosure, the antigen-binding fragment of the antibody is Fab, Fab', (Fab')₂, Fv, scFv, or VHH, or a combination thereof. In the present disclosure, "Fab" is a fragment obtained by treating an antibody molecule with papain (which cleaves the amino acid residue at position 224 of the H chain). Approximately half of the N-terminus side of the H chain is linked to the entire L chain via a disulfide bond. In the present disclosure, "F(ab')₂" is an antibody fragment obtained by treating the portion below the two disulfide bonds in the hinge region of an antibody molecule with pepsin. It has a molecular weight of approximately 100,000 Da and contains two Fab regions linked at the hinge position. In the present disclosure, "Fab'" is an antibody fragment obtained by cleaving the disulfide bonds in the hinge region of the above-described F(ab')₂. Fab' can be prepared by treating F(ab')₂, which specifically recognizes and binds to an antigen, with a reducing agent such as dithiothreitol. In the present disclosure, "Fv" refers to the smallest functional fragment of an antibody molecule that retains the antigen-binding site and consists of a light chain variable region and a heavy chain variable region, which are linked together via non-covalent bonds. In the present disclosure, "scFv" refers to an antibody fragment that includes the heavy chain variable region (VH) and light chain variable region (VL) of an antibody. The size of scFv is generally 1/6 that of a complete antibody, and it is preferably an amino acid chain sequence encoded by a single nucleotide chain. In the present disclosure, "VHH" refers to a single variable region of a heavy chain antibody and possesses antigen-binding capacity.

As used herein, the terms "full-length antibody", "full-length mAb", or "full-length monoclonal antibody" are all composed of at least two identical light chains and at least two identical heavy chains linked by interchain disulfide bonds, such as immunoglobulin G (IgG), immunoglobulin A (IgA), immunoglobulin M (IgM), immunoglobulin D (IgD), or immunoglobulin E (IgE).

As used herein, the term "expression vector" generally refers to a nucleic acid molecule that can be inserted into a suitable host for self-replication and transfer the inserted nucleic acid molecule into a host cell and/or between host cells. The nucleic acid molecule includes a nucleotide sequence capable of expressing a target protein. The expression vector may include a vector primarily used to insert DNA or RNA into cells, a vector primarily used to replicate DNA or RNA, and a vector primarily used to express, i.e., transcribe and/or translate, DNA or RNA. The expression vector also includes vectors with multiple functions as described above. The expression vector may be a polynucleotide capable of being transcribed and translated into a polypeptide when introduced into a suitable host cell. Generally, the expression vector can produce the desired expression product by culturing an appropriate host cell containing the expression vector.

As used herein, the term "recombinant cell" generally refers to a cell with stably inherited unique traits that are obtained by modifying, engineering, or recombining the genetic material of a recipient cell (also referred to as a host cell) using genetic engineering technology or cell fusion technology. The term "host cell" refers to a prokaryotic cell or a eukaryotic cell, into which a recombinant expression vector can be introduced. As used herein, the term "transformed" or "transfected" refers to the introduction of a nucleic acid (such as an expression vector) into a cell by various techniques known in the art. Suitable host cells may be transformed or transfected with the DNA sequence of the present disclosure and may be used for the expression and/or secretion of target proteins. Examples of suitable host cells that can be used in the present disclosure include immortalized hybridoma cells, NS/0 myeloma cells, 293 cells, Chinese hamster ovary (CHO) cells, HeLa cells, Cap cells (cells derived from human amniotic fluid), and CoS cells.

As used herein, the term "pharmaceutical composition" generally refers to a medicament in unit dosage form and may be prepared by any method well known in the pharmaceutical field. All methods include the step of combining the active ingredient with a carrier, which constitutes one or more auxiliary ingredients. Generally, the composition is prepared by uniformly and sufficiently combining the active fusion protein with a liquid carrier, a finely divided solid carrier, or both.

As used herein, the term "pharmaceutically acceptable excipient" may include any solvent, solid excipient, diluent, or other liquid excipient suitable for a particular intended dosage form. Except to the extent that any conventional excipients are incompatible with the fusion protein of the present disclosure, for example, by producing any undesirable biological effects or otherwise interacting in a deleterious manner with any other components of the pharmaceutically acceptable composition, their uses are also contemplated by the present disclosure.

As used herein, the term "administering" or "administration" refers to introducing a predetermined amount of substance into a patient by a certain suitable route. The fusion protein or the pharmaceutical composition of the present disclosure may be administered by any common route, provided that it can reach the intended tissue. Various routes of administration are contemplated, including intraperitoneal administration, intravenous injection, intramuscular injection, subcutaneous injection, etc., although the present disclosure is not limited to these exemplary administration routes. Preferably, the composition of the present disclosure is administered by intravenous injection or subcutaneous injection.

As used herein, the term "treatment" or "treat" refers to obtaining a desired pharmacological and/or physiological effect. The effect may be prophylactic in terms of completely or partially preventing a disease or its symptoms, and/or therapeutic in terms of partially or completely curing a disease and/or adverse effects caused by the disease. As used herein, the term "treatment" or "treat" encompasses treatment of diseases in mammals, especially humans, including: (a) preventing the occurrence of diseases or disorders in individuals who are susceptible to diseases but have not been diagnosed with the diseases; (b) inhibiting diseases, for example, retarding the progression of the diseases; or (c) alleviating diseases, such as alleviating the symptoms associated with the diseases. As used herein, the term "treatment" or "treat" includes treating, curing, alleviating, ameliorating, mitigating or inhibiting a disease in an individual by administrating the fusion protein or the pharmaceutical composition to the individual, including, but not limited to, administering a medicament containing the fusion protein described herein to an individual in need thereof.

### Detailed Description of Cytokine Fusion Protein and Use thereof in the Present Disclosure

The present disclosure provides a fusion protein containing an IL-12 cytokine, a nucleic acid molecule, an expression vector, a recombinant cell, a fusion protein complex, an immunotherapy cell, a pharmaceutical composition, and use thereof in the manufacture of a medicament for treating or preventing cancer, an infectious disease, or an autoimmune disease, which will be described in detail below.

### Fusion Protein

In a first aspect of the present disclosure, the present disclosure provides a fusion protein. According to embodiments of the present disclosure, the fusion protein includes a first structural unit, a second structural unit, and a third structural unit. The first structural unit is selected from a first Fc fragment, or a first antibody or antigen-binding fragment thereof. The first antibody or antigen-binding fragment thereof has a binding activity to a tumor antigen or an immune checkpoint. The second structural unit is selected from a cleavable linker or a non-cleavable linker. The cleavable linker is capable of being cleaved by a protease expressed in a tumor tissue. The third structural unit includes an IL-12 cytokine or functional fragment thereof. The first structural unit is linked to one or more third structural units, the third structural unit being linked to the C-terminus of the first structural unit via the second structural unit. The third structural unit of the fusion protein of the present disclosure contains the IL-12 cytokine. The second structural unit mediates steric hindrance of the first structural unit, thereby masking an activity of the IL-12 cytokine in the third structural unit, in particular, without requiring a conventional affinity masking strategy. Therefore, the fusion protein can reduce toxic and side effects induced by the IL-12 cytokine without incorporating any antibody or receptor of the IL-12 cytokine, thus exhibiting the advantages of enhanced safety.

Further, the inventors have found that the N-terminus of the third structural unit is affected by the steric hindrance of the first structural unit, such that its binding is restricted in a fused state, resulting in a relatively low biological activity. After cleavage of the cleavable linker in the second structural unit, the N-terminus or both the N-terminus and the C-terminus of the third structural unit is released, thereby restoring its affinity for the receptor and recovering its biological activity to a high level. As a result, the toxic and side effects on non-target tissues are reduced while the therapeutic effect on the target is enhanced.

As used herein, the term "X₁ is linked to the C-terminus of X₃ via X₂" means that the sequence from the N-terminus to the C-terminus is X₃-X₂-X₁. For example, "the third structural unit is linked to the C-terminus of the first structural unit via the second structural unit" means that the sequence from the N-terminus to the C-terminus is the first structural unit-the second structural unit-the third structural unit. The term "X₁ is linked to the N-terminus of X₃ via X₂" means that the sequence from the N-terminus to the C-terminus is X₁-X₂-X₃. For example, "the third structural unit is linked to the N-terminus of the first structural unit via the second structural unit" means that the sequence from the N-terminus to the C-terminus is the third structural unit-the second structural unit-the first structural unit.

In some embodiments of the present disclosure, "IL-12" refers to interleukin 12, including any natural (wild-type) IL-12, functional fragments or mutants thereof, preferably mammalian interleukin 12. In some embodiments, mammalian interleukin 12 includes, but is not limited to, interleukin 12 derived from human, mice/rats, pigs, rabbits, sheep/goats, monkeys, and cats. In some embodiments, the IL-12 cytokine is selected from a murine IL-12 cytokine or a human IL-12 cytokine. All structures of the present disclosure are applicable to the murine or human IL-12 cytokine, that is, the IL-12 cytokine is at least one of murine or human IL-12.

According to the embodiments of the present disclosure, when the first structural unit is linked to one third structural unit, the second structural unit is selected from a non-cleavable linker.

In some embodiments of the present disclosure, in the fusion protein of the present disclosure, the first structural unit is linked to the C-terminus of the third structural unit via the non-cleavable linker (the C-terminus of the cytokine) of the second structural unit. Structures of the fusion proteins are illustrated in LIC401, LIC410, and LIC415 in FIG. 1. From the N-terminus to the C-terminus, it sequentially includes: A chain: P35 subunit, non-cleavable linker (the C-terminus of the cytokine), and antibody Knob sequence; and B chain: P40 subunit, non-cleavable linker (the C-terminus of the cytokine), and antibody Hole sequence. The targeting of the two antibody chains may be the same or different. In some embodiments, the fusion of different subunits of the cytokine with Fc-Knob or Fc-Hole is interchangeable without altering their structure and function. For example, in LIC401, Fc-Knob is linked to the IL-12 p35 subunit via a flexible linker, and Fc-Hole is linked to the IL-12 p40 subunit via a flexible linker. If it is replaced such that Fc-Knob is linked to the IL-12 P40 subunit via a flexible linker and Fc-Hole is linked to the IL-12 P35 subunit via a flexible linker, the activity of IL-12 remains unchanged. The present disclosure has found that IL-12 located at the N-terminus of the fusion protein exhibits stronger activity and significantly stimulates spleen enlargement in mice when injected. In this way, LIC401 can be used as a positive control molecule.

In some embodiments of the present disclosure, the first structural unit of the fusion protein of the present disclosure is linked to the N-terminus of the third structural unit via a non-cleavable linker (the N-terminus of the cytokine) of the second structural unit. In some embodiments, structures of the fusion proteins are illustrated in LIC402, LIC406, LIC411, LIC416 in FIG. 1. The fusion protein, from the N-terminus to the C-terminus, sequentially includes: A chain: antibody Knob sequence, non-cleavable linker (the N-terminus of the cytokine), and P35 subunit; and B chain: antibody Hole sequence, non-cleavable linker (the N-terminus of the cytokine), and P40 subunit. The targets of the two antibody chains may be the same or different. In other embodiments, the structures of the fusion proteins are illustrated in LIC428, LIC436, LIC442, LIC443, and LIC444 in FIG. 1. The fusion protein, from N-terminus to C-terminus, sequentially includes antibody, non-cleavable linker (the N-terminus of the cytokine), and P35 subunit-P40 subunit of IL-12. The fusion order of the P35 subunit-P40 subunit can be the P35 subunit at the N-terminus and the P40 subunit at the C-terminus, or the P40 subunit at the N-terminus and the P35 subunit at the C-terminus. In addition, the non-cleavable linker and the P35 subunit-P40 subunit fusion protein can be symmetrically linked to the C-terminus of two Fc chains of the antibody, or to the C-terminus of one Fc chain. The present disclosure has found that, compared with the fusion protein with IL-12 located at the N-terminus, IL-12 located at the C-terminus of the fusion protein exhibits significantly lower activity. Further, heterodimeric IL-12 forms at the C-terminus (such as LIC406) exhibit weaker activity than homodimeric IL-12 forms (such as LIC428).

According to the embodiments of the present disclosure, when the first structural unit is linked to one third structural unit, the second structural unit is selected from a cleavable linker.

According to the embodiments of the present disclosure, when the first structural unit is linked to a plurality of third structural units, the second structural units linked to the plurality of third structural units contain at least one cleavable linker, and preferably, the second structural units linked to the plurality of third structural units are all the cleavable linkers.

According to the embodiments of the present disclosure, when the first structural unit is linked to a plurality of third structural units, the plurality of the second structural units are all the cleavable linkers.

In some embodiments of the present disclosure, the first structural unit of the fusion protein of the present disclosure is linked to the N-terminus of the third structural unit via a cleavable linker of the second structural unit. The structures of the fusion proteins are illustrated in LIC403, LIC407, LIC412, and LIC417 in FIG. 1. The fusion protein, from the N-terminus to the C-terminus, sequentially includes: A chain: antibody Knob sequence, cleavable linker, and P35 subunit; and B chain: antibody Hole sequence, cleavable linker, and P40 subunit. The targets of the two antibody chains may be the same or different. In other embodiments, the structures of the fusion proteins are illustrated in LIC445 in FIG. 1. The fusion protein, from the N-terminus to the C-terminus, sequentially includes antibody, cleavable linker, and IL-12 P35 subunit-P40 subunit fusion protein. The fusion order of the P35 subunit-P40 subunit can be the P35 subunit at the N-terminus and the P40 subunit at the C-terminus, or the P40 subunit at the N-terminus and the P35 subunit at the C-terminus. The targets of the two antibody chains may be the same or different. The present disclosure has found that the cleavable fusion protein with IL-12 located at the C-terminus exhibits different activities before and after cleavage and has better safety.

According to the embodiments of the present disclosure, when the first structural unit is linked to a plurality of third structural units, the plurality of second structural units contain at least one cleavable linker.

In some embodiments of the present disclosure, the first structural unit of one chain of the fusion protein is linked to the N-terminus of the third structural unit via a non-cleavable linker (the N-terminus of the cytokine) of the second structural unit. The first structural unit of the other chain is linked to the N-terminus of the third structural unit via a cleavable linker of the second structural unit. The structures of the fusion proteins are illustrated in LIC404, LIC405, LIC408, LIC409, LIC413, LIC414, LIC418, and LIC419 in FIG. 1. The fusion protein, from the N-terminus to the C-terminus, sequentially includes: A chain: antibody Knob sequence, non-cleavable linker, and P35 subunit; and B chain: antibody Hole sequence, cleavable linker, and P40 subunit; or A chain: antibody Knob sequence, cleavable linker, and P35 subunit; and B chain: antibody Hole sequence, non-cleavable linker, and P40 subunit. The targets of the two antibody chains may be the same or different. The present disclosure has found that, compared with the non-cleavable fusion protein with IL-12 located at the C-terminus, the single-chain cleavable fusion protein with IL-12 located at the C-terminus exhibits stronger stimulatory activity on lymphocytes after cleavage.

According to the embodiments of the present disclosure, the fusion protein includes no antibody of IL-12 cytokine or antigen-binding fragment thereof.

According to the embodiments of the present disclosure, the first antibody or antigen-binding fragment thereof is selected from a first full-length antibody, a first Fab fragment, a first ScFv fragment, or a first VHH fragment.

According to the embodiments of the present disclosure, the tumor antigen or the immune checkpoint is selected from at least one of Her2, EGFR, VEGF, VEGFR, Claudin. 18.2, B7-H3, Nectin-4, MSLN, Trop2, Siglec-9, Siglec-15, PD-L1, PD-1, TIGIT, LAG3, TIM-3, CTLA-4, BTLA, or VISTA.

In the present disclosure, "EGFR" refers to an epidermal growth factor receptor, whose mutation or overexpression generally causes tumors. After dimerization, EGFR activates downstream signal pathways in cells, which are associated with proliferation of tumor cells, angiogenesis, tumor invasion, metastasis, and apoptosis inhibition. The antibody or antigen-binding fragment that binds to EGFR can inhibit the activity of EGFR and prevent its phosphorylation, thereby inhibiting activation of downstream signal pathways and further achieving anti-tumor effects.

In the present disclosure, "VEGF" refers to a vascular endothelial growth factor, which acts on endothelial cells through a paracrine mechanism and plays an important role in promoting angiogenesis, inhibiting endothelial cell apoptosis, and increasing vascular permeability. VEGF is overexpressed in nearly all human tumors and tumor cell lines. The antibody or antigen-binding fragment that binds to VEGF can inhibit binding of VEGF to its receptors VEGFR-1 and VEGFR-2 on endothelial cells, thereby inactivating VEGF and reducing tumor angiogenesis, and thus inhibiting tumor growth.

In the present disclosure, "Claudin 18.2" refers to the 18.2 isomer molecule of the Claudin family of tight junction proteins and is a highly specific cell surface molecule. It is only expressed on differentiated gastric mucosal epithelial cells in normal tissues and is highly expressed in solid tumors such as gastric cancer, pancreatic cancer, ovarian cancer, cholangiocarcinoma, and lung adenocarcinoma. The antibody that binds to Claudin 18.2 can specifically recognize tumor cells and trigger antibody-dependent cellular cytotoxicity (ADCC), complement-dependent cytotoxicity (CDC), apoptosis, and inhibition of cell proliferation, possessing a strong ability to eliminate cancer cells and to control disease.

In the present disclosure, "PD-L1" refers to programmed death-ligand 1 and is an immune checkpoint protein. Tumor cells overexpress PD-L1, which continuously activates the PD-1/PD-L1 signal pathway and causes various types of immune suppression. The antibody or antigen-binding fragment that binds to PD-L1, as a blocking agent of the immune checkpoint protein, can inhibit the PD-1/PD-L1 pathway, block the co-inhibitory function of CD80 and PD-L1, and facilitate the comprehensive activation of T-cell functions and the promotion of cytokine production.

In the present disclosure, "PD-1" refers to a programmed death protein 1 and a co-inhibitory receptor that can be induced on the surfaces of T cells, B cells, monocytes, and natural killer cells.

In the present disclosure, "TIGIT" refers to a protein containing T cell immunoglobulin and ITIM domain. Abnormal expression of TIGIT can be detected in a variety of tumors, which can lead to immune cell dysfunction and is associated with tumor progression and poor prognosis. Blocking TIGIT can reverse immune cell exhaustion and exert anti-tumor effects, making it a new-generation target for immunotherapy.

In the present disclosure, "LAG3" refers to lymphocyte activation gene-3, "TIM-3" refers to T cell immunoglobulin mucin molecule 3, and "CTLA-4" refers to cytotoxic T lymphocyte-associated antigen-4. All three are immunosuppressive receptors and participate in the immune regulation of T cells.

According to the embodiments of the present disclosure, the IL-12 cytokine or functional fragment thereof includes at least one of a p35 fragment of IL-12 or functional fragment thereof, and a p40 fragment of IL-12 or functional fragment thereof.

It should be noted that, when the IL-12 cytokine or functional fragment thereof includes the p35 fragment of IL-12 or functional fragment thereof and the p40 fragment of IL-12 or functional fragment thereof, it is a P70 fragment of IL-12 or functional fragment thereof.

According to the embodiments of the present disclosure, the IL-12 cytokine or functional fragment thereof has an amino acid sequence as set forth in any one of SEQ ID NO: 1, SEQ ID NO: 2, SEQ ID NO: 70, and SEQ ID NO: 71.

According to the embodiments of the present disclosure, the IL-12 cytokine or functional fragment thereof includes two subunit fragments: a P35 fragment of IL-12 or functional fragment thereof, and a P40 fragment of IL-12 or functional fragment thereof. The third structural unit further includes a non-cleavable linker peptide, the two subunit fragments being linked via the non-cleavable linker peptide.

According to the embodiments of the present disclosure, the IL-12 cytokine or functional fragment thereof includes two subunit fragments: a p35 fragment of IL-12 or functional fragment thereof, and a p40 fragment of IL-12 or functional fragment thereof. The C-terminus of one of the two subunit fragments is linked to the N-terminus of the linker peptide, and the C-terminus of the linker peptide is linked to the N-terminus of the other of the two subunit fragments.

According to the embodiments of the present disclosure, the linker peptide includes at least one of (GS)n, (GGS)n, (GGSG)n, (GSSG)n, (GGGS)n, (GGGGS)n, and (GSGGS)n, where n is any integer between 1 and 10.

In an optional embodiment of the present disclosure, n is 2, 3, 4, 5, 6, 7, or 8.

According to the embodiments of the present disclosure, the linker peptide has a length of 1 to 60 amino acids, such as 2, 3, 4, 5, 6, 7, 8, 9, 10, 11, 12, 13, 14, 15, 16, 17, 18, 19, 20, 21, 22, 23, 24, 25, 26, 27, 28, 29, 30, 32, 34, 36, 38, 40, 42, 44, 46, 48, 50, 52, 54, 56, 58, 60, or any two point values between them used as range values between endpoint values.

According to the embodiments of the present disclosure, the linker peptide has an amino acid sequence as set forth in (GGGGS)n, where n is 3, 4, 5, or 6.

According to the embodiments of the present disclosure, the linker peptide has an amino acid sequence as set forth in SEQ ID NO: 75.

According to the embodiments of the present disclosure, the third structural unit has an amino acid sequence as set forth in SEQ ID NO: 1, SEQ ID NO: 2, SEQ ID NO: 70, SEQ ID NO: 71, SEQ ID NO: 76, or SEQ ID NO: 77.

In an optional embodiment of the present disclosure, the second structural unit is a cleavable linker.

In an optional embodiment of the present disclosure, the second structural unit is a cleavable linker.

In an optional embodiment of the present disclosure, the fusion protein includes two monomers. Two second structural units are a cleavable linker and a non-cleavable linker, respectively. In an optional embodiment of the present disclosure, the fusion protein includes two monomers. Both second structural units are cleavable linkers. In an optional embodiment of the present disclosure, the fusion protein includes two monomers. Both two second structural units are non-cleavable linkers.

According to the embodiments of the present disclosure, the protease expressed in the tumor tissue includes a matrix metalloproteinase, a serine protease, or an asparagine endopeptidase.

According to the embodiments of the present disclosure, the cleavable linker includes a protease substrate sequence and optionally a flexible peptide segment.

According to the embodiments of the present disclosure, the flexible peptide segment includes at least one of (GS)n, (GGS)n, (GGSG)n, (GSSG)n, (GGGS)n, (GGGGS)n, and (GSGGS)n, where n is any integer between 1 and 10.

According to the embodiments of the present disclosure, the flexible peptide segment has a length of 1 to 60 amino acids, such as 2, 3, 4, 5, 6, 7, 8, 9, 10, 11, 12, 13, 14, 15, 16, 17, 18, 19, 20, 21, 22, 23, 24, 25, 26, 27, 28, 29, 30, 32, 34, 36, 38, 40, 42, 44, 46, 48, 50, 52, 54, 56, 58, 60, or any two point values between them used as range values between endpoint values, for example, 5 to 30 amino acids, 5 to 25 amino acids, or 5 to 20 amino acids.

In some embodiments of the present disclosure, the second structural unit of the present disclosure includes a peptide linker cleavable by a matrix metalloproteinase. All the peptide linkers cleavable by the matrix metalloproteinase that are known in the art, such as those disclosed in WO2019010219A, can be used in the present disclosure, and the above-described document is incorporated herein by reference. In some embodiments, the amino acid sequence of the second structural unit includes QLLGFLTA, which is a substrate for the matrix metalloproteinases MMP-2/9/14.

In some embodiments of the present disclosure, the second structural unit of the present disclosure includes a peptide linker cleavable by a serine protease such as urokinase and trypsin. All the peptide linkers cleavable by the serine protease that are known in the art can be used in the present disclosure. In some embodiments, the amino acid sequence of the second structural unit includes LSGRSDNH, which is a substrate for the urokinase.

In some embodiments of the present disclosure, the second structural unit of the present disclosure includes a peptide linker cleavable by the asparagine endopeptidase. All the peptide linkers cleavable by the asparagine endopeptidase that are known in the art can be used in the present disclosure. In some embodiments, the amino acid sequence of the second structural unit includes AANL, which is a substrate for the asparagine endopeptidase.

According to the embodiments of the present disclosure, the protease substrate sequence is selected from QLLGFLTA, LSGRSDNH, GFFY, RQARAVGG, or AANL.

According to the embodiments of the present disclosure, the cleavable linker is a flexible peptide segment-protease substrate sequence, or a flexible peptide segment-protease substrate sequence-flexible peptide segment.

According to the embodiments of the present disclosure, the cleavable linker has a length of 15 to 60 amino acids, such as 15, 16, 17, 18, 19, 20, 21, 22, 23, 24, 25, 26, 27, 28, 29, 30, 32, 34, 36, 38, 40, 42, 44, 46, 48, 50, 52, 54, 56, 58, 60, or any two point values between them used as range values between endpoint values, for example, 15 to 50 amino acids, 15 to 45 amino acids, or 15 to 40 amino acids.

In an optional embodiment of the present disclosure, the cleavable linker is a flexible peptide segment-protease substrate sequence-flexible peptide segment. Each flexible peptide segment independently has a length of 10 to 20 amino acids (such as 10, 11, 12, 13, 14, 15, 16, 17, 18, 19, or 20), and the protease substrate sequence is selected from QLLGFLTA, LSGRSDNH, GFFY, RQARAVGG, or AANL. In an optional embodiment of the present disclosure, the amino acid sequence of the flexible peptide segment is GSSGG, GSSGGSGGSGGSG, GSSGGSGGSGGS, SSGGSGGSGGSG, or GSSGGSGGSGGSGSSGGSGG.

According to the embodiments of the present disclosure, the cleavable linker has an amino acid sequence as set forth in SEQ ID NO: 7, SEQ ID NO: 8, SEQ ID NO: 78, SEQ ID NO: 79, SEQ ID NO: 80, SEQ ID NO: 83, or SEQ ID NO: 84.

According to the embodiments of the present disclosure, the non-cleavable linker includes at least one of (GS)n, (GGS)n, (GGSG)n, (GSSG)n, (GGGS)n, (GGGGS)n, and (GSGGS)n, where n is any integer between 1 and 10.

According to the embodiments of the present disclosure, the non-cleavable linker has a length of 1 to 60 amino acids, such as 15, 16, 17, 18, 19, 20, 21, 22, 23, 24, 25, 26, 27, 28, 29, 30, 32, 34, 36, 38, 40, 42, 44, 46, 48, 50, 52, 54, 56, 58, 60, or any two point values between them used as range values between endpoint values, for example, 5 to 50 amino acids, 5 to 40 amino acids, 5 to 30 amino acids, or 10 to 20 amino acids.

According to the embodiments of the present disclosure, the non-cleavable linker has an amino acid sequence as set forth in (GGGGS)n, where n is 3, 4, 5, or 6.

According to the embodiments of the present disclosure, the non-cleavable linker has an amino acid sequence as set forth in SEQ ID NO: 5 or SEQ ID NO: 6.

According to the embodiments of the present disclosure, the first structural unit is a first ScFv fragment. The C-terminus of the first structural unit is linked to the N-terminus of the second structural unit, and the C-terminus of the second structural unit is linked to the N-terminus of the third structural unit; or the C-terminus of the third structural unit is linked to the N-terminus of the second structural unit, and the C-terminus of the second structural unit is linked to the N-terminus of the first structural unit.

According to the embodiments of the present disclosure, the C-terminus of the first structural unit is linked to the N-terminus of the second structural unit, and the C-terminus of the second structural unit is linked to the N-terminus of the third structural unit.

According to the embodiments of the present disclosure, the first structural unit is a first Fc fragment or a first VHH fragment. The first Fc fragment or the first VHH fragment is linked to one or two third structural units, each of the one or two third structural units being linked to the first Fc fragment or the first VHH fragment via the second structural unit.

According to the embodiments of the present disclosure, the C-terminus of the first structural unit is linked to the N-terminus of the second structural unit, and the C-terminus of the second structural unit is linked to the N-terminus of the third structural unit; or the C-terminus of the third structural unit is linked to the N-terminus of the second structural unit, and the C-terminus of the second structural unit is linked to the N-terminus of the first structural unit.

According to the embodiments of the present disclosure, the C-terminus of the first structural unit is linked to the N-terminus of the second structural unit, and the C-terminus of the second structural unit is linked to the N-terminus of the third structural unit.

According to the embodiments of the present disclosure, when the first Fc fragment or the first VHH fragment is linked to one third structural unit, the IL-12 cytokine or functional fragment thereof in the one third structural unit includes two subunit fragments: a P35 fragment of IL-12 or functional fragment thereof, and a P40 fragment of IL-12 or functional fragment thereof, and preferably the P35 fragment of IL-12 or functional fragment thereof, and the P40 fragment of IL-12 or functional fragment thereof.

According to the embodiments of the present disclosure, when the first Fc fragment or the first VHH fragment is linked to two third structural units, each of two second structural units is independently selected from a cleavable linker or a non-cleavable linker.

According to the embodiments of the present disclosure, two second structural units are all the cleavable linkers, or all the non-cleavable linkers, or one is a cleavable linker and the other is a non-cleavable linker.

According to the embodiments of the present disclosure, when the first Fc fragment or the first VHH fragment is linked to two third structural units, the IL-12 cytokines or the functional fragments thereof in the two third structural units are the same or different.

According to the embodiments of the present disclosure, the IL-12 cytokines or the functional fragments thereof in the two third structural units are a P35 fragment of IL-12 or functional fragment thereof, and a P40 fragment of IL-12 or functional fragment thereof, respectively.

According to the embodiments of the present disclosure, the fusion protein consists of the first structural unit, the second structural unit, and the third structural unit, and when the first structural unit is the first Fc fragment, the IL-12 cytokine or functional fragment thereof in the third structural unit includes two subunit fragments: a P35 fragment of IL-12 or functional fragment thereof, and a P40 fragment of IL-12 or functional fragment thereof. The two subunit fragments are linked to two fragments of the first Fc fragment, respectively.

According to the embodiments of the present disclosure, the first structural unit is a first full-length antibody linked to the one or more third structural units. Each of the one or more third structural units is linked to the first full-length antibody via the second structural unit.

According to the embodiments of the present disclosure, the first full-length antibody is linked to one third structural unit. One third structural unit is linked to the C-terminus of a heavy chain in the first full-length antibody via the second structural unit.

According to the embodiments of the present disclosure, the first full-length antibody is linked to a plurality of third structural units. Each of the plurality of third structural units is linked to the C-terminus of a heavy chain in the first full-length antibody via the second structural unit.

According to the embodiments of the present disclosure, the first full-length antibody is linked to two third structural units. The two third structural units are linked to the C-termini of two heavy chains in the first full-length antibody via the second structural units, respectively.

According to the embodiments of the present disclosure, when the first full-length antibody is linked to one third structural unit, the IL-12 cytokine or functional fragment thereof in the one third structural unit includes two subunit fragments: a P35 fragment of IL-12 or functional fragment thereof, and a P40 fragment of IL-12 or functional fragment thereof, and preferably the P35 fragment of IL-12 or functional fragment thereof and the P40 fragment of IL-12 or functional fragment thereof.

According to the embodiments of the present disclosure, when the first full-length antibody is linked to a plurality of third structural units, each of the plurality of second structural units is independently selected from a cleavable linker or a non-cleavable linker, and preferably, each of the plurality of second structural units is a cleavable linker.

According to the embodiments of the present disclosure, when the first full-length antibody is linked to two third structural units, each of two second structural units is independently selected from a cleavable linker or a non-cleavable linker.

According to the embodiments of the present disclosure, two second structural units are all the cleavable linkers, or all the non-cleavable linkers, or one is a cleavable linker and the other is a non-cleavable linker.

According to the embodiments of the present disclosure, when the first full-length antibody is linked to two third structural units, and when the two third structural units are linked to the C-termini of two heavy chains in the first full-length antibody via the second structural units, respectively, the IL-12 cytokines or functional fragments thereof in the two third structural units are the same or different. Preferably, the IL-12 cytokines or functional fragments thereof in the two third structural units are a P35 fragment of IL-12 or functional fragment thereof, and a P40 fragment of IL-12 or functional fragment thereof, respectively.

According to the embodiments of the present disclosure, the first structural unit is a first Fab fragment linked to one or two third structural units. Each of the one or two third structural units is linked to the C-terminus of the first Fab fragment via the second structural unit.

According to the embodiments of the present disclosure, the first Fab fragment is linked to one third structural unit. The one third structural unit is linked to the C-terminus of the CH1 fragment or the C-terminus of the CL fragment in the first Fab fragment via the second structural unit.

According to the embodiments of the present disclosure, the first Fab fragment is linked to two third structural units. The two third structural units are linked to the C-terminus of a CH1 fragment and the C-terminus of a CL fragment in the first Fab fragment via the second structural units, respectively.

According to the embodiments of the present disclosure, the first Fab fragment is linked to one third structural unit, the IL-12 cytokine or functional fragment thereof in the one third structural unit includes two subunit fragments: a P35 fragment of IL-12 or functional fragment thereof, and a P40 fragment of IL-12 or functional fragment thereof, and preferably the P35 fragment of IL-12 or functional fragment thereof, and the P40 fragment of IL-12 or functional fragment thereof.

According to the embodiments of the present disclosure, the first Fab fragment is linked to two third structural units, and each of two second structural units is independently selected from a cleavable linker or a non-cleavable linker.

According to the embodiments of the present disclosure,two second structural units are all the cleavable linkers, or all the non-cleavable linkers, or one is a cleavable linker, and the other is a non-cleavable linker.

According to the embodiments of the present disclosure, the first Fab fragment is linked to two third structural units, and the IL-12 cytokines or functional fragments thereof in the two third structural units are the same or different. Preferably, the IL-12 cytokines or functional fragments thereof in the two third structural units are a P35 fragment of IL-12 or functional fragment thereof, and a P40 fragment of IL-12 or functional fragment thereof, respectively.

According to the embodiments of the present disclosure, the fusion protein includes two monomers when the first Fab fragment is linked to one third structural unit, and when the one third structural unit is linked to the C-terminus of a CH1 fragment in the first Fab fragment via the second structural unit.

According to the embodiments of the present disclosure, in the two monomers, the IL-12 cytokine or functional fragment thereof of the third structural unit is a P35 fragment of IL-12 or functional fragment thereof, and a P40 fragment of IL-12 or functional fragment thereof. The P35 fragment of IL-12 or functional fragment thereof is linked to the P40 fragment of IL-12 or functional fragment thereof via a disulfide bond.

According to the embodiments of the present disclosure, the fusion protein further includes a fourth structural unit selected from a second Fc fragment, or a second antibody or antigen-binding fragment thereof. The second antibody or antigen-binding fragment thereof has a binding activity to a tumor antigen or an immune checkpoint.

Unless otherwise specified, the Fc fragment (e.g., the first Fc fragment and/or the second Fc fragment) herein includes a CH2 domain, a CH3 domain, and optionally a hinge region. In an embodiment of the present disclosure, the C-terminus of the CH2 domain is linked to the N-terminus of the CH3 domain. In another embodiment of the present disclosure, the C-terminus of the hinge region is linked to the N-terminus of the CH2 domain, and the C-terminus of the CH2 domain is linked to the N-terminus of the CH3 domain. In some embodiments, the two heavy chains of the fusion protein of the present disclosure may be identical or not completely identical. For example, an Fc fragment containing a "Knob-in-Hole" mutation may be used to avoid mismatch.

As used herein, the term "knob-into-hole structure" refers to a structure formed by introducing a knob mutation and a hole mutation into the CH3 domains of antibody heavy chain constant regions, thereby facilitating heavy chain pairing to form a heterodimer. For example, Fc-Knob is a human IgG1-Fc in the CH3 domain selected from S354C and T366W; and Fc-Hole is a human IgG1-Fc in the CH3 domain selected from T366S, L368A, Y407V, and optionally Y349C. In some embodiments, the fusion of different subunits of the cytokine with Fc-Knob or Fc-Hole is interchangeable without altering their structure and function. For example, LIC401, Fc-Knob of LIC401 is linked to the IL-12 p35 subunit via a flexible linker, and Fc-Hole of LIC401 is linked to the IL-12 p40 subunit via a flexible linker. If it is replaced such that Fc-Knob is linked to the IL-12 P40 subunit via a flexible linker and Fc-Hole is linked to the IL-12 P35 subunit via a flexible linker, the activity of IL-12 remains unchanged.

In some embodiments, the Fc fragment (such as the first Fc fragment and/or the second Fc fragment) is derived from human IgG1 or IgG4, and includes one or more amino acid substitutions, additions, or deletions compared to the wild type, such that the fusion protein weakens or even eliminates ADCC and/or CDC functions, reducing nonspecific immune responses.

In an optional embodiment of the present disclosure, the first Fc fragment is linked to the second Fc fragment via the knob-into-hole structure.

In an optional embodiment of the present disclosure, the Fc-Knob fragment has an amino acid sequence as set forth in SEQ ID NO: 3, and the Fc-Hole fragment has an amino acid sequence as set forth in SEQ ID NO: 4.

In some embodiments of the present disclosure, the third structural unit is located at the N-terminus of the fusion protein, as illustrated in LIC401, LIC410, and LIC415 of FIG. 1.

In some embodiments of the present disclosure, the third structural unit is located at the C-terminus of the fusion protein, as illustrated in LIC402 to LIC409, LIC411 to LIC414, LIC416 to LIC419, LIC428, LIC436, and LIC442 to LIC445 of FIG. 1.

According to the embodiments of the present disclosure, the third structural unit is located between the first structural unit and the fourth structural unit of the fusion protein, as illustrated in LIC420 to LIC427, LIC429 to LIC435, and LIC437 to LIC441 of FIG. 1.

According to the embodiments of the present disclosure, the second antibody or antigen-binding fragment thereof is selected from a second full-length antibody, a second Fab fragment, a second ScFv fragment, or a second VHH fragment.

According to the embodiments of the present disclosure, the first structural unit is the first Fc fragment, and the fourth structural unit is selected from a second Fab fragment, a second ScFv fragment, or a second VHH fragment, or the first structural unit is selected from a first Fab fragment, a first ScFv fragment, or a first VHH fragment, and the fourth structural unit is the second Fc fragment. The third structural unit is located between the first structural unit and the fourth structural unit. That is, the fusion protein, from the N-terminus to the C-terminus, sequentially includes the first structural unit, the third structural unit, and the fourth structural unit, forming a "sandwich" structure.

According to the embodiments of the present disclosure, the C-terminus of the fourth structural unit is linked to the N-terminus of the first structural unit, or the C-terminus of the third structural unit is linked to the N-terminus of the fourth structural unit.

According to the embodiments of the present disclosure, the fusion protein further includes a fifth structural unit. The fourth structural unit is linked to the first structural unit or the third structural unit via the fifth structural unit.

According to the embodiments of the present disclosure, the fifth structural unit is selected from a cleavable linker or a non-cleavable linker. The definitions of the cleavable linker or non-cleavable linker of the fifth structural unit refer to those of the cleavable linker or non-cleavable linker in the second structural unit.

It should be noted that each of the second structural unit and the fifth structural unit in the present disclosure is independently selected from a cleavable linker or a non-cleavable linker.

According to the embodiments of the present disclosure, when the third structural unit is located between the first structural unit and the fourth structural unit, the third structural unit is linked to the first structural unit via the second structural unit, and the third structural unit is linked to the fourth structural unit via the fifth structural unit. That is, the fusion protein, from the N-terminus to the C-terminus, sequentially includes the first structural unit, the second structural unit, the third structural unit, the fifth structural unit, and the fourth structural unit, forming a "sandwich" structure.

In an optional embodiment of the present disclosure, in the above-described "sandwich" structure, for the dimeric fusion protein, the at least one second structural unit is a cleavable linker.

In some embodiments of the present disclosure, the first structural unit of the fusion protein is linked to the N-terminus of the third structural unit via a non-cleavable linker (the N-terminus of the cytokine) of the second structural unit, and the fourth structural unit is linked to the C-terminus of the third structural unit via a non-cleavable linker (the C-terminus of the cytokine) of the fifth structural unit. The structures of the fusion proteins are illustrated in LIC420, LIC424, LIC429, and LIC432 in FIG. 1. The fusion protein, from the N-terminus to the C-terminus, sequentially includes: an antibody or antibody fragment such as Fab, a non-cleavable linker (the N-terminus of the cytokine), a P35 subunit or a P40 subunit or fusion proteins of the P35 subunit and the P40 subunit, a non-cleavable linker (the C-terminus of the cytokine), and an antibody or antibody fragment such as Fc. The Fab fragment may be one or two. The present disclosure has found that a single Fab is at the N-terminus, it exhibits a stronger masking effect on the activity of IL-12 than the steric hindrance masking of two Fabs.

In some embodiments of the present disclosure, the first structural unit of the fusion protein is linked to the N-terminus of the third structural unit via a cleavable linker of the second structural unit, and the fourth structural unit is linked to the C-terminus of the third structural unit via a non-cleavable linker (the C-terminus of the cytokine) of the fifth structural unit. The structures of the fusion proteins are illustrated in LIC421, LIC425, LIC430, and LIC433 in FIG. 1. The fusion protein, from the N-terminus to the C-terminus, sequentially includes: an antibody or antibody fragment such as Fab, a cleavable linker, a P35 subunit or a P40 subunit or fusion proteins of the P35 subunit and the P40 subunit, a non-cleavable linker (the C-terminus of the cytokine), and an antibody or antibody fragment such as Fc. The Fab fragment may be two or one. The present disclosure has found that the activity of IL12 is significantly enhanced after enzymatic cleavage of the cleavable linker of the second structural unit.

In some embodiments of the present disclosure, in a chain of the fusion protein of the present disclosure, the first structural unit is linked to the N-terminus of the third structural unit via a cleavable linker of the second structural unit, and the fourth structural unit is linked to the C-terminus of the third structural unit via a non-cleavable linker (the C-terminus of the cytokine) of the fifth structural unit. In the other chain, the first structural unit is linked to the N-terminus of the third structural unit via a non-cleavable linker (the N-terminus of the cytokine) of the second structural unit, and the second fourth structural unit is linked to the C-terminus of the third structural unit via a non-cleavable linker (the C-terminus of the cytokine) of the fifth structural unit. The structures of the fusion proteins are illustrated in LIC422, LIC423, LIC426, LIC427, LIC431, LIC434, and LIC435 in FIG. 1. The fusion protein, from N-terminus to C-terminus, sequentially includes: an antibody or antibody fragment such as Fab, a cleavable (non-cleavable) linker, a P35 subunit or a P40 subunit or fusion proteins of the P35 subunit and the P40 subunit, a non-cleavable linker (the C-terminus of the cytokine), and an antibody or antibody fragment such as Fc. The Fab may be two or one. The present disclosure has found that the activity of IL12 is enhanced after enzymatic cleavage of the cleavable linker of the second structural unit.

In some embodiments of the present disclosure, in a chain of the fusion protein of the present disclosure, the first structural unit is linked to the N-terminus of a segment of the fourth structural unit via a non-cleavable linker (subunits of the cytokine in tandem) of the second structural unit. In the other chain, the first structural unit is linked to the N-terminus of the third structural unit via a non-cleavable linker (the N-terminus of the cytokine) of the second structural unit, and the other segment of the fourth structural unit is linked to the C-terminus of the third structural unit via a non-cleavable linker (the C-terminus of the cytokine) of the fifth structural unit. The structures of the fusion proteins are illustrated in LIC437, LIC438, LIC439, LIC440, and LIC441 in FIG. 1. The fusion protein, from the N-terminus to the C-terminus, sequentially includes: chain A: antibody or antibody fragment, non-cleavable linker (subunits of the cytokine in tandem), and antibody or antibody fragment; and chain B: antibody or antibody fragment, non-cleavable linker (the N-terminus of subunits of the cytokine), P35 subunit-P40 subunit fusion protein or P40 subunit-P35 subunit fusion protein, non-cleavable linker (the C-terminus of the cytokine), and antibody or antibody fragment. The targets of the two chains of the antibody may be the same or different.

According to the embodiments of the present disclosure, when the first structural unit is the first Fc fragment or a first full-length antibody, the fourth structural unit is selected from a second Fab fragment, a second ScFv fragment, or a second VHH fragment. The C-terminus of the fourth structural unit is linked to the N-terminus of the first structural unit, or the C-terminus of the third structural unit is linked to the N-terminus of the fourth structural unit. Preferably, the C-terminus of the fourth structural unit is linked to the N-terminus of the fifth structural unit, and the C-terminus of the fifth structural unit is linked to the N-terminus of the first structural unit, or the C-terminus of the third structural unit is linked to the N-terminus of the fifth structural unit, and the C-terminus of the fifth structural unit is linked to the N-terminus of the fourth structural unit;

According to the embodiments of the present disclosure, when the first structural unit is a first Fab fragment, a first ScFv fragment, or a first VHH fragment, the fourth structural unit is selected from the second Fc fragment. The C-terminus of the third structural unit is linked to the N-terminus of the fourth structural unit. Preferably, the C-terminus of the third structural unit is linked to the N-terminus of the fifth structural unit, and the C-terminus of the fifth structural unit is linked to the N-terminus of the fourth structural unit.

According to the embodiments of the present disclosure, when the first structural unit is a first Fab fragment, a first ScFv fragment, or a first VHH fragment, the fourth structural unit is selected from the second Fc fragment, and the N-terminus of each fragment in the fourth structural unit is linked to the C-terminus of the third structural unit. Preferably, the N-terminus of each fragment in the fourth structural unit is linked to the C-terminus of the third structural unit via the fifth structural unit.

According to the embodiments of the present disclosure, the fusion protein includes:
a first peptide chain having an amino acid sequence as set forth in SEQ ID NO: 23, and a second peptide chain having an amino acid sequence as set forth in SEQ ID NO: 24 (i.e., LIC402);
a first peptide chain having an amino acid sequence as set forth in SEQ ID NO: 25, and a second peptide chain having an amino acid sequence as set forth in SEQ ID NO: 26 (i.e., LIC403);
a first peptide chain having an amino acid sequence as set forth in SEQ ID NO: 23, and a second peptide chain having an amino acid sequence as set forth in SEQ ID NO: 26 (i.e., LIC404);
a first peptide chain having an amino acid sequence as set forth in SEQ ID NO: 25, and a second peptide chain having an amino acid sequence as set forth in SEQ ID NO: 24 (i.e., LIC405);
a first peptide chain having an amino acid sequence as set forth in SEQ ID NO: 9, a second peptide chain having an amino acid sequence as set forth in SEQ ID NO: 27, a third peptide chain having an amino acid sequence as set forth in SEQ ID NO: 28, and a fourth peptide chain having an amino acid sequence as set forth in SEQ ID NO: 9 (i.e., LIC406);
a first peptide chain having an amino acid sequence as set forth in SEQ ID NO: 9, a second peptide chain having an amino acid sequence as set forth in SEQ ID NO: 29, and a third peptide chain having an amino acid sequence as set forth in SEQ ID NO: 30, and a fourth peptide chain having an amino acid sequence as set forth in SEQ ID NO: 9 (i.e., LIC407);
a first peptide chain having an amino acid sequence as set forth in SEQ ID NO: 9, a second peptide chain having an amino acid sequence as set forth in SEQ ID NO: 27, and a third peptide chain having an amino acid sequence as set forth in SEQ ID NO: 30, and a fourth peptide chain having an amino acid sequence as set forth in SEQ ID NO: 9 (i.e., LIC408);
a first peptide chain having an amino acid sequence as set forth in SEQ ID NO: 9, a second peptide chain having an amino acid sequence as set forth in SEQ ID NO: 29, and a third peptide chain having an amino acid sequence as set forth in SEQ ID NO: 28, and a fourth peptide chain having an amino acid sequence as set forth in SEQ ID NO: 9 (i.e., LIC409);
a first peptide chain having an amino acid sequence as set forth in SEQ ID NO: 33 and a second peptide chain having an amino acid sequence as set forth in SEQ ID NO: 34 (i.e., LIC411);
a first peptide chain having an amino acid sequence as set forth in SEQ ID NO: 35 and a second peptide chain having an amino acid sequence as set forth in SEQ ID NO: 36 (i.e., LIC412);
a first peptide chain having an amino acid sequence as set forth in SEQ ID NO: 33 and a second peptide chain having an amino acid sequence as set forth in SEQ ID NO: 36 (i.e., LIC413);
a first peptide chain having an amino acid sequence as set forth in SEQ ID NO: 35 and a second peptide chain having an amino acid sequence as set forth in SEQ ID NO: 34 (i.e., LIC414);
a first peptide chain having an amino acid sequence as set forth in SEQ ID NO: 33 and a second peptide chain having an amino acid sequence as set forth in SEQ ID NO: 38 (i.e., LIC416);
a first peptide chain having an amino acid sequence as set forth in SEQ ID NO: 35 and a second peptide chain having an amino acid sequence as set forth in SEQ ID NO: 39 (i.e., LIC417);
a first peptide chain having an amino acid sequence as set forth in SEQ ID NO: 33 and a second peptide chain having an amino acid sequence as set forth in SEQ ID NO: 39 (i.e., LIC418);
a first peptide chain having an amino acid sequence as set forth in SEQ ID NO: 35 and a second peptide chain having an amino acid sequence as set forth in SEQ ID NO: 38 (i.e., LIC419);
a first peptide chain having an amino acid sequence as set forth in SEQ ID NO: 9, a second peptide chain having an amino acid sequence as set forth in SEQ ID NO: 40, a third peptide chain as set forth in SEQ ID NO: 41, and a fourth peptide chain as set forth in SEQ ID NO: 9 (i.e., LIC420);
a first peptide chain having an amino acid sequence as set forth in SEQ ID NO: 9, and a second peptide chain having an amino acid sequence as set forth in SEQ ID NO: 42, a third peptide chain having an amino acid sequence as set forth in SEQ ID NO: 43, and a fourth peptide chain having an amino acid sequence as set forth in SEQ ID NO: 9 (i.e., LIC421);
a first peptide chain having an amino acid sequence as set forth in SEQ ID NO: 9, a second peptide chain having an amino acid sequence as set forth in SEQ ID NO: 40, a third peptide chain having an amino acid sequence as set forth in SEQ ID NO: 43, and a fourth peptide chain having an amino acid sequence as set forth in SEQ ID NO: 9 (i.e., LIC422);
a first peptide chain having an amino acid sequence as set forth in SEQ ID NO: 9, a second peptide chain having an amino acid sequence as set forth in SEQ ID NO: 41, a third peptide chain having an amino acid sequence as set forth in SEQ ID NO: 42, and a fourth peptide chain having an amino acid sequence as set forth in SEQ ID NO: 9 (i.e., LIC423);
a first peptide chain having an amino acid sequence as set forth in SEQ ID NO: 44, and a second peptide chain having an amino acid sequence as set forth in SEQ ID NO: 40 (i.e., LIC424);
a first peptide chain having an amino acid sequence as set forth in SEQ ID NO: 45, and a second peptide chain having an amino acid sequence as set forth in SEQ ID NO: 43 (i.e., LIC425);
a first peptide chain having an amino acid sequence as set forth in SEQ ID NO: 44, and a second peptide chain having an amino acid sequence as set forth in SEQ ID NO: 43 (i.e., LIC426);
a first peptide chain having an amino acid sequence as set forth in SEQ ID NO: 45 and a second peptide chain having an amino acid sequence as set forth in SEQ ID NO: 40 (i.e., LIC427);
a first peptide chain having an amino acid sequence as set forth in SEQ ID NO: 46 and a second peptide chain having an amino acid sequence as set forth in SEQ ID NO: 9 (i.e., LIC428);
a first peptide chain having an amino acid sequence as set forth in SEQ ID NO: 47 and a second peptide chain having an amino acid sequence as set forth in SEQ ID NO: 9 (i.e., LIC429);
a first peptide chain having an amino acid sequence as set forth in SEQ ID NO: 48 and a second peptide chain having an amino acid sequence as set forth in SEQ ID NO: 9 (i.e., LIC430);
a first peptide chain having an amino acid sequence as set forth in SEQ ID NO: 9, a second peptide chain having an amino acid sequence as set forth in SEQ ID NO: 49, a third peptide chain having an amino acid sequence as set forth in SEQ ID NO: 50, and a fourth peptide chain having an amino acid sequence as set forth in SEQ ID NO: 9 (i.e., LIC431);
a first peptide chain having an amino acid sequence as set forth in SEQ ID NO: 51 and a second peptide chain having an amino acid sequence as set forth in SEQ ID NO: 52 (i.e., LIC432);
a first peptide chain having an amino acid sequence as set forth in SEQ ID NO: 53 and a second peptide chain having an amino acid sequence as set forth in SEQ ID NO: 50 (i.e., LIC433);
a first peptide chain having an amino acid sequence as set forth in SEQ ID NO: 53 and a second peptide chain having an amino acid sequence as set forth in SEQ ID NO: 52 (i.e., LIC434);
a first peptide chain having an amino acid sequence as set forth in SEQ ID NO: 51 and a second peptide chain having an amino acid sequence as set forth in SEQ ID NO: 50 (i.e., LIC435);
a first peptide chain having an amino acid sequence as set forth in SEQ ID NO: 9, a second peptide chain having an amino acid sequence as set forth in SEQ ID NO: 54, a third peptide chain having an amino acid sequence as set forth in SEQ ID NO: 12, and a fourth peptide chain having an amino acid sequence as set forth in SEQ ID NO: 9 (i.e., LIC436);
a first peptide chain having an amino acid sequence as set forth in SEQ ID NO: 9, a second peptide chain having an amino acid sequence as set forth in SEQ ID NO: 55, a third peptide chain having an amino acid sequence as set forth in SEQ ID NO: 52, and a fourth peptide chain having an amino acid sequence as set forth in SEQ ID NO: 9 (i.e., LIC437);
a first peptide chain having an amino acid sequence as set forth in SEQ ID NO: 56 and a second peptide chain having an amino acid sequence as set forth in SEQ ID NO: 57 (i.e., LIC438);
a first peptide chain having an amino acid sequence as set forth in SEQ ID NO: 56 and a second peptide chain having an amino acid sequence as set forth in SEQ ID NO: 58 (i.e., LIC439);
a first peptide chain having an amino acid sequence as set forth in SEQ ID NO: 59 and a second peptide chain having an amino acid sequence as set forth in SEQ ID NO: 60 (i.e., LIC440);
a first peptide chain having an amino acid sequence as set forth in SEQ ID NO: 59 and a second peptide chain having an amino acid sequence as set forth in SEQ ID NO: 61 (i.e., LIC441);
a first peptide chain having an amino acid sequence as set forth in SEQ ID NO: 62 and a second peptide chain having an amino acid sequence as set forth in SEQ ID NO: 63 (i.e., LIC442);
a first peptide chain having an amino acid sequence as set forth in SEQ ID NO: 62 and a second peptide chain having an amino acid sequence as set forth in SEQ ID NO: 64 (i.e., LIC443);
a first peptide chain having an amino acid sequence as set forth in SEQ ID NO: 66 and a second peptide chain having an amino acid sequence as set forth in SEQ ID NO: 65 (i.e., LIC403-1);
a first peptide chain having an amino acid sequence as set forth in SEQ ID NO: 81 and a second peptide chain having an amino acid sequence as set forth in SEQ ID NO: 82 (i.e., LIC403-2);
a first peptide chain having an amino acid sequence as set forth in SEQ ID NO: 67 and a second peptide chain having an amino acid sequence as set forth in SEQ ID NO: 68 (i.e., LIC425-1);
a first peptide chain having an amino acid sequence as set forth in SEQ ID NO: 9, a second peptide chain having an amino acid sequence as set forth in SEQ ID NO: 69, a third peptide chain having an amino acid sequence as set forth in SEQ ID NO: 12, and a fourth peptide chain having an amino acid sequence as set forth in SEQ ID NO: 9 (i.e., LIC444); and
a first peptide chain having an amino acid sequence as set forth in SEQ ID NO: 74 and a second peptide chain having an amino acid sequence as set forth in SEQ ID NO: 73 (i.e., LIC445).

That is, the connection order of domains of the fusion protein of the present disclosure, from the N-terminus to the C-terminus, is any one selected from the following:
an IL-12 cytokine or functional fragment thereof of the third structural unit, a non-cleavable linker (the C-terminus of the cytokine) of the second structural unit, Fc-Knob (Hole) or Fc-Knob (Hole) + ScFv of the first structural unit, where two ScFv targets may be the same or different (as illustrated in LIC401, LIC410, and LIC415 of/in FIG. 1);
Fc-Knob (Hole), a full-length mAb, or ScFv + Fc-Knob into Hole (two ScFv targets may be the same or different) of the first structural unit, a non-cleavable linker (the N-terminus of the cytokine) of the second structural unit, and an IL-12 cytokine or functional fragment thereof (as illustrated in LIC402, LIC406, LIC411, LIC416, LIC428, LIC436, LIC442, LIC443 in FIG. 1) of the third structural unit;
Fc-Knob (Hole), a full-length mAb, or ScFv + Fc-Knob into Hole (two ScFv targets may be the same or different) of the first structural unit, a cleavable linker of the second structural unit, and an IL-12 cytokine or functional fragment thereof (as illustrated in LIC403, LIC407, LIC412, LIC417 in FIG. 1) of the third structural unit;
Fc-Knob (Hole), a full-length mAb, or ScFv + Fc-Knob into Hole (two ScFv targets may be the same or different) of the first structural units of one chain, a non-cleavable linker (the N-terminus of the cytokine) of the second structural unit, and an IL-12 cytokine or functional fragment thereof of the third structural unit; and Fc-Knob (Hole), a full-length mAb, or ScFv + Fc-Knob into Hole (two ScFv targets may be the same or different) of the first structural unit of the other chain, a cleavable linker of the second structural unit, and an IL-12 cytokine or functional fragment thereof (as illustrated in LIC404, LIC405, LIC408, LIC409, LIC413, LIC414, LIC418, and LIC419 in FIG. 1) of the third structural unit;
a Fab fragment (one or two) of the first structural unit, a non-cleavable linker (the N-terminus of the cytokine) of the second structural unit, an IL-12 cytokine or functional fragment thereof of the third structural unit, a non-cleavable linker (the C-terminus of the cytokine) of the fifth structural unit, and the N-terminus of the Fc fragment of the fourth structural unit (as illustrated in LIC420, LIC424, LIC429, and LIC432 in FIG. 1);
a Fab fragment (one or two) of the first structural unit, a non-cleavable linker (the N-terminus of the cytokine) of the second structural unit, an IL-12 cytokine or functional fragment thereof of the third structural unit, a non-cleavable linker (the C-terminus of the cytokine) of the fifth structural unit, and the N-terminus of the Fc fragment of the fourth structural unit (as illustrated in LIC421, LIC425, LIC430, and LIC433 in FIG. 1);
a Fab or VL and/or VH fragment of one chain, a cleavable linker of the second structural unit, an IL-12 cytokine or functional fragment thereof of the third structural unit, a non-cleavable linker (the the C-terminus of the cytokine) of the second structural unit, and a Fc fragment of the first structural unit; and a Fab or VL and/or VH fragment of the other chain, a non-cleavable linker (the the C-terminus of the cytokine) of the second structural unit, and an IL-12 cytokine or functional fragment thereof of the third structural unit (as illustrated in LIC422, LIC423, LIC426, LIC427, LIC431, LIC434, and LIC435 in FIG. 1);
Fab or ScFv of the first structural unit of one chain, a non-cleavable linker (subunits of the cytokine in tandem) of the second structural unit, and one fragment of Fc of the fourth structural unit; and Fab or ScFv of the first structural unit of the other chain, a non-cleavable linker (the N-terminus of the cytokine) of the second structural unit, an IL-12 cytokine or functional fragment thereof of the third structural unit, a non-cleavable linker (the N-terminus of the cytokine) of the fifth structural unit, and the other fragment of Fc of the fourth structural unit (as illustrated in LIC437, LIC438, and LIC439 in FIG. 1), where the two Fabs or ScFvs may be the same or different; and
one fragment of the Fc fragment of the first structural unit of one chain, a non-cleavable linker (subunits of the cytokine in tandem) of the second structural unit, and Fab or ScFv of the fourth structural unit; and the other fragment of the Fc fragment of the first structural unit of the other chain, a non-cleavable linker (the N-terminus of the cytokine) of the second structural unit, an IL-12 cytokine or functional fragment thereof of the third structural unit, a non-cleavable linker (the N-terminus of the cytokine) of the fifth structural unit, and Fab or ScFv of the fourth structural unit (as illustrated in LIC440 and LIC441 in FIG. 1), where the two Fabs or ScFvs may be the same or different.

The fusion protein of the present disclosure may be selected from any one of the following structures:
In an optional embodiment, the C-terminus of the third structural unit is linked to the N-terminus of the second structural unit, and the C-terminus of the second structural unit is linked to the N-terminus of the first structural unit. The second structural unit is a cleavable linker. As an example, where the first structural unit is an antibody Fc fragment, the second structural unit is a cleavable linker, and the third structural unit is IL-12P35 or IL-12P40, the fusion protein, from the N-terminus to the C-terminus, sequentially includes an Fc fragment, a cleavable linker, and IL-12P35 or IL-12P40 (as illustrated in LIC403 of FIG. 1). The present disclosure has found that Fc and the linker form steric hindrance to IL-12, thereby affecting the activity of IL-12. As a result, the activity of LIC403 is masked before cleavage and is relatively low. However, after cleavage, IL-12 is released, the steric hindrance is eliminated, and the activity is restored. Consequently, compared with the IL-12 monomer, LIC403 exhibits significantly enhanced safety.

In an optional embodiment, the C-terminus of the third structural unit is linked to the N-terminus of the second structural unit, and the C-terminus of the second structural unit is linked to the N-terminus of the first structural unit. In an optional embodiment of the present disclosure, the third structural unit is selected from an Fc fragment. The C-terminus of one fragment of the third structural unit is linked to the N-terminus of the second structural unit, and the C-terminus of the second structural unit is linked to the N-terminus of the first structural unit. The C-terminus of the other fragment of the third structural unit is linked to the N-terminus of the second structural unit, and the C-terminus of the second structural unit is linked to the N-terminus of the first structural unit. Two second structural units are a cleavable linker and a non-cleavable linker, respectively. As an example, where the first structural unit is an antibody Fc fragment, one of the second structural units is a cleavable linker and the other one of the second structural units is a non-cleavable linker (the N-terminus of the cytokine), and the third structural unit is IL-12P35 or IL-12P40, the fusion protein, from the N-terminus to the C-terminus, sequentially includes : an Fc fragment, a cleavable (non-cleavable) linker, and IL-12P35 or IL-12P40 (as illustrated in LIC404 and LIC405 of FIG. 1). The present disclosure has found that Fc and the linker form steric hindrance to IL-12, thereby affecting the activity of IL-12. As a result, the activities of LIC404 and LIC405 are masked before cleavage and are relatively low. However, after cleavage, a single IL-12 subunit is released, the steric hindrance is partially eliminated, and the activity is partially restored. Consequently, compared with complete cleavage, single-chain release can have a longer half-life. Consequently, compared with the IL-12 monomer, LIC404 exhibits significantly enhanced safety.

In an optional embodiment, the C-terminus of the third structural unit is linked to the N-terminus of the second structural unit, and the C-terminus of the second structural unit is linked to the N-terminus of the first structural unit. The second structural unit is a cleavable linker. As an example, where the first structural unit is an antibody targeting a tumor antigen or an immune checkpoint, the second structural unit is a cleavable linker, and the third structural unit is IL-12P35 or IL-12P40, the fusion protein, from the N-terminus to the C-terminus, sequentially includes a full-length antibody, a cleavable linker, and IL-12P35 or IL-12P40 (as illustrated in LIC407 of FIG. 1). The present disclosure has found that, the full-length antibody and the linker form steric hindrance to IL-12, thereby affecting the activity of IL-12. As a result, the activity of LIC407 is masked before cleavage and is relatively low. However, after cleavage, IL-12 is released, the steric hindrance is eliminated, and the activity is restored. Consequently, compared with the IL-12 monomer, LIC407 exhibits significantly enhanced safety.

In an optional embodiment, the C-terminus of the third structural unit is linked to the N-terminus of the second structural unit, and the C-terminus of the second structural unit is linked to the N-terminus of the first structural unit. In an optional embodiment of the present disclosure, the third structural unit is selected from a full-length antibody. The C-terminus of a CH3 fragment of the third structural unit is linked to the N-terminus of the second structural unit, and the C-terminus of the second structural unit is linked to the N-terminus of the first structural unit. The C-terminus of the other CH3 fragment of the third structural unit is linked to the N-terminus of the second structural unit, and the C-terminus of the second structural unit is linked to the N-terminus of the first structural unit. Two second structural units are a cleavable linker and a non-cleavable linker, respectively. As an example, where the first structural unit is a full-length antibody targeting a tumor antigen or functionally associated with PD-L1, one of the second structural units is a cleavable linker and the other one is a non-cleavable linker (the N-terminus of the cytokine), and the third structural unit is IL-12P35 or IL-12P40, the fusion protein, from the N-terminus to the C-terminus, sequentially includes: a full-length antibody, a cleavable (non-cleavable) linker, and IL-12P35 or IL-12P40 (as illustrated in LIC408 and LIC409 of FIG. 1). The present disclosure has found that the full-length antibody and the linker form steric hindrance to IL-12, thereby affecting the activity of IL-12. As a result, the activities of LIC408 and LIC409 are masked before cleavage and are relatively low. However, after cleavage, a single IL-12 subunit is released, the steric hindrance is partially eliminated, and the activity is partially restored. Consequently, compared with complete cleavage, single-chain release can have more stable targeting and a longer half-life, resulting in significantly enhanced safety of LIC408 and LIC409 compared with the IL-12 monomer.

In an optional embodiment, the C-terminus of the fourth structural unit is linked to the N-terminus of the third structural unit, the C-terminus of the third structural unit is linked to the N-terminus of the second structural unit, and the C-terminus of the second structural unit is linked to the N-terminus of the first structural unit. In an optional embodiment of the present disclosure, the third structural unit is selected from the Fc segment. In one chain, the C-terminus of the fourth structural unit is linked to the N-terminus of the third structural unit, the C-terminus of the third structural unit is linked to the N-terminus of the second structural unit, and the C-terminus of the second structural unit is linked to the N-terminus of the first structural unit. In the other chain, the C-terminus of the other CH3 segment of the third structural unit is linked to the N-terminus of the second structural unit, and the C-terminus of the second structural unit is linked to the N-terminus of the first structural unit. Two second structural units are all cleavable linkers. As an example, where the fourth structural unit is scFv, the first structural unit is an antibody Fc fragment, the second structural unit is a cleavable linker, and the third structural unit is IL-12P35 or IL-12P40, the fusion protein, from the N-terminus to the C-terminus, sequentially includes scFv, an Fc fragment, a cleavable linker, and IL-12P35 or IL-12P40 (as illustrated in LIC412 of FIG. 1). The two scFvs may be different (as illustrated in LIC417 in FIG. 1). The present disclosure has found that, the antibody scFv + Fc and the linker form steric hindrance to IL-12, thereby affecting the activity of IL-12. As a result, the activity of LIC412 is masked before cleavage and is relatively low. However, after cleavage, IL-12 is released, the steric hindrance is eliminated, and the activity is restored. Consequently, compared with the IL-12 monomer, LIC412 and LIC417 exhibit significantly enhanced safety.

In an optional embodiment, the C-terminus of the fourth structural unit is linked to the N-terminus of the third structural unit, the C-terminus of the third structural unit is linked to the N-terminus of the second structural unit, and the C-terminus of the second structural unit is linked to the N-terminus of the first structural unit. In an optional embodiment of the present disclosure, the third structural unit is selected from an Fc fragment. In one chain, the C-terminus of the fourth structural unit is linked to the N-terminus of the third structural unit, the C-terminus of the third structural unit is linked to the N-terminus of the second structural unit, and the C-terminus of the second structural unit is linked to the N-terminus of the first structural unit. In the other chain, the C-terminus of the other CH3 fragment of the third structural unit is linked to the N-terminus of the second structural unit, and the C-terminus of the second structural unit is linked to the N-terminus of the first structural unit. Two second structural units are cleavable linkers or non-cleavable linkers. As an example, where the fourth structural unit is scFv, the first structural unit is an antibody Fc fragment, a cleavable (non-cleavable) linker, and IL-12P35 or IL-12P40 (as illustrated in LIC413 and LIC414 of FIG. 1). The two ScFvs may be different (as illustrated in LIC418 and LIC419 of FIG. 1). The present disclosure has found that the antibody ScFv + Fc and the linker form steric hindrance to IL-12, thereby affecting the activity of IL-12. As a result, the activities of LIC413, LIC414, LIC418, and LIC419 are masked before cleavage and are relatively low. However, after cleavage, a single IL-12 subunit is released, the steric hindrance is partially eliminated, and the activities are partially restored. Consequently, compared with complete cleavage, single-chain release can have more stable targeting and a longer half-life, resulting in significantly enhanced targeting and safety of LIC413, LIC414, LIC418, and LIC419 compared with the IL-12 monomer.

In an optional embodiment, the C-terminus of the fourth structural unit is linked to the N-terminus of the fifth structural unit, the C-terminus of the fifth structural unit is linked to the N-terminus of the third structural unit, the C-terminus of the third structural unit is linked to the N-terminus of the second structural unit, and the C-terminus of the second structural unit is linked to the N-terminus of the first structural unit. In an optional embodiment of the present disclosure, the third structural unit is selected from an Fc segment. In one chain, the C-terminus of the fourth structural unit is linked to the N-terminus of the fifth structural unit, the C-terminus of the fifth structural unit is linked to the N-terminus of the third structural unit, the C-terminus of the third structural unit is linked to the N-terminus of the second structural unit, and the C-terminus of the second structural unit is linked to the N-terminus of the first structural unit. In the other chain, the C-terminus of the fourth structural unit is linked to the N-terminus of the fifth structural unit, the C-terminus of the fifth structural unit is linked to the N-terminus of the third structural unit, the C-terminus of the third structural unit is linked to the N-terminus of the second structural unit, and the C-terminus of the second structural unit is linked to the N-terminus of the first structural unit. Two second structural units are all non-cleavable linkers, and the two fifth structural units are all cleavable linkers. As an example, where the first structural unit is an Fc fragment, the second structural unit is a non-cleavable linker, the third structural unit is IL-12P35 or IL-12P40, the fourth structural unit is Fab (one or two), and the fifth structural unit is a cleavable linker, the fusion protein, from the N-terminus to the C-terminus, sequentially includes Fab, a cleavable linker, IL-12P35 or IL-12P40, a non-cleavable linker (the C-terminus of the cytokine), and Fc (two Fabs are illustrated in LIC421 of FIG. 1, and a single Fab is illustrated in LIC425 of FIG. 1). The present disclosure has found that two Fabs and the linker form steric hindrance to IL-12, thereby affecting the activity of IL-12. As a result, the activity of LIC421 is masked before cleavage and is relatively low. However, after cleavage, IL-12 is released, the steric hindrance is eliminated, and the activity is restored. Consequently, the disulfide bond masking ability in a single Fab is stronger than the steric hindrance of two Fabs, resulting in significantly enhanced safety of LIC421 and LIC425 compared with the IL-12 monomer.

In an optional embodiment, the C-terminus of the fourth structural unit is linked to the N-terminus of the fifth structural unit, the C-terminus of the fifth structural unit is linked to the N-terminus of the third structural unit, the C-terminus of the third structural unit is linked to the N-terminus of the second structural unit, and the C-terminus of the second structural unit is linked to the N-terminus of the first structural unit. In an optional embodiment of the present disclosure, the third structural unit is selected from the Fc segment. In one chain, the C-terminus of the fourth structural unit is linked to the N-terminus of the fifth structural unit, the C-terminus of the fifth structural unit is linked to the N-terminus of the third structural unit, the C-terminus of the third structural unit is linked to the N-terminus of the second structural unit, and the C-terminus of the second structural unit is linked to the N-terminus of the first structural unit. In the other chain, the C-terminus of the fourth structural unit is linked to the N-terminus of the fifth structural unit, the C-terminus of the fifth structural unit is linked to the N-terminus of the third structural unit, the C-terminus of the third structural unit is linked to the N-terminus of the second structural unit, and the C-terminus of the second structural unit is linked to the N-terminus of the first structural unit. Two second structural units are all non-cleavable linkers, and the two fifth structural units are a cleavable linker and a non-cleavable linker, respectively. As an example, where the first structural unit is an Fc fragment, the second structural unit is a non-cleavable linker, the third structural unit is IL-12P35 or IL-12P40, the fourth structural unit is Fab (one or two), and the fifth structural unit is a cleavable linker, the fusion protein, from the N-terminus to the C-terminus, sequentially includes Fab, a cleavable linker, IL-12P35 or IL-12P40, a non-cleavable linker (the C-terminus of the cytokine), and Fc (two Fabs are illustrated in LIC422 and LIC423 of FIG. 1, and a single Fab is illustrated in LIC426 and LIC427 of FIG. 1). The present disclosure has found that two Fabs and the linker form steric hindrance to IL-12, thereby affecting the activity of IL-12. The disulfide bond masking ability in a single Fab is stronger than the steric hindrance of two Fabs. As a result, the activities of LIC422, LIC423, LIC426, and LIC427 are masked before cleavage and are relatively low. However, after cleavage, a single L-12 subunit is released, the steric hindrance is partially eliminated, and the activity is partially restored. Compared with complete cleavage, single-chain release can have more stable targeting and a longer half-life, resulting in significantly enhanced targeting and safety of LIC422, LIC423, LIC426, and LIC427 compared with the IL-12 monomer.

In an optional embodiment, the C-terminus of the fourth structural unit is linked to the N-terminus of the fifth structural unit, the C-terminus of the fifth structural unit is linked to the N-terminus of the third structural unit, the C-terminus of the third structural unit is linked to the N-terminus of the second structural unit, and the C-terminus of the second structural unit is linked to the N-terminus of the first structural unit. In an optional embodiment of the present disclosure, the third structural unit is selected from an Fc segment. In one chain, the C-terminus of the fourth structural unit is linked to the N-terminus of the fifth structural unit, the C-terminus of the fifth structural unit is linked to the N-terminus of the third structural unit, the C-terminus of the third structural unit is linked to the N-terminus of the second structural unit, and the C-terminus of the second structural unit is linked to the N-terminus of the first structural unit. In the other chain, the C-terminus of the fourth structural unit is linked to the N-terminus of the fifth structural unit, the C-terminus of the fifth structural unit is linked to the N-terminus of the third structural unit, the C-terminus of the third structural unit is linked to the N-terminus of the second structural unit, and the C-terminus of the second structural unit is linked to the N-terminus of the first structural unit. Two second structural units are all non-cleavable linkers, and the two fifth structural units are all cleavable linkers. As an example, where the first structural unit is an Fc fragment, the second structural unit is a non-cleavable linker, the third structural unit is IL-12P70, the fourth structural unit is Fab (one or two), and the fifth structural unit is a cleavable linker, the fusion protein, from the N-terminus to the C-terminus, sequentially includes Fab, a cleavable linker, IL-12P70, a non-cleavable linker (the C-terminus of the cytokine), and Fc (two Fabs are illustrated in LIC430 of FIG. 1, and a single Fab is illustrated in LIC433 of FIG. 1). The present disclosure has found that Fab and Fc form steric hindrance to IL-12, thereby affecting the activity of IL-12. As a result, the activity of LIC430 is masked before cleavage and is relatively low. However, after cleavage, IL-12 is released, the steric hindrance is eliminated, and the activity is restored. Consequently, the disulfide bond masking ability in a single Fab is stronger than the steric hindrance of two Fabs, resulting in significantly enhanced safety of LIC430 and LIC433 compared with the IL-12 monomer.

In an optional embodiment, the C-terminus of the fourth structural unit is linked to the N-terminus of the fifth structural unit, the C-terminus of the fifth structural unit is linked to the N-terminus of the third structural unit, the C-terminus of the third structural unit is linked to the N-terminus of the second structural unit, and the C-terminus of the second structural unit is linked to the N-terminus of the first structural unit. In an optional embodiment of the present disclosure, the third structural unit is selected from an Fc segment. In one chain, the C-terminus of the fourth structural unit is linked to the N-terminus of the fifth structural unit, the C-terminus of the fifth structural unit is linked to the N-terminus of the third structural unit, the C-terminus of the third structural unit is linked to the N-terminus of the second structural unit, and the C-terminus of the second structural unit is linked to the N-terminus of the first structural unit. In the other chain, the C-terminus of the fourth structural unit is linked to the N-terminus of the fifth structural unit, the C-terminus of the fifth structural unit is linked to the N-terminus of the third structural unit, the C-terminus of the third structural unit is linked to the N-terminus of the second structural unit, and the C-terminus of the second structural unit is linked to the N-terminus of the first structural unit. Two second structural units are all non-cleavable linkers, and the two fifth structural units are a cleavable linker and a non-cleavable linker, respectively. As an example, where the first structural unit is an Fc fragment, the second structural unit is a non-cleavable linker, the third structural unit is IL-12P70, the fourth structural unit is Fab (one or two), and the fifth structural unit is a cleavable linker, the fusion protein, from the N-terminus to the C-terminus sequentially includes Fab, a cleavable linker, IL-12P70, a non-cleavable linker (the C-terminus of the cytokine), and Fc (two Fabs are illustrated in LIC431 of FIG. 1, and a single Fab is illustrated in LIC434 and LIC435 of FIG. 1). The present disclosure has found that Fab and Fc form steric hindrance to IL-12, thereby affecting the activity of IL-12. The disulfide bond masking ability in a single Fab is stronger than the steric hindrance of two Fabs. As a result, the activities of LIC431, LIC434, and LIC435 are masked before cleavage and are relatively low. However, after cleavage, a single L-12 subunit is released, the steric hindrance is partially eliminated, and the activity is partially restored. Compared with complete cleavage, single-chain release can have more stable targeting and a longer half-life, resulting in significantly enhanced targeting and safety of LIC431, LIC434, and LIC435 compared with the IL-12 monomer.

In an optional embodiment, the third structural unit is linked to the first structural unit and the fourth structural unit via the N-terminus and the C-terminus, respectively. As an example, where the first structural unit is Fab, ScFv, or Fc, the second structural unit is a non-cleavable linker, the third structural unit is IL-12P70, the fourth structural unit is Fab, ScFv, or Fc, and the fifth structural unit is a non-cleavable linker, A chain of the fusion protein, from the N-terminus to the C-terminus, sequentially includes Fab or ScFv or Fc, a cleavable linker (cellular subunits in tandem), and Fc or Fab or ScFv; and chain B sequentially includes Fab or ScFv or Fc, a non-cleavable linker (N-terminus of the cytokine), IL-12P70, and Fc or Fab or ScFv (as illustrated in LIC437-LIC441 of FIG. 1). The present disclosure has found that Fab and Fc form steric hindrance to IL-12, thereby affecting the activity of IL-12. As a result, the activity of IL-12 in LIC437 to LIC441 is weakened, resulting in significantly enhanced safety compared with the IL-12 monomer.

### Nucleic Acid Molecule, Expression Vector, and Recombinant Cell

In a second aspect of the present disclosure, the present disclosure provides a nucleic acid molecule. According to the embodiments of the present disclosure, the nucleic acid molecule encodes the fusion protein described in the first aspect. The nucleic acid molecule can encode the fusion protein described in the first aspect.

The nucleic acid of the present disclosure may be DNA or RNA molecules, or nucleic acid analogs. The nucleic acid molecule of the present disclosure may include naturally occurring or artificially generated nucleic acid residues. The nucleic acid molecule of the present disclosure may be single-stranded or double-stranded, linear or circular, natural or synthetic, and unless otherwise specified, there is no restriction on its size. The nucleic acid molecule may also include a promoter, which may be homologous or heterologous.

It should be noted that, for the nucleic acid mentioned in the present disclosure, those skilled in the art should understand that they actually include any one or both of the complementary double strands. In this specification and claims, for convenience, only one strand is given in most cases, but the other strand complementary to the one strand is actually also disclosed. In addition, the nucleic acid sequences of the present disclosure include a DNA form or an RNA form, the disclosure of one of which implies the disclosure of the other.

In a third aspect of the present disclosure, the present disclosure provides an expression vector. According to the embodiments of the present disclosure, the expression vector carries the nucleic acid described in the second aspect. When the above-described nucleic acid is connected to the expression vector, the nucleic acid can be directly or indirectly connected to the control elements on the vector, as long as these control elements can control the translation and expression and the like of the nucleic acid. These control elements may be directly derived from the expression vector itself, or may be exogenous, i.e., derived from the expression vector itself. The nucleic acid is operably connected to the control elements.

As used herein, "operably connected" means that an exogenous gene is connected to the expression vector, in such a manner that the control elements in the expression vector, such as transcription control sequences and translation control sequences, can play their expected functions of regulating the transcription and translation of the exogenous gene. Common vectors may be, for example, plasmids, cosmids, viruses, bacteriophages, and other vectors commonly used in genetic engineering. In some embodiments, these vectors are suitable for transforming cells, eukaryotic cells such as fungal cells, and microbial cells such as yeast or prokaryotic cells. In a preferred embodiment, these vectors are suitable for stable transformation of bacterial cells, for example, to transcribe the nucleic acid molecules of the present disclosure.

According to some specific embodiments of the present disclosure, once the expression vector is introduced into suitable recipient cells, the expression of the fusion protein described in the first aspect can be effectively achieved under the mediation of the regulatory system, thereby obtaining a large amount of the fusion protein *in vitro*.

In an embodiment, the expression vector may contain a marker gene, an origin of replication that ensures replication in the selected host, a promoter, and a transcription termination signal. Between the promoter and the termination signal, there is preferably at least one restriction site into which the nucleic acid sequence/molecule to be expressed can be inserted.

According to the embodiments of the present disclosure, the expression vector comprises a eukaryotic expression vector or a prokaryotic expression vector.

In an optional embodiment of the present disclosure, the expression vector is a plasmid expression vector or a viral expression vector, such as a lentiviral expression vector. Preferably, the expression vector of the present disclosure is selected from pET series expression vectors, pGEX series expression vectors, pcDNA series expression vectors, and pCMV series expression vectors. More preferably, the expression vector of the present disclosure is the pMF09 expression vector.

In a fourth aspect of the present disclosure, the present disclosure provides a recombinant cell. According to the embodiments of the present disclosure, the recombinant cell carries the nucleic acid described in the second aspect or the expression vector described in the third aspect, or expresses the fusion protein described in the first aspect. Under suitable conditions, the recombinant cell can be used to effectively express the fusion protein described in the first aspect intracellularly.

According to some specific embodiments of the present disclosure, the recombinant cell can efficiently and abundantly express the fusion protein under suitable conditions. The fusion protein has stronger specificity, a longer half-life, and higher efficacy with reduced toxic and side effects and higher safety.

It should be noted that, the "suitable conditions" refer to conditions suitable for the expression of the fusion protein described in the present disclosure. Those skilled in the art can easily understand that the conditions suitable for the expression of the fusion protein include, but are not limited to, appropriate transformation or transfection methods, appropriate transformation or transfection conditions, healthy host cell states, appropriate host cell density, suitable cell culture environment, and suitable cell culture time. The "suitable conditions" are not particularly limited, and those skilled in the art can optimize the most suitable conditions for the expression of the above-described fusion protein according to the specific environment of the laboratory.

According to the embodiments of the present disclosure, the recombinant cell is obtained by introducing the expression vector described in the third aspect into a host cell.

In an embodiment, the fusion protein is expressed in the recombinant cell, and the fusion protein is subsequently isolated and typically purified to a pharmaceutically acceptable purity. For protein expression, the nucleic acid encoding the protein is inserted into an expression vector using standard methods. Expression is carried out in suitable stable host cells, and the protein is recovered from the cells (supernatant or lysed cells).

In another embodiment, the nucleic acid molecule of the present disclosure and/or the vector containing the nucleic acid molecule of the present disclosure can be transduced, transformed, transfected, or introduced into the host cell by other means. For example, the host cell is a eukaryotic or prokaryotic cell, preferably a eukaryotic cell. As a non-limiting example, the host cell is a mammalian cell. The host cell of the present disclosure may be human, yeast, or fungal cells, such as Chinese hamster ovary (CHO) cells, baby hamster kidney cells (BHK, ATCC CCL 10), Sertoli cells from baby mice, monkey kidney cells (COS cells), monkey kidney CVI cells transformed by SV40 (COS-7, ATCC CRL 1651), human embryonic kidney (HEK-293) cells, monkey kidney (CVI, ATCC CCL-70) cells, African green monkey kidney (VERO-76, ATCC CRL-1587) cells, human cervical cancer (HELA, ATCC CCL-2) cells, etc. Preferably, the host cell of the present disclosure is a human HEK293 cell.

The present disclosure further provides a method for preparing the above-described fusion protein. The method includes culturing the host cell of the present disclosure under conditions suitable for expression of the fusion protein, and recovering the fusion protein from the cell or cell culture supernatant. In an embodiment, the method for preparing the fusion protein of the present disclosure includes constructing an expression vector containing a gene encoding the fusion protein, constructing the host cell containing the expression vector through transiently transfection of the host cell, culturing the host cell, collecting the cell supernatant, and purifying the fusion protein by affinity chromatography using Protein A/G as a filler.

Suitable conditions for expressing the above-described fusion protein should be known to those skilled in the art. Those skilled in the art can select an applicable culture medium based on experience to culture the cells under conditions suitable for the growth of the host cells. After the host cell grows to an appropriate cell density, the selected promoter is induced using a suitable method (such as temperature shift or chemical induction), and then the cells are cultured for an additional period of time. The recombinant polypeptide in the above method can be expressed intracellularly, on the cell membrane, or secreted extracellularly.

Methods for isolating and purifying the fusion protein are well known to those skilled in the art. Examples of these methods include, but are not limited to, conventional renaturation, treatment with protein precipitants, centrifugation, osmotic shock, ultra-treatment, ultracentrifugation, molecular sieve chromatography, adsorption chromatography, ion exchange chromatography, high-performance liquid chromatography, and various other liquid chromatography techniques, as well as combinations of these methods.

### Fusion Protein Complex, Immunotherapy Cell, and Pharmaceutical Composition

In a fifth aspect of the present disclosure, the present disclosure provides a fusion protein complex. According to the embodiments of the present disclosure, the fusion protein complex includes the fusion protein described in the first aspect. As can be seen above, the third structural unit of the fusion protein described in the first aspect contains the IL-12 cytokine. The second structural unit mediates steric hindrance of the first structural unit to mask an activity of the IL-12 cytokine in the third structural unit, particularly without requiring a conventional affinity masking strategy. Therefore, the fusion protein can reduce toxic and side effects induced by the IL-12 cytokine without incorporating any antibody or receptor of the IL-12 cytokine and thus exhibiting the advantages of enhanced safety. In this way, the fusion protein complex of the present disclosure has advantages of enhanced safety and can be used for treating cancer, an infectious disease, or an autoimmune disease.

An embodiment of the present disclosure relates to a fusion protein complex, which may be a dimeric protein formed by the binding of two different or identical fusion proteins. That is, the fusion protein complex may be a homodimer or a heterodimer. The fusion proteins in the fusion protein complex can be linked to each other via interchain bonds formed between Fc fragments, preferably covalently linked via disulfide bonds formed between the Fc fragments to form a dimer. In some embodiments, the fusion protein complex includes two fusion proteins of the present disclosure.

In a sixth aspect of the present disclosure, the present disclosure provides an immunotherapy cell. According to the embodiments of the present disclosure, the immunotherapy cell expresses the fusion protein described in the first aspect. As can be seen above, the third structural unit of the fusion protein described in the first aspect contains the IL-12 cytokine. The second structural unit mediates steric hindrance of the first structural unit to mask an activity of the IL-12 cytokine in the third structural unit, particularly without requiring a conventional affinity masking strategy. Therefore, the fusion protein can reduce toxic and side effects induced by the IL-12 cytokine without incorporating any antibody or receptor of the IL-12 cytokine and thus exhibiting advantages of enhanced safety. In this way, the immunotherapy cell containing the fusion protein described above has advantages of enhanced safety and can be used for treating cancer, an infectious disease, or an autoimmune disease.

In a seventh aspect of the present disclosure, the present disclosure provides a pharmaceutical composition. According to the embodiments of the present disclosure, the pharmaceutical composition includes the fusion protein described in the first aspect, the nucleic acid molecule described in the second aspect, the expression vector described in the third aspect, the recombinant cell described in the fourth aspect, the fusion protein complex described in the fifth aspect, or the immunotherapy cell described in the sixth aspect. As can be seen above, the third structural unit of the fusion protein described in the first aspect contains the IL-12 cytokine. The second structural unit mediates steric hindrance of the first structural unit to mask an activity of the IL-12 cytokine in the third structural unit, particularly without requiring a conventional affinity masking strategy. Therefore, the fusion protein can reduce toxic and side effects induced by the IL-12 cytokine without incorporating any antibody or receptor of the IL-12 cytokine and thus exhibiting advantages of enhanced safety. In this way, the pharmaceutical composition containing the fusion protein described above has advantages of enhanced safety and can be used for treating cancer, an infectious disease, or an autoimmune disease.

In some embodiments, the pharmaceutical composition of the present disclosure further includes a monoclonal antibody targeting a tumor antigen or an immune checkpoint, such as PD-1, PD-L1, LAG3, TIM-3, CTLA-4, Her2, EGFR, Claudin 18.2, VEGF, CD20, and CD33. Combined use with the fusion protein of the present disclosure can exert a synergistic anti-tumor effect and enhance the therapeutic efficacy on diseases.

In some embodiments of the present disclosure, the pharmaceutical composition may further include a pharmaceutically acceptable excipient. Examples of suitable pharmaceutical carriers are well known in the art. The pharmaceutical compositions containing such carriers can be formulated by well-known conventional methods. In some embodiments, the pharmaceutical composition of the present disclosure may further contain other active ingredients for treatment.

### Use or Therapeutic Method

In an eighth aspect of the present disclosure, the present disclosure provides use of the fusion protein described in the first aspect, the fusion protein complex described in the fifth aspect, the immunotherapy cell described in the sixth aspect, or the pharmaceutical composition described in the seventh aspect in the manufacture of a medicament for treating cancer, an infectious disease, or an autoimmune disease.

The present disclosure provides use of the fusion protein described in the first aspect, the fusion protein complex described in the fifth aspect, the immunotherapy cell described in the sixth aspect, or the pharmaceutical composition described in the seventh aspect in the treatment of cancer, an infectious disease, or an autoimmune disease.

The present disclosure provides the fusion protein described in the first aspect, the fusion protein complex described in the fifth aspect, the immunotherapy cell described in the sixth aspect, or the pharmaceutical composition described in the seventh aspect, for use in the treatment of cancer, an infectious disease, or an autoimmune disease.

The above-described fusion protein, fusion protein complex, immunotherapy cell, and pharmaceutical composition of the present disclosure can be administered in different ways, such as via injection (e.g., intravenous, subcutaneous, intramuscular, intraperitoneal, and intradermal). Preferably, the pharmaceutical composition of the present disclosure is in the form of a lyophilized formulation or an aqueous solution. The clinical dosage regimen is determined by the attending physician and clinical factors. As is well known in the medical field, the dosage for any individual patient depends on many factors, including the patient's physical build, body surface area, age, drug to be administered, gender, time and route of administration, general health, and other drugs administered simultaneously. The fusion protein, the fusion protein complex, the immunotherapy cell, and the pharmaceutical composition of the present disclosure can be administered locally or systemically. Preferably, it can be administered intravenously or subcutaneously. The fusion protein, the fusion protein complex, the immunotherapy cell, and the pharmaceutical composition of the present disclosure can also be administered directly to the target site, for example, through targeted drug delivery to internal or external target sites.

The cancer includes, but is not limited to, leukemia (e.g., acute leukemia, acute lymphocytic leukemia, acute myeloid leukemia, acute myeloblastic leukemia, acute promyelocytic leukemia, acute myelomonocytic leukemia, acute monocytic leukemia, acute erythrocytic leukemia, chronic leukemia, chronic myeloid leukemia, or chronic lymphocytic leukemia), polycythemia vera, lymphoma (Hodgkin's disease, non-Hodgkin's disease), macroglobulinemia, and solid tumors such as sarcomas and malignant tumors (e.g., fibrosarcoma, myxosarcoma, liposarcoma, chondrosarcoma, osteosarcoma, chordoma, angiosarcoma, endotheliosarcoma, lymphangiosarcoma, lymphangioendotheliosarcoma, synovioma, mesothelioma, Ewen's tumor, leiomyosarcoma, rhabdomyosarcoma, colon cancer, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinoma, cystadenocarcinoma, medullary carcinoma, bronchogenic carcinoma, renal cell carcinoma, liver cancer, cholangiocarcinoma, choriocarcinoma, seminoma, embryonal carcinoma, Wilms' tumor, cervical cancer, uterine cancer, testicular cancer, lung cancer, small cell lung cancer, bladder cancer, epithelial carcinoma, glioma, astrocytoma, medulloblastoma, craniopharyngioma, ependymoma, pinealma, hemangioblastoma, acoustic neuroma, oligodendroglioma, schwannoma, meningioma, melanoma, neuroblastoma, and retinoblastoma). The infectious disease includes, but is not limited to, smallpox virus infection, HIV infection, bacterial infection, fungal infection, and HBV infection. The autoimmune disease includes, but is not limited to, multiple sclerosis, psoriasis, rheumatoid arthritis, systemic lupus erythematosus, ankylosing spondylitis, Crohn's disease, gastritis, and mucositis.

In the present disclosure, the medicament includes therapeutic cells, such as CAR T cells and CAR NK cells. The fusion protein of the present disclosure is expressed or modified on the surface of the cells, which helps enhance the survival and activity of cells at the tumor site, thereby improving the efficacy of cell therapy for diseases.

In an eighth aspect of the present disclosure, the present disclosure provides a method for treating cancer, an infectious disease, or an autoimmune disease. According to the embodiments of the present disclosure, the method includes: administering to a subject a pharmaceutically acceptable amount of the fusion protein described in the first aspect, the fusion protein complex described in the fifth aspect, the immunotherapy cell described in the sixth aspect, or the pharmaceutical composition described in the seventh aspect.

The solutions of the present disclosure will be illustrated with reference to examples. Those skilled in the art can understand that the following examples are only used to illustrate the present disclosure and should not be regarded as limitations of the scope of the present disclosure. If the specific technologies or conditions are not specified in the examples, they shall be carried out according to the technologies or conditions as described in the literature in the art or according to the product instructions. The reagents and instruments used without indicating the manufacturer are all conventional products that can be purchased commercially.

### Example 1: Preparation of IL-12-non-cleavable-Fc (LIC401) Fusion Protein

### 1. Construction of Expression Vector Encoding LIC401 Fusion Protein

The nucleic acid sequence encoding the LIC401 fusion protein (the corresponding amino acid sequences were as set forth in SEQ ID NO: 21 and SEQ ID NO: 22) was prepared by gene synthesis and PCR technology. As illustrated in FIG. 1, IL-12 located at the N-terminus in the LIC401 fusion protein is linked to Fc via a non-cleavable linker. IL-12 contained a p35 subunit and a p40 subunit.

The obtained nucleic acid sequence was inserted between the Hind III and BamH I restriction sites of the pMF09 plasmid through homologous recombination. The resulting homologous recombination product was transformed into the DH5α strain to obtain the expression vector pMF09/LIC401. Strains with correctly constructed vectors were verified by sequencing. After culturing to the logarithmic growth phase, glycerol with a final concentration of 20% was added for long-term storage at -80°C.

### 2. Plasmid Amplification

Strains containing the expression vector pMF09/LIC401 were cultured in an expanded volume at an inoculum ratio of 1:100. Plasmids were extracted by the alkaline lysis method using the E.Z.N.A. Endo-free Plasmid Maxi Kit (catalog number D6926-03) provided by OMEGA, filtered through a sterile filter membrane, and then stored at -20°C.

### 3. Cell Culture and Transient Transfection for Expression of LIC401 Fusion Protein

### 1) Cell Preparation

Human HEK293E cells were cultured in suspension. The formula of the medium was prepared by mixing two media SFM4HEK293 Medium (Hyclone, USA) and Freestyle 293 Expression Medium (Invitrogen, USA) at a volume ratio of 1:1, followed by the addition of fetal bovine serum with a final concentration of 2%. The suspension was passaged into the fresh medium at a density of 1×10⁶ cells/mL and cultured in suspension in a constant temperature shaker (5% CO₂) at 37°C with a rotational speed of 125 rpm.

One day before transfection, cells were passaged into a 500 mL conical culture flask, and the cell state and density were adjusted to 4×10⁶ cells/mL with a culture volume of 200 mL.

On the day of transfection, when the cell density reached 6×10⁶ cells/mL and the cell viability exceeded 95%, the cells were subjected to transfection. 100 mL of cell culture was subjected to centrifugation to obtain cells. The cells were washed once with Freestyle 293 medium and then suspended in 100 mL of Freestyle 293 medium.

### 2) Plasmid Dilution

The plasmid was used at a mass of 0.5 µg plasmid per 10⁶ cells, diluted to 40 ng/µL with Freestyle 293 medium, and mixed thoroughly.

| | Mass ratio of Knob chain to Hole chain | |
|---|---|---|
| | 1:1 | |
| Mass/µg | 150 | 150 |
| Concentration, ng/µl | 435 | 330 |
| Dilute to 40 ng/µL, Volume/mL | 344.8µl→3.75ml | 454.5µl→3.75ml |

### 3) Plasmid Encapsulation with PEI

Plasmid and PEI (polyethylenimine, transfection reagent, purchased from Polysciences) were mixed at a mass ratio of 1:5. Specifically, 1.5 mL of PEI was added to the diluted plasmid, mixed thoroughly, and allowed to stand for 10 to 15 minutes for encapsulation. The time was controlled as the mixture became turbid.

### 4) PEI Transfection

The PEI-encapsulated plasmid was added to the cells and placed in a shaker for culture.

### 5) Medium Supplementation at 4 h

After 4 hours, an equal volume of 100 mL of SFM4 HEK293 medium, 1 mL of VPA (200X, 750 mmol/L, final concentration of 3.75 mmol/L), and 400 µL of G418 (50 mg/mL) were added.

### 6) Medium Supplementation at 24 h

200 µL of anti-clumping solution (1000X) and 5.0 mL of 20% TN1 (final concentration of 0.5%) were added.

### 7) Cell viability was monitored. After 6 days to 7 days of culture, when the cell viability dropped below 50%, the cell culture supernatant was collected for subsequent purification step.

### 4. Purification of LIC401 Fusion Protein

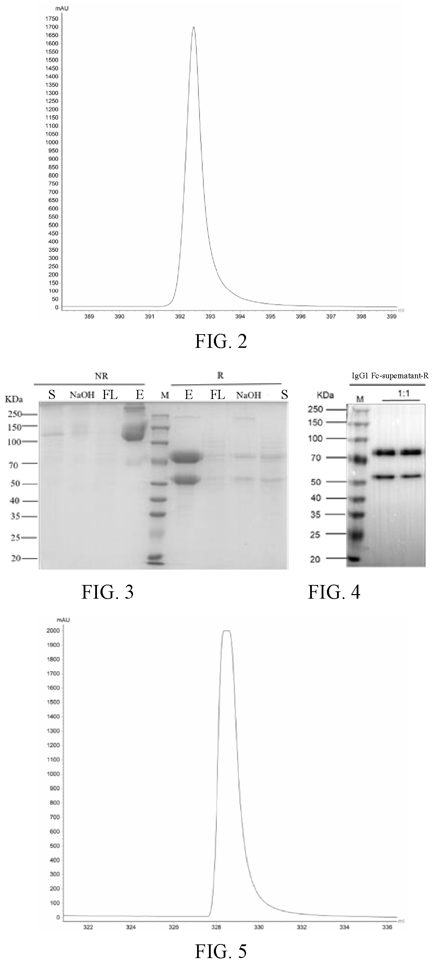

1) Sample Preparation: The cell suspension was centrifuged at 7000 rpm for 30 minutes. The precipitate was discarded, and the supernatant was filtered through a 0.45 µm filter membrane for later use.
2) The protein purification system was rinsed with 20% ethanol. The Protein A column was rinsed and equilibrated with 10 column volumes of double-distilled water and loading buffer, respectively.
3) The sample was loaded using a peristaltic pump of the AKTA start protein purifier. The flow rate was set according to the column volume, generally not exceeding one column volume per minute, and the flow-through was collected.
4) After loading, the Protein A column was re-equilibrated with 10 column volumes of loading buffer.
5) Elution was performed using an elution buffer (citric acid-sodium citrate buffer, pH=2.8). The eluate was collected in fractions, with 1 mL per tube. The elution peak was monitored according to ultraviolet absorbance value at 280 nm. An appropriate amount of 1 M Tris-HCl (pH=9.0) was added to the collection tube containing the elution peak to adjust the pH of the target protein solution to approximately 7.0 to 8.0. A diagram showing protein A affinity chromatography purification of the LIC401 fusion protein is illustrated in FIG. 2. An ultraviolet absorption peak exceeding 1000 mAU can be observed, indicating that the target protein was eluted by the elution buffer.
6) The Protein A column was rinsed with 150 mM NaOH solution, followed by sequential rinse with 10 column volumes of double-distilled water and 20% ethanol, leaving the column filler fully immersed in ethanol.
7) The purity of each elution tube was detected by SDS-PAGE. All elution tubes were combined, and after ultrafiltration and buffer exchange operations, the protein was dissolved in PBS for later use in the next step. The SDS-PAGE electrophoretogram of the fusion protein LIC401 after affinity chromatography purification is illustrated in FIG. 3. The target protein was obtained after affinity chromatography. The molecular weight of the sample subjected to non-reducing treatment was between 100 kDa and 150 kDa, while the molecular weight of the single chain of the sample subjected to reducing treatment was between 50 kDa and 70 kDa, with two bands. The two bands were polypeptide chains having amino acid sequences as set forth in SEQ ID NO: 21 and SEQ ID NO: 22, respectively.

### 5. Western Blot Verification of LIC401 Fusion Protein Expression

1) An appropriate amount of the LIC401 fusion protein purified by affinity chromatography was subjected to SDS-PAGE. Electrophoresis conditions were 90 V for 20 min and then 100 V for 70 min.
2) The target protein was transferred to a PVDF membrane using the wet transfer method. The transfer condition was 200 mA for 90 min.
3) After transfer, the PVDF membrane was blocked with 5% skimmed milk at room temperature for 2 h or overnight at 4°C.
4) A goat anti-human heavy chain and light chain-HRP antibody was directly used as the secondary antibody to verify the expression of the antibody fragment in the LIC401 fusion protein. The incubation time was 1 h at room temperature.
5) After membrane washing and ECL chemiluminescence, the results illustrated in FIG. 4 were obtained. These results demonstrated that the non-reduced LIC401 fusion protein exhibited a band between 100 kDa and 150 kDa, while the reduced fusion protein exhibited two chains bands between 50 kDa and 70 kDa, which were consistent with the SDS-PAGE results of the previous purified protein. In addition, the binding of the secondary antibody to the antibody fragment of the LIC401 fusion protein in Western Blot confirmed the expression of this portion.

### Example 2: Preparation of Fc-non-cleavable-IL-12 (LIC402 and LIC402-1) Fusion Protein

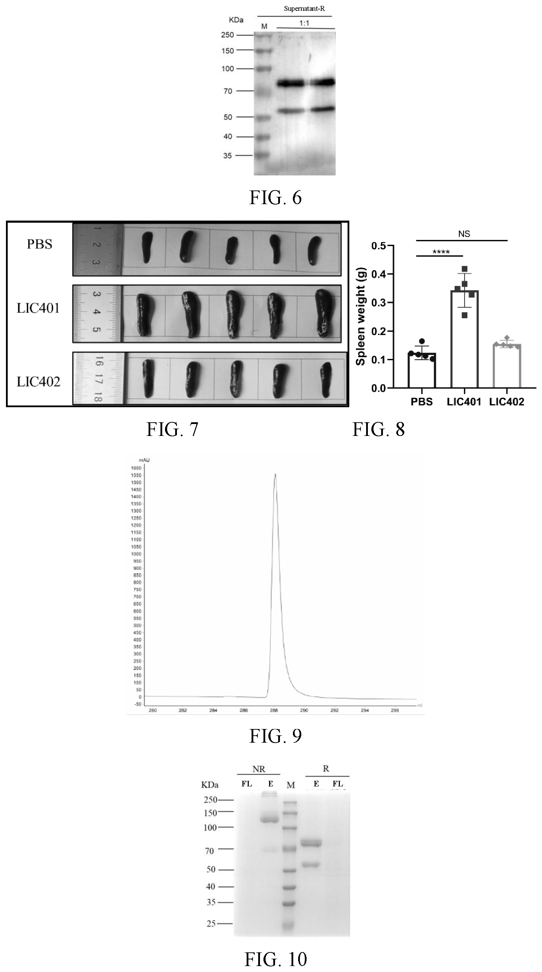

The LIC402 and LIC402-1 fusion proteins were prepared using the same method as in Example 1. The DNA fragments encoding amino acid sequences as set forth in SEQ ID NO: 23 and SEQ ID NO: 24 (LIC402), as well as those encoding amino acid sequences as set forth in SEQ ID NO: 86 and SEQ ID NO: 87 (LIC402-1) were prepared by chemical gene synthesis and PCR technology, followed by plasmid construction. As illustrated in FIG. 1, the Fc fragment located at the N-terminus in the LIC402 fusion protein was linked to IL-12 via a non-cleavable linker. IL-12 contained a P35 subunit and a P40 subunit. The elution peak of protein A affinity chromatography and Western Blot of the prepared fusion protein were illustrated in FIG. 5 and FIG. 6, respectively. An ultraviolet absorbance peak exceeding 1000 mAU was observed, indicating that the target protein was eluted by the elution buffer. The molecular weight of the reduced fusion protein was between 45 kDa and 75 kDa, with two bands.

### Example 3: Assay of Splenic Stimulatory Activity of LIC401 and LIC402 Fusion Proteins

The splenic stimulatory activity of LIC401 prepared in Example 1 and LIC402 prepared in Example 2 was evaluated by using female Balb/c mice aged 6 to 8 weeks. Mice were intravenously injected with PBS, LIC401 (0.1 mg/kg), or LIC402 (0.1 mg/kg) on day 1, day 4, and day 7. On day 9, the mice were sacrificed, and their spleens were isolated, observed, and weighed. The results were illustrated in FIG. 7 and FIG. 8. For LIC401, splenomegaly was significantly stimulated at a dose of 0.1 mg/kg, while for LIC402 fusion protein, splenomegaly was not observed apparently at a dose of 0.1 mg/kg. These results demonstrated that IL-12 located at the N-terminus of the fusion protein exhibited strong biological activity, while the activity of IL-12 located at the C-terminus of the fusion protein was masked by the steric hindrance of Fc.

### Example 4: Preparation of Fc-double-cleavable-IL-12 (LIC403) Fusion Protein

The LIC403 fusion protein, LIC403-1 fusion protein, and LIC403-2 fusion protein were prepared using the same method as in Example 1 (the only difference among the three proteins was their second structural units). The DNA fragments were prepared by chemical gene synthesis and PCR technology, followed by plasmid construction. Subsequent expression and purification steps yielded the LIC403 fusion proteins. The amino acid sequences corresponding to the LIC403 fusion protein were as set forth in SEQ ID NO: 25 and SEQ ID NO: 26; the amino acid sequences corresponding to the LIC403-1 fusion protein were as set forth in SEQ ID NO: 65 and SEQ ID NO: 66; and the amino acid sequences corresponding to the LIC403-2 fusion protein were as set forth in SEQ ID NO: 81 and SEQ ID NO: 82. As illustrated in FIG. 1, the Fc fragment located at the N-terminus in each of the LIC403 fusion protein, the LIC403-1 fusion protein, and the LIC403-2 fusion protein was linked to IL-12 via a non-cleavable linker. IL-12 contained a P35 subunit and a P40 subunit. The results found that all three fusion proteins were successfully prepared.

This Example exemplarily showed the detection results of the LIC403 fusion protein. The elution peak of protein A affinity chromatography and SDS-PAGE of the prepared LIC403 fusion protein were illustrated in FIG. 9 and FIG. 10, respectively. An ultraviolet absorbance peak exceeding 1000 mAU can be observed, indicating that the target protein was eluted by the elution buffer. The molecular weight of the reduced fusion protein was between 45 kDa and 75 kDa, with two bands.

### Example 5: Activity Assay and Safety Evaluation in vivo of LIC401 to LIC403

### 1. In Vitro Activity Assay of LIC403 Fusion Protein, LIC403-1 Fusion Protein, and LIC403-2 Fusion Protein

The fusion protein LIC403, LIC403-1, or LIC403-2 prepared in Example 4 and urokinase uPA were added to an EP tube at a mass ratio of 20:1. After cleavage in a 37°C water bath for 12 hours, 3-fold serial dilution was performed to obtain cleaved LIC403.

Spleens were isolated from normal BALB/c mice, and single-cell suspensions were prepared. Specifically, the mice were sacrificed via cervical dislocation and immersed in an alcohol cup for 5 minutes. The mice were dissected using autoclaved scissors and forceps and the spleens were removed. 4 ml of sterile RPMI 1640 medium was placed in a 35 mm culture dish. The spleens were ground using the plunger of a sterile syringe. The obtained cell suspension was centrifuged and resuspended in medium (RPMI 1640 + 10% FBS + 1% Pen/Strep + 4 mm Glutamax + 50 mm β-mercaptoethanol).

4 × 10⁵ splenocytes were seeded into each well of a 96-well plate. Serially diluted LIC403 at different concentrations, cleaved LIC403, and the control fusion protein (LIC401 prepared by using the method in Example 1 and LIC402 prepared by using the method in Example 2) were added and incubated in a 5% CO₂ incubator at 37°C for 60 hours. The 96-well plate was centrifuged at 300 × g for 10 minutes, and the cell supernatant was collected. IFNγ production was measured using a mouse IFNγ AlphaLISA kit. The results found that fusion proteins LIC403, LIC403-1, and LIC403-2 all exhibited improved safety and biological activities and can reduce systemic toxic and side effects while maintaining significant anti-tumor effects.

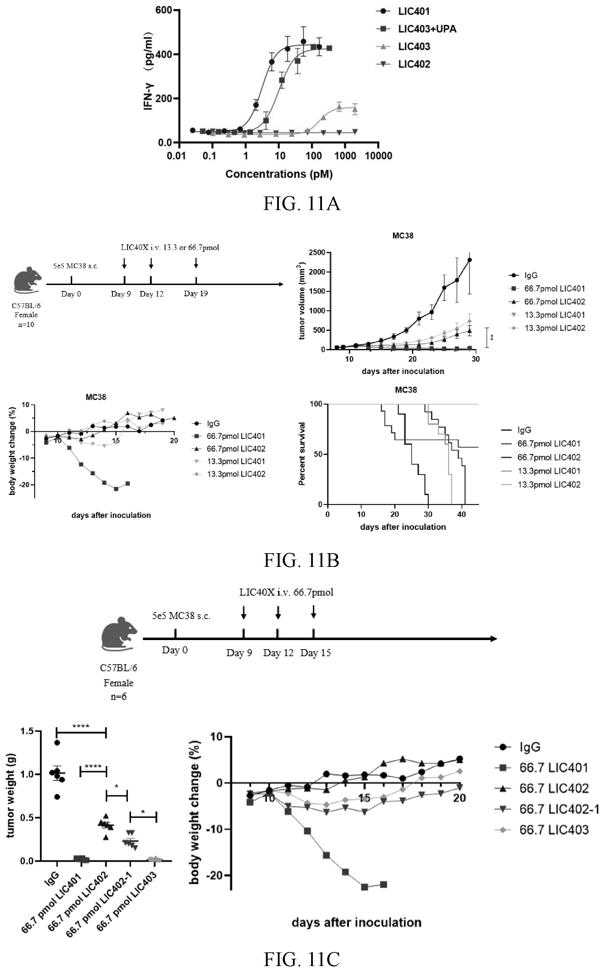

This example exemplarily showed the detection results of the LIC403 fusion protein. As illustrated in FIG. 11A, the stimulatory activity EC₅₀ of the positive control LIC401 on splenocytes was 3.10 pM, whereas negative control LIC402 showed almost no stimulatory activity. The stimulatory activity of LIC403 without urokinase cleavage was weak, with an activity reduced by at least 100-fold compared with the positive control. However, after cleavage, the EC₅₀ of LIC403 was calculated as 10.06 pM, with an activity nearly restored to the level of LIC401. These results suggested that the Fc fragment located at the N-terminus can exert a masking effect on the activity of the IL-12 cytokine and released IL-12 exhibited higher biological activity after the linker was cleaved. As a result, the LIC403 fusion protein exhibited enhanced safety and biological activity and can reduce systemic toxic and side effects while maintaining significant anti-tumor effects.

### 2. Activity Assay in vivo and Safety Evaluation in vivo of LIC401 to LIC403

The fusion protein LIC401 prepared in Example 1, the fusion proteins LIC402 and LIC402-1 prepared in Example 2, and the fusion protein LIC403 prepared in Example 4 were subjected to the following experiments:

2.1 Mouse colon cancer cells MC-38 were cultured and resuspended in PBS to 5×10⁶ cells/ml. 100 µL/cell was subcutaneously inoculated into each female C57BL/6 mouse aged 6 to 8 weeks. On day 9 after tumor bearing, the average tumor volume of the mice was approximately 75 mm³. The mice were randomly and evenly divided into 5 groups: IgG, LIC401-13.3 pmol, LIC402-13.3 pmol, LIC401-66.7 pmol, and LIC402-66.7 pmol, with 10 mice in each group. The mice in each group were administered via tail vein on days 9, 12, and 19 after tumor bearing. Tumor growth and body weight change situations of mice were continuously observed and recorded. The survival endpoint was defined as a tumor volume reaching 2000 mm³.

The test results were illustrated in FIG. 11B. In the MC38 model, LIC401 exhibited strong toxicity in terms of safety. At a dose of 66.7 pmol, mice experienced a 22% decrease in body weight, and four mice died after the second administration. At a dose of 13.3 pmol, mice experienced an approximately 5% decrease in body weight. In contrast, mice in the LIC402 group did not experience a decrease in body weight.

2.2 Mouse colon cancer cells MC-38 were cultured and resuspended in PBS to 5×10⁶ cells/ml. 100 µL/cell was subcutaneously inoculated into each female C57BL/6 mouse aged 6 to 8 weeks. On day 9 after tumor bearing, the average tumor volume of the mice was approximately 86 mm³. The mice were randomly and evenly divided into 5 groups: IgG, LIC401-66.7 pmol, LIC402-66.7 pmol, LIC403-66.7 pmol, and LIC402-1-66.7 pmol, with 6 mice in each group. The mice in each group were administered via tail vein on days 9, 12, and 15 after tumor bearing. Tumor growth and body weight change situations of mice were continuously observed and recorded. The mice were dissected and their tumor weight was measured when the average tumor volume in the IgG group reached 1000 mm³.

The test results were illustrated in FIG. 11C. In the MC38 model, mice administered with LIC402-1 at a dose of 66.7 pmol experienced an approximately 6% decrease in body weight, whereas mice administered with LIC403 at the identical dose only experienced a 4% decrease in body weight. A tumor inhibition rate of LIC402-1 was 80%, and a tumor inhibition rate of LIC403 was 99%. At the dose of 66.7 pmol, LIC402-1 showed a higher tumor inhibition rate than that of LIC402.

2.3 Mouse prostate cancer cells RM-1 were cultured and resuspended in PBS to 5×10⁶ cells/ml. 100 µL/cell was subcutaneously inoculated into each male C57BL/6 mouse aged 6 to 8 weeks. On day 10 after tumor bearing, the average tumor volume of the mice was approximately 100 mm³. The mice were randomly and evenly divided into 4 groups: IgG, LIC401, LIC402, and LIC403, with 10 mice in each group. The first intravenous tail vein administration was performed, followed by administration once every three days for a total of three administrations. The dose of each of LIC401 to LIC403 was 13.3 pmol. Tumor growth and body weight change situations of the mice were continuously observed and recorded. The survival endpoint was defined as a tumor volume reaching 2000 mm³.

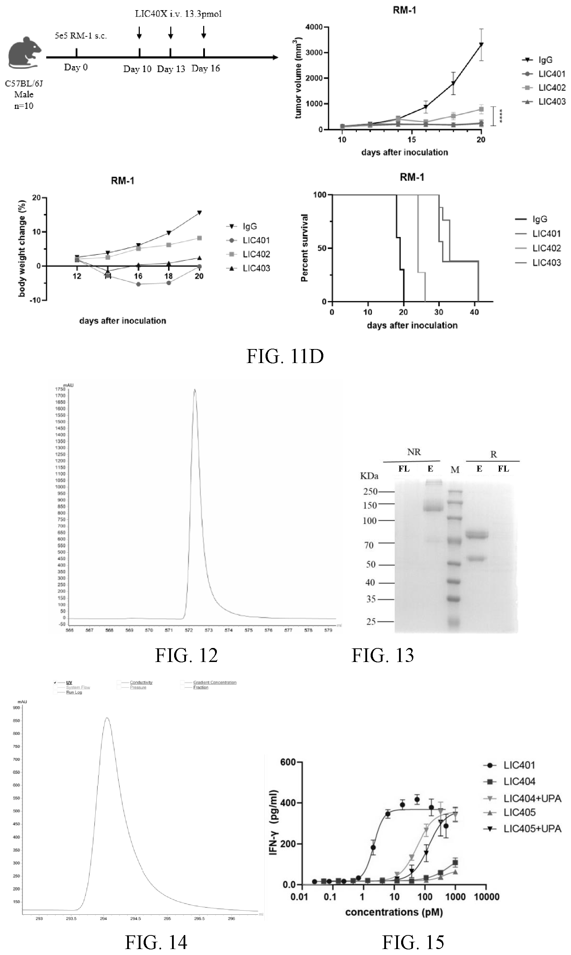

The test results were illustrated in FIG. 11D. In the RM-1 model, both LIC401 and LIC403 showed strong anti-tumor effects, with tumor inhibition rates of 92% and 93%, respectively. In terms of systemic toxicity, LIC401 caused a 5% decrease in mouse body weight, while mice in the LIC402 and LIC403 groups did not experience a decrease in body weight.

### Example 6: Preparation of Fc-single-cleavable-IL-12 (LIC404) Fusion Protein

The LIC404 fusion protein was prepared using the same method as in Example 1. The DNA fragments encoding amino acid sequences as set forth in SEQ ID NO: 23 and SEQ ID NO: 26 were prepared by chemical gene synthesis and PCR technology, followed by plasmid construction. As illustrated in FIG. 1, the Fc fragment in the LIC404 fusion protein was located at the N-terminus, a chain was linked to IL-12P35 via a non-cleavable linker and another chain was linked to IL-12P40 via a cleavable linker. The elution peak of protein A affinity chromatography and SDS-PAGE of the prepared fusion protein were illustrated in FIG. 12 and

FIG. 13, respectively. An ultraviolet absorbance peak exceeding 1000 mAU can be observed, indicating that the target protein was eluted by the elution buffer. The molecular weight of the reduced fusion protein was between 45 kDa and 75 kDa, with two bands.

### Example 7: Preparation of Fc-single-cleavable-IL-12 (LIC405) Fusion Protein

The LIC405 fusion protein was prepared using the same method as in Example 1. The DNA fragments encoding amino acid sequences as set forth in SEQ ID NO: 25 and SEQ ID NO: 24 were prepared by chemical gene synthesis and PCR technology, followed by plasmid construction. As illustrated in FIG. 1, the Fc fragment in the LIC405 fusion protein was located at the N-terminus, a chain was linked to IL-12P35 via a cleavable linker and another chain was linked to IL-12P40 via a non-cleavable linker. The elution peak of protein A affinity chromatography of the prepared fusion protein was illustrated in FIG. 14. An ultraviolet absorbance peak exceeding 800 mAU can be observed, indicating that the target protein was eluted by the elution buffer.

### Example 8: In Vitro Activity Assay of LIC404 Fusion Protein, LIC405 Fusion Protein, and Control Fusion Protein

The *in vitro* activities of the LIC404 fusion protein prepared in Example 6 and the LIC405 fusion protein prepared in Example 7 were determined using the same method as in Example 5, with LIC401 as a positive control. The results were illustrated in FIG. 15. The stimulatory activity EC₅₀ of the positive control LIC401 on splenocytes was 2.14 pM. The stimulatory activity of LIC404 without urokinase cleavage was weak, with an EC₅₀ greater than 600 pM and an activity reduced by at least 200-fold compared with the positive control. However, the activity of LIC404 was partially restored after single-chain cleavage, and its calculated EC₅₀ was 55.27 pM. LIC405 had a similar activity to that of LIC404. After enzymatic cleavage, the single chain can release a subunit, which increased the flexibility of the cytokine while retaining fusion of the Fc fragment, resulting in a significantly prolonged half-life compared to the IL-12 monomer.

### Example 9: Preparation of PDL1 mAb-double-cleavable-IL-12 (LIC407) fusion protein

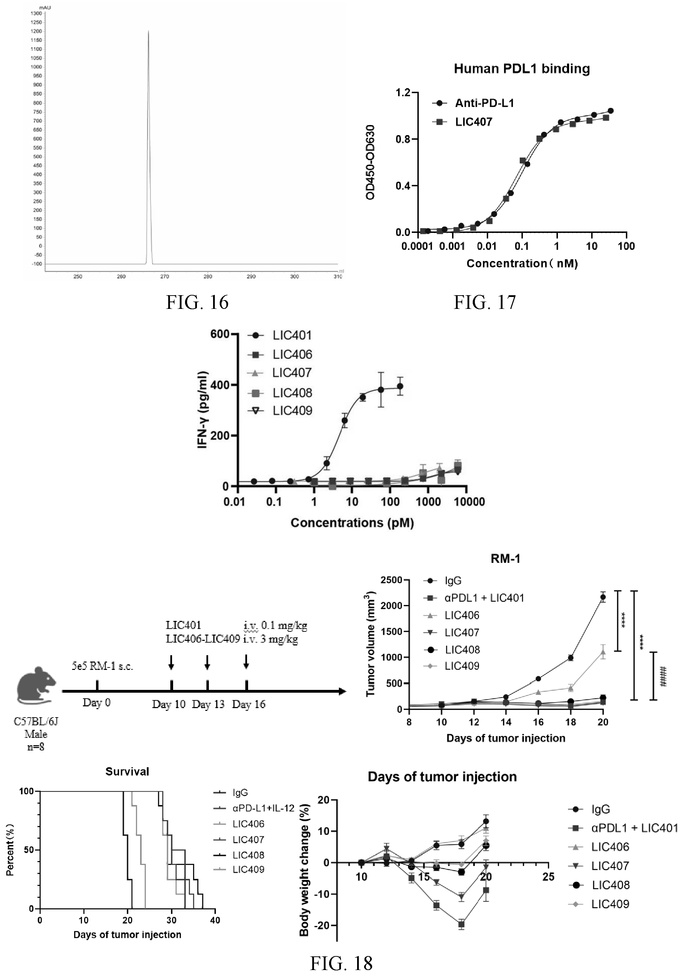

1. LIC403 can be fused with antibodies targeting different antigens to generate additional bifunctional proteins, such as anti-PD-L1 mAb. The LIC407 fusion protein was prepared using the same method as in Example 1. The DNA fragments encoding amino acid sequences as set forth in SEQ ID NO: 9, SEQ ID NO: 29, and SEQ ID NO: 30 were prepared by chemical gene synthesis and PCR technology, followed by plasmid construction. As illustrated in FIG. 1, the PDL1 antibody located at the N-terminus in the LIC407 fusion protein was linked to IL-12P35 and IL-12P40 via a cleavable linker. The elution peak of protein A affinity chromatography of the prepared fusion protein was illustrated in FIG. 16. An ultraviolet absorbance peak exceeding 1000 mAU can be observed, indicating that the target protein was eluted by the elution buffer.
2. Affinity Assay of PD-L1 mAb or LIC407 to PD-L1 Antigen via ELISA
   1) Human PD-L1 antigen was coated. PD-L1 was diluted to 1.0 µg/mL using ELISA coating solution. 100 µL was added to each well of a 96-well plate and coated overnight at 4°C.
   2) Blocking, the coating solution was discarded and the plate was washed four times with PBST. 250 µL per well of blocking solution (5% BSA in PBST) was added and incubated at room temperature for 2 h.
   3) Incubation with primary antibody, the blocking solution was discarded and the plate was washed four times with PBST. The serially diluted PD-L1 mAb or LIC407 fusion protein was added to each well at a volume of 100 µL per well and incubated at room temperature for 2 h.
   4) Incubation with secondary antibody, the primary antibody was discarded and the plate was washed four times with PBST. The HRP-conjugated secondary antibody (goat anti-human IgG (H+L)) was diluted with PBST at a ratio of 1:10,000, added to each well at a volume of 100 µL/well, and incubated at room temperature for 1 h.
   5) Color development, TMB substrate solution was added to each well at a volume of 100 µL and incubated at 37°C in the dark for 5 min. The reaction was terminated by adding 50 µL of stop solution (2M H₂SO₄) to each well. Absorbance was measured at 450 nm. Data were processed using GraphPad 8.0, as illustrated in FIG. 17. It can be seen that, fusion of PD-L1 mAb with IL-12 still retained a high affinity to PD-L1 comparable to that of the PDL1 mAb.

### Example 10: Preparation of PDL1 mAb-non-(single-) cleavable IL-12 (LIC406, LIC408, LIC409) Fusion Proteins

The LIC406, LIC408, and LIC409 fusion proteins were prepared using the same method as in Example 1. The DNA fragments were prepared by chemical gene synthesis and PCR technology, followed by plasmid construction. The fusion proteins were obtained after expression and purification. The amino acid sequences corresponding to the LIC406 fusion protein were as set forth in SEQ ID NO: 27, SEQ ID NO: 28, and SEQ ID NO: 29; the amino acid sequences corresponding to the LIC408 fusion protein were as set forth in SEQ ID NO: 27, SEQ ID NO: 30, and SEQ ID NO: 9; and the amino acid sequences corresponding to the LIC409 fusion protein were as set forth in SEQ ID NO: 29, SEQ ID NO: 28, and SEQ ID NO: 9. As illustrated in FIG. 1, the PDL1 mAb located at the N-terminus in the LIC406 fusion protein was linked to IL-12P35 and IL-12P40 via a non-cleavable linker. The PDL1 monoclonal antibody in the LIC408 fusion protein was located at the N-terminus, a chain was linked to IL-12P35 via a non-cleavable linker and another chain was linked to IL-12P40 via a cleavable linker. The PDL1 mAb in the LIC409 fusion protein was located at the N-terminus, a chain was linked to IL-12P35 via a cleavable linker and another chain was linked to IL-12P40 via a non-cleavable linker.

### Example 11: Activity Assay and Safety Evaluation in Vivo of LIC406, LIC407, LIC408, and LIC409 Fusion Proteins

### 1. In Vitro Activity Assay of LIC406, LIC407, LIC408, and LIC409 Fusion Proteins

The *in vitro* activities of the LIC406, LIC407, LIC408, and LIC409 fusion proteins prepared in Example 9 or Example 10 were determined using the same method as in Example 5, with LIC401 as a positive control. The results were illustrated in FIG. 18. Compared with the positive control, LIC401, the LIC407, LIC408, and LIC409 fusion proteins exhibited weak stimulatory activities on splenocytes, with all EC₅₀ greater than 400 pM. This indicated that the PDL1 mAb located at the N-terminus of IL-12 can mask the activity of IL-12. Molecules of this format can also enhance the targeting of IL-12.

### 2. In Vivo Activity Assay and In Vivo Safety Evaluation of LIC406, LIC407, LIC408, and LIC409

The fusion protein LIC401+αPDL1 prepared in Example 1, as well as LIC406, LIC407, LIC408, and LIC409 prepared in Example 9 or Example 10 using the same method as in Example 5, were subjected to the following experiments:

Mouse prostate cancer cells RM-1 were cultured and resuspended in PBS to 5×10⁶ cells/ml. 100 µL/cell was subcutaneously inoculated into each male C57BL/6 mouse aged 6 to 8 weeks. On day 10 after tumor bearing, the average tumor volume of the mice was approximately 100 mm³. The mice were randomly and evenly divided into 6 groups: IgG, LIC401+αPDL1, LIC406, LIC407, LIC408, and LIC409, with 8 mice in each group. The first intravenous administration was performed via the tail vein, followed by administration once every three days for a total of three administrations. The dose of LIC401 was 0.1 mg/kg, and the dose of each of αPDL1, LIC406, LIC407, LIC408, and LIC409 was 3 mg/kg. Tumor growth and body weight change situations of the mice were continuously observed and recorded. The survival endpoint was defined as a tumor volume reaching 2000 mm³.

The test results were illustrated in FIG. 18. In terms of safety reflected by body weight changes, LIC401 at 0.1 mg/kg induced a significant decrease of approximately 20% in the body weight of mice. LIC406 can markedly improve the safety of IL-12. By introducing different linkers, a balance between safety and anti-tumor efficacy can be achieved. The body weight loss caused by LIC407 was reduced to 10%, whereas LIC408 and LIC409 caused almost no decrease in the body weight of mice.

### Example 12: Preparation of PDL1-ScFv-Fc-double (single)-cleavable-IL-12 (LIC412, LIC413, and LIC414) Fusion Proteins

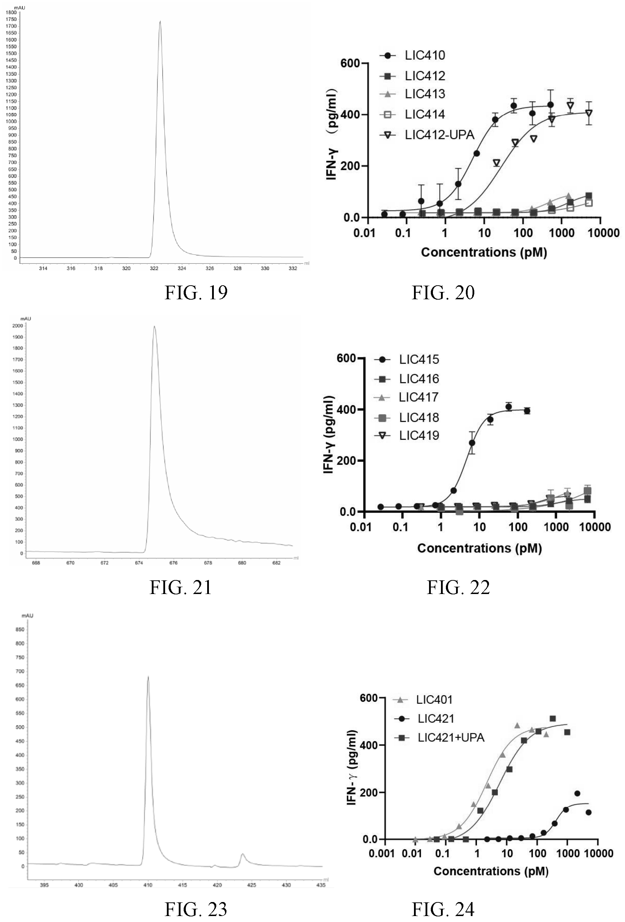

The LIC412, LIC413, and LIC414 fusion proteins were prepared using the same method as in Example 1. The DNA fragments were prepared by chemical gene synthesis and PCR technology, followed by plasmid construction. The fusion proteins were obtained after expression and purification. The amino acid sequences corresponding to the LIC412 fusion protein were as set forth in SEQ ID NO: 35 and SEQ ID NO: 36; the amino acid sequences corresponding to the LIC413 fusion protein were as set forth in SEQ ID NO: 33 and SEQ ID NO: 36; and the amino acid sequences corresponding to the LIC414 fusion protein were as set forth in SEQ ID NO: 35 and SEQ ID NO: 34. As illustrated in FIG. 1, the PDL1-ScFv-Fc fragment in the LIC412, LIC413 and LIC414 fusion proteins was located at the N-terminus and linked to IL-12P35 and IL-12P40 via a cleavable linker (or a non-cleavable linker). The elution peak of protein A affinity chromatography of the prepared LIC412 fusion protein was illustrated in FIG. 19. An ultraviolet absorbance peak exceeding 1000 mAU can be observed, indicating that the target protein was eluted by the elution buffer.

### Example 13: Activity Assay of LIC412 and Its Control Fusion Proteins

The *in vitro* activities of the LIC412, single-cleavable LIC413, and LIC414 fusion proteins prepared in Example 12 were determined using the same method as in Example 5, with LIC401 as a positive control and LIC411 as a negative control. The results were illustrated in FIG. 20. Compared with the positive control, LIC410, the LIC412, LIC413, and LIC414 fusion proteins exhibited weak stimulatory activities on splenocytes, and their EC₅₀ was significantly lower than that of the positive control. The activity of LIC412 was restored after cleavage by urokinase, indicating that the ScFv + Fc combination located at the N-terminus of IL-12 can mask the activity of IL-12.

### Example 14: Preparation of PD1/PDL1-ScFv-Fc-double (single)-cleavable-IL-12 (LIC417, LIC418, and LIC419) Fusion Proteins

The LIC417, LIC418, and LIC419 fusion proteins were prepared using the same method as in Example 1. The DNA fragments were prepared by chemical gene synthesis and PCR technology, followed by plasmid construction. The fusion proteins were obtained after expression and purification. The amino acid sequences corresponding to the LIC417 fusion protein were as set forth in SEQ ID NO: 35 and SEQ ID NO: 39, the amino acid sequences corresponding to the LIC418 fusion protein were as set forth in SEQ ID NO: 33 and SEQ ID NO: 39, and the amino acid sequences corresponding to the LIC419 fusion protein were as set forth in SEQ ID NO: 35 and SEQ ID NO: 38. As illustrated in FIG. 1, the PD1/PDL1-ScFv-Fc fragment in the LIC417, LIC418, and LIC419 fusion proteins was located at the N-terminus and linked to IL-12P35 and IL-12P40 via a cleavable linker (or a non-cleavable linker). The elution peak of protein A affinity chromatography of the prepared LIC417 fusion protein was illustrated in FIG. 21. An ultraviolet absorbance peak exceeding 1000 mAU can be observed, indicating that the target protein was eluted by the elution buffer.

### Example 15: Activity Assay of LIC417 and Its Control Fusion Proteins

The *in vitro* activities of the LIC417, single-cleavable LIC418, and LIC419 fusion proteins prepared in Example 14 were determined using the same method as in Example 5, with LIC415 as a positive control and LIC416 as a negative control. The results were illustrated in FIG. 22. Compared with the positive control, LIC415, LIC417, LIC418, and LIC419 fusion proteins exhibited weak stimulatory activities on splenocytes. This indicated that ScFv + Fc of different targets located at the N-terminus of IL-12 can mask the activity of IL-12. Molecules of this format can also enhance the targeting of IL-12.

### Example 16: Preparation of PDL1-(Fab)₂-double (single)-cleavable-IL-12-Fc (LIC421, LIC422, and LIC423) Fusion Proteins

The LIC421, LIC422, and LIC423 fusion proteins were prepared using the same method as in Example 1. The DNA fragments were prepared by chemical gene synthesis and PCR technology, followed by plasmid construction. The fusion proteins were obtained after expression and purification. The amino acid sequences corresponding to the LIC421 fusion protein were as set forth in SEQ ID NO: 9, SEQ ID NO: 42, and SEQ ID NO: 43, the amino acid sequences corresponding to the LIC422 fusion protein were as set forth in SEQ ID NO: 9, SEQ ID NO: 40, and SEQ ID NO: 43, and the amino acid sequences corresponding to the LIC423 fusion protein were as set forth in SEQ ID NO: 9, SEQ ID NO: 42, and SEQ ID NO: 41. As illustrated in FIG. 1, the PDL1-Fab fragment in the LIC421, LIC422, and LIC423 fusion proteins was located at the N-terminus, the Fc fragment was located at the C-terminus, and the PDL1-Fab and Fc were linked to IL-12P35 and IL-12P40 via a cleavable linker (or a non-cleavable linker). The elution peak of protein A affinity chromatography of the prepared LIC421 fusion protein was illustrated in FIG. 23. An ultraviolet absorbance peak exceeding 500 mAU can be observed, indicating that the target protein was eluted by the elution buffer.

### Example 17: Activity Assay of PDL1-(Fab)₂-double-cleavable-IL-12-Fc (LIC421) Fusion Protein

The *in vitro* activity of the LIC421 fusion protein prepared in Example 16 was determined using the same method as in Example 5, with LIC401 as a positive control. The results were illustrated in FIG. 24. The stimulatory activity EC₅₀ of the positive control LIC401 on splenocytes was 2.2 pM. The stimulatory activity of LIC421 without urokinase cleavage was weak, with an EC₅₀ greater than 400 pM and an activity reduced by at least 200-fold compared with the positive control. This demonstrated that Fab can mask the activity of IL-12, similar to the steric hindrance formed by the hinge of Fc. The activity of LIC421 was almost completely restored after UPA cleavage, and its calculated EC₅₀ was 5.8 pM. Molecular formats where IL-12 was located in the middle of the fusion protein exhibited a high masking activity, and its activity was completely restored after cleavage. In addition, the Fc fusion was retained after cleavage, which can prolong the half-life of the cytokine and exert a more sustained therapeutic effect.

### Example 18: Activity Assay of PDL1-(Fab)₂-single-cleavable-IL-12-Fc (LIC422 and LIC423) Fusion Proteins

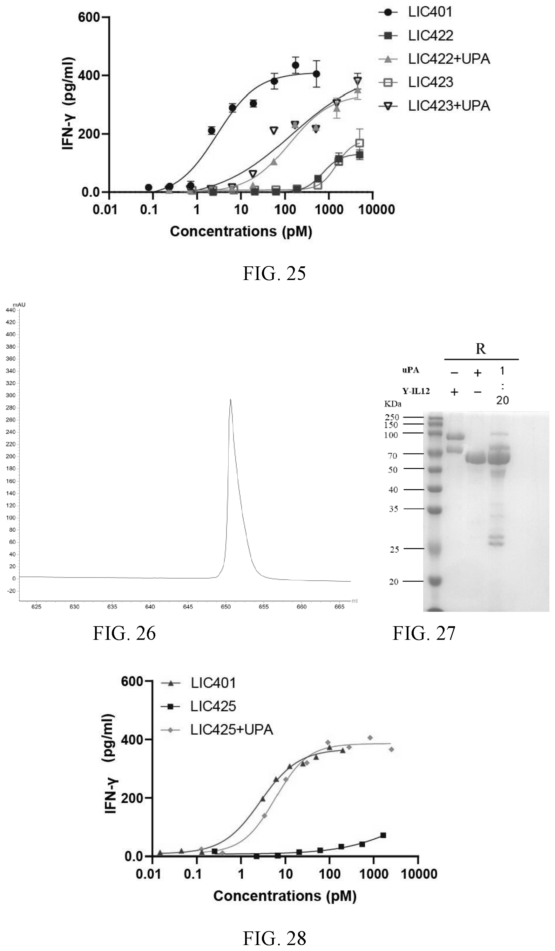

The *in vitro* activities of the LIC422 and LIC423 fusion proteins prepared in Example 16 were determined using the same method as in Example 5, with LIC401 as a positive control. The results were illustrated in FIG. 25. The stimulatory activities of LIC422 and LIC423 cleaved by urokinase were partially restored compared with that before cleavage, but were weaker than that of the positive control. This demonstrated that Fab can mask the activity of IL-12, similar to the steric hindrance formed by the hinge of the Fc. Single Fab fusion was retained after cleavage, which is still targeted and can prolong the half-life of cytokines, and exert a more sustained therapeutic effect.

### Example 19: Preparation of PDL1-Fab-double (single/non)-cleavable-IL-12-Fc (LIC424, LIC425, LIC426, and LIC427) Fusion Proteins

The LIC424, LIC425, LIC426, and LIC427 fusion proteins were prepared using the same method as in Example 1. The DNA fragments were prepared by chemical gene synthesis and PCR technology, followed by plasmid construction. The fusion proteins were obtained after expression and purification. The amino acid sequences corresponding to the LIC425 fusion protein were as set forth in SEQ ID NO: 44 and SEQ ID NO: 40, the amino acid sequences corresponding to the LIC425 fusion protein were as set forth in SEQ ID NO: 43 and SEQ ID NO: 45, the amino acid sequences corresponding to the LIC426 fusion protein were as set forth in SEQ ID NO: 43 and SEQ ID NO: 44, and the amino acid sequences corresponding to the LIC427 fusion protein were as set forth in SEQ ID NO: 40 and SEQ ID NO: 45. As illustrated in FIG. 1, in the LIC424, LIC425, LIC426, and LIC427 fusion proteins, the VL and VH (single Fab) fragments were located at the N-terminus, the Fc fragment was located at the C-terminus, and single Fab and the Fc were linked to IL-12P35 and IL-12P40 via a cleavable linker (or a non-cleavable linker). The disulfide bonds between VL and VH and the disulfide bonds between Fc-Hinge can be used to form upper and lower "locks" to mask the activity of IL-12. The elution peak of protein A affinity chromatography of the prepared LIC425 fusion protein was illustrated in FIG. 26. An ultraviolet absorbance peak exceeding 300 mAU can be observed, indicating that the target protein was eluted by the elution buffer.

### Example 20: In Vitro Enzymatic Cleavage of LIC425 Fusion Protein

The fusion protein LIC425 prepared in Example 19 and urokinase uPA were added to an EP tube at a mass ratio of 20:1, where the mass of LIC425 was 10 µg, and the mass of the urokinase was 0.5 µg. Then, the volume was made up to 20 µL with PBS. A non-cleaved protein group and a UPA group were set as controls. The mass of the corresponding components of the control group and the cleavage system was consistent. Cleavage was performed at 37°C for 12 h, and the cleavage situations were verified by SDS-PAGE. The results were illustrated in FIG. 27. Under this condition, more than 85% of LIC425 can be cleaved by urokinase. Theoretically, the molecular weight of Y-IL12-uPA-VH-single chain was 87.2 kDa, and the molecular weight of Y-IL12-uPA-VL-single chain was 73.8 kDa. After cleavage, the molecular weight of the fragment VL-CL ranged from 24.3 to 25.1 KDa, the molecular weight of P35-Fc-Knob ranged from 48.7 to 49.6 KDa, the molecular weight of VH-CH1 ranged from 22.9 to 23.8 KDa, and the molecular weight of P40-Fc-Hole ranged from 62.5 to 63.4 KDa. The SDS-PAGE results showed that the molecular weights of the uncleaved LIC425 fusion protein and cleaved fragments were consistent with the theoretical values. Because the purchased uPA was medicinal urokinase and contained a large amount of albumin, the band in the picture was thicker.

### Example 21: In Vitro Activity Assay of LIC425 Fusion Protein

The *in vitro* activity of the LIC425 fusion protein prepared in Example 19 was determined using the same method as in Example 5, with LIC401 as a positive control. The results were illustrated in FIG. 28. The stimulatory activity EC₅₀ of the positive control LIC401 on splenocytes was 2.77 pM. The stimulatory activity of LIC425 without urokinase cleavage was weaker, with an EC₅₀ greater than 400 pM, which was larger than the EC₅₀ of LIC421. This demonstrated that the upper and lower "locks" formed by the disulfide bonds between VL and VH and the disulfide bonds between the Fc-Hinge had a better masking effect on the activity of IL-12 than the steric hindrance of the two Fabs. The activity of LIC425 was almost completely restored after UPA cleavage, and its calculated EC₅₀ was 5.96 pM. The heterologous molecule of IL-12 located in the middle of the fusion protein exhibited a high masking activity and good activity recovery after cleavage. In addition, the Fc fusion was restored after cleavage, which can prolong the half-life of the cytokine.

### Example 22: Affinity Assay of PD-L1 mAb or LIC425 to PD-L1 Antigen via ELISA

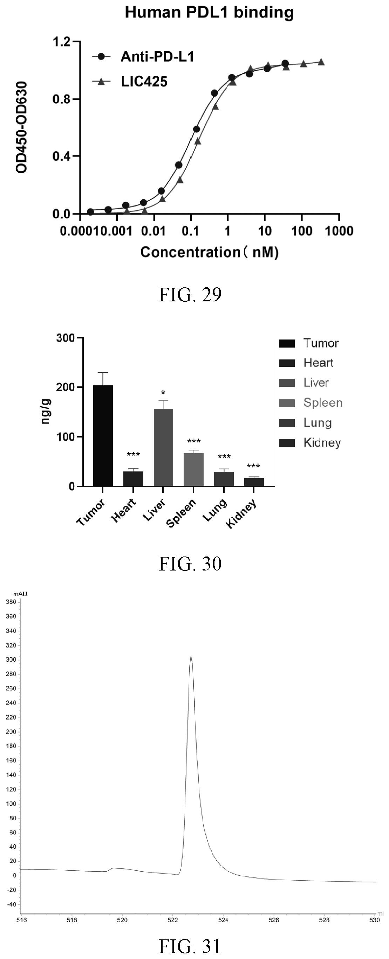

The affinity of a single Fab in the LIC425 fusion protein prepared in Example 19 to the antigen was determined using the same method as in Example 9, with PDL1 mAb as a positive control. The results were illustrated in FIG. 29. The affinity Kd value of the PDL1 mAb to the PDL1 antigen was 0.1 nM, and the affinity Kd value to the PD-L1 antigen after fusion with IL-12 was 0.17 nM. There was no significant difference between the two values, indicating that the single Fab form still allowed the LIC425 fusion protein to have a targeting ability comparable to PDL1.

### Example 23: ELISA Determination of Tissue Targeting of LIC425 Fusion Protein

C57BL/6N mice aged 6 to 8 weeks were subcutaneously inoculated with B16F10 melanoma. When the tumor volume reached 800 mm³, 3 mg/kg of the LIC425 fusion protein prepared in Example 19 was injected via a single tail vein. 12 hours later, blood was collected from the heart, the abdominal vein was transected, the heart was perfused with 40 mL of PBS until the lungs turned whitish, and the blood color of the liver faded away (turned yellow). The heart, liver, spleen, lungs, kidneys, and tumors were collected, and the total weight was weighed. 50 mg to 100 mg of the collected tissue was cut to prepare a 10% tissue homogenate. Three mice were used in total. The blood drug concentration in each tissue was detected by ELISA. The results, as illustrated in FIG. 30, indicated that the distribution of LIC425 in tissue exhibited certain specificity, LIC425 had a low content in the heart, lungs, and kidneys, indicating strong tumor targeting and mainly distributed in the tumor tissue, but a small amount was also enriched in the liver and spleen. Specifically, the concentration of LIC45 in the tumors was 6.56-fold that in the heart, 1.30-fold that in the liver, 3.08-fold that in the spleen, 6.86-fold that in the lungs, and 11.9-fold that in the kidneys. In summary, the LIC425 molecule, that is, IL-12 fused with the PDL1 antibody Fab, exhibited excellent tumor-targeting ability, which was beneficial for the efficacy of anti-tumor drugs.

### Example 24: Preparation of PDL1-Fab-double-cleavable-IL-12-Fc (LIC425-1) Fusion Protein

In the LIC425 fusion protein prepared in Example 19, the VL and VH of the PDL1 antibody were replaced with the VL and VH of the PD1 antibody, and the cleavable linker of the urokinase substrate in LIC425 was replaced with the cleavable linker of the matrix metalloproteinase substrate, thereby regenerating a new fusion protein LIC425-1. The LIC425-1 fusion protein was prepared using the same method as in Example 1. The DNA fragments encoding the amino acid sequences as set forth in SEQ ID NO: 67 and SEQ ID NO: 68 were prepared by chemical gene synthesis and PCR technology, followed by plasmid construction. The schematic diagram of LIC425-1 was the same as that of the LIC425 fusion protein molecule in FIG. 1. The VL and VH (single Fab) fragments were located at the N-terminus, Fc was located at the C-terminus, and Fab and Fc were linked to IL-12P35 and IL-12P40 via a cleavable linker. The disulfide bond between the VL and VH and the disulfide bond between Fc-Hinge can be used to form upper and lower "locks" to mask the activity of IL-12. The elution peak of protein A affinity chromatography of the prepared fusion protein was illustrated in FIG. 31. An ultraviolet absorbance peak exceeding 300 mAU can be observed, indicating that the target protein was eluted by the elution buffer.

### Example 25: In Vivo Safety Evaluation of LIC425-1 Fusion Protein

The *in vitro* activity assay results of the LIC425 cell in Example 21 showed that the activity of IL-12 in this structure was weakened, suggesting that LIC425-like molecules may have relatively low peripheral immune stimulatory activity and systemic toxic and side effects. Therefore, compared with the positive control drug LIC401, the safety in vivo of LIC425-1, one of the LIC425-like molecules prepared in Example 24, was evaluated in normal C57BL/6 mice. The specific steps were as follows:

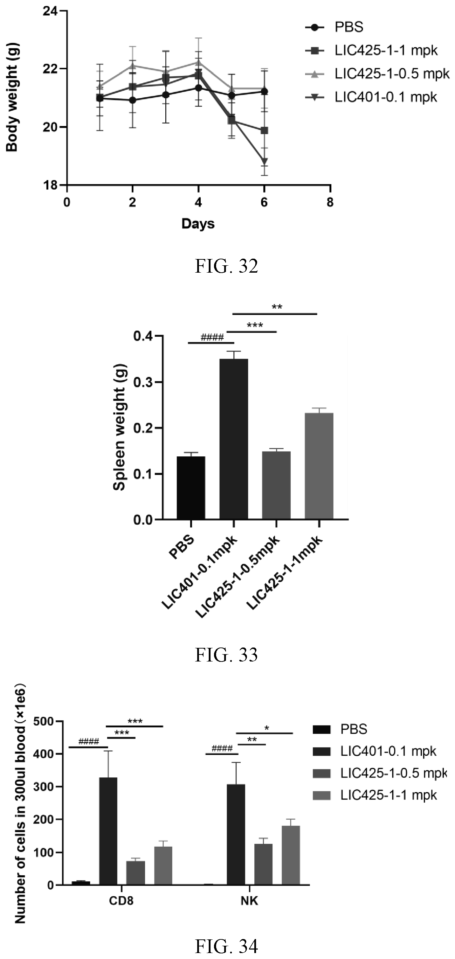

In this example, C57BL/6N mice were selected and divided into 4 groups with four mice per group, as illustrated in FIG. 32. The mice in all four groups were administered the drug via tail vein on day 1 and day 4 (the type, dosage, and route of administration for each group were illustrated in FIG. 32), and their body weight was monitored. The body weight results were illustrated in FIG. 32. LIC401, administered twice at a dose of 0.1 mg/kg, induced severe weight loss, slowed movement, and frizzy hair in the mice. In contrast, LIC425 exerted no effect on mouse body weight at a dose of 0.5 mg/kg, and induced weight loss in mice at a dose of 1 mg/kg, with a smaller magnitude of weight loss compared with the LIC401 fusion protein at 0.1 mg/kg.

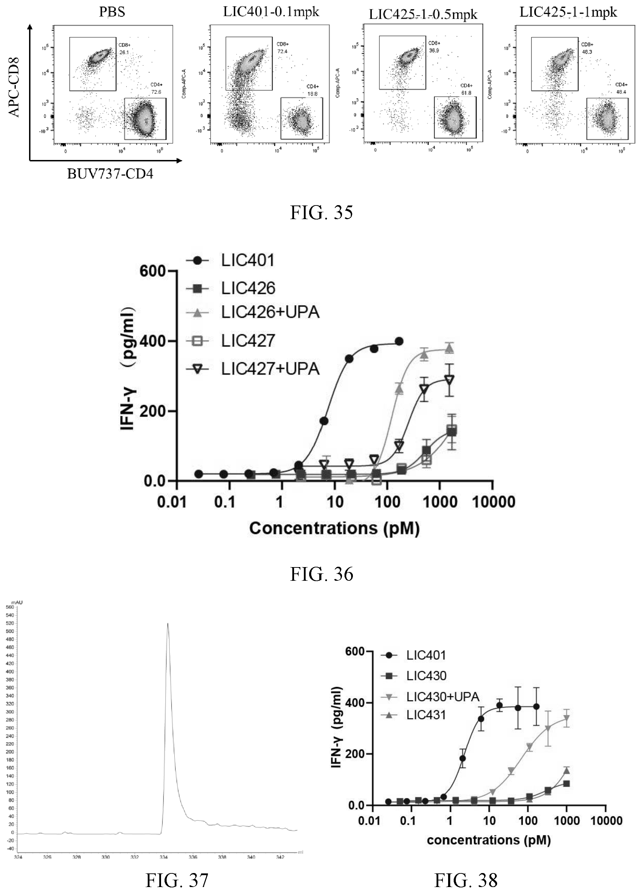

To further investigate the underlying mechanism of the improved safety of LIC425-1, mice were sacrificed on day 6, and spleens and peripheral blood were collected for analysis. The splenic stimulation results, as illustrated in FIG. 33, indicated that the spleen weight in the LIC401 group was significantly higher than that in the PBS group, while the spleen weight in the LIC425-1 group was significantly lower than that in the positive control LIC401 group. This demonstrated that the single Fab fragment fused with the PD1 antibody can significantly mask the immune stimulatory activity of IL-12 in the peripheral blood. Correspondingly, flow cytometry analysis results, as illustrated in FIG. 34, showed that in peripheral blood, the stimulation of CD8⁺ T cells and NK cells by LIC425 molecules at doses of 0.5 mg/kg and 1 mg/kg was significantly lower than that of the IL12-Fc (LIC401) group at a dose of 0.1 mg/kg. Consistently, as illustrated in FIG. 35, the stimulation of CD8⁺ T cells by LIC425-1 molecules in the spleen was also significantly weaker than that of LIC401 at a dose of 0.1 mg/kg.

### Example 26: Activity Assay of PDL1-Fab-single-cleavable-IL-12-Fc (LIC426 and LIC427) Fusion Proteins

The *in vitro* activities of the LIC426 and LIC427 fusion proteins prepared in Example 19 were determined using the same method as in Example 5, with LIC401 as a positive control. The results were illustrated in FIG. 36. Compared with the positive control, LIC401, the LIC426 and LIC427 fusion proteins exhibited weaker stimulatory activities on splenocytes, but their activities were recovered after cleavage by urokinase.

### Example 27: Preparation of PDL1-(Fab)₂-double (single)-cleavable-IL-12 tandem-Fc (LIC430 and LIC431) Fusion Proteins

The LIC430 and LIC431 fusion proteins were prepared using the same method as in Example 1. The DNA fragments were prepared by chemical gene synthesis and PCR technology, followed by plasmid construction. The fusion proteins were obtained after expression and purification. The amino acid sequences corresponding to the LIC430 fusion protein were as set forth in SEQ ID NO: 9 and SEQ ID NO: 48, and the amino acid sequences corresponding to the LIC431 fusion protein were as set forth in SEQ ID NO: 9, SEQ ID NO: 49, and SEQ ID NO: 50. As illustrated in FIG. 1, in the LIC430 and LIC431 fusion proteins, the Fab fragment was located at the N-terminus, the Fc fragment was located at the C-terminus, and the Fab fragment and the Fc were linked to IL-12P70 via a cleavable linker (or a non-cleavable linker). One molecule of LIC430 contained two molecules of IL-12P70. The elution peak of protein A affinity chromatography of the prepared LIC430 fusion protein was illustrated in FIG. 37. An ultraviolet absorbance peak exceeding 500 mAU can be observed, indicating that the target protein was eluted by the elution buffer.

### Example 28: Activity Assay of LIC430 and Control Fusion Proteins

The *in vitro* activities of the LIC430 and single-cleavable LIC431 fusion proteins prepared in Example 27 were determined using the same method as in Example 5, with LIC401 as a positive control. The results were illustrated in FIG. 38. Compared with the positive control, LIC401, the LIC430 and LIC431 fusion proteins exhibited weaker stimulatory activities on splenocytes. After cleavage by urokinase, the activity of LIC430 was recovered, but lower than that of monovalent IL12. This indicated that the activity of heteromeric IL12 (with subunits of IL-12 arranged in parallel) was superior to that of homomeric IL-12 (with subunits of IL-12 arranged in tandem).

### Example 29: Preparation of PDL1-Fab-double (single)-cleavable-IL-12 tandem-Fc (LIC433, LIC434, and LIC435) Proteins

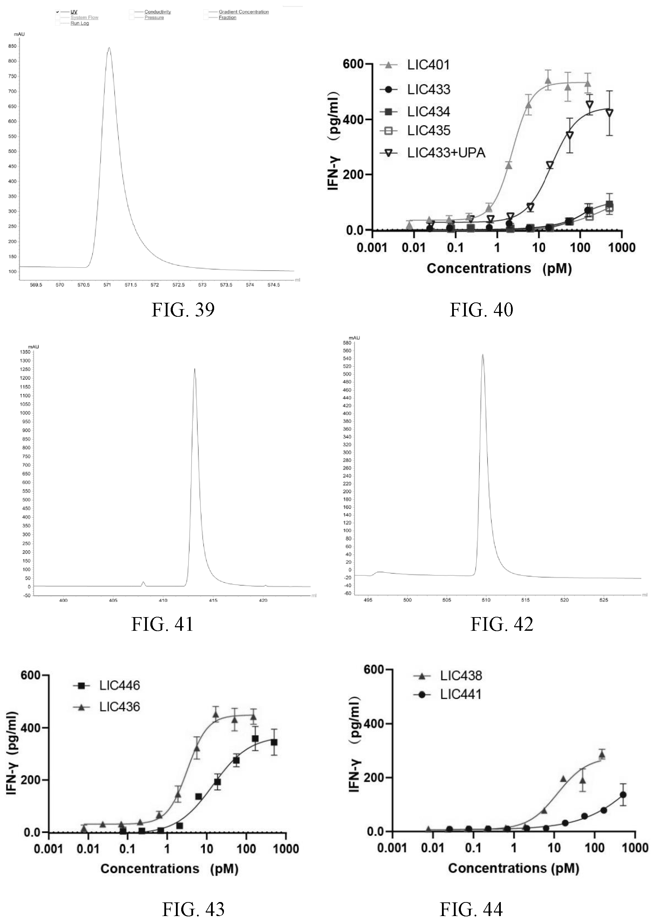

The LIC433, LIC434, and LIC435 fusion proteins were prepared using the same method as in Example 1. The DNA fragments were prepared by chemical gene synthesis and PCR technology, followed by plasmid construction. The fusion proteins were obtained after expression and purification. The amino acid sequences corresponding to the LIC433 fusion protein were as set forth in SEQ ID NO: 53 and SEQ ID NO: 50, the amino acid sequences corresponding to the LIC434 fusion protein were as set forth in SEQ ID NO: 53 and SEQ ID NO: 52, and the amino acid sequences corresponding to the LIC435 fusion protein were as set forth in SEQ ID NO: 51 and SEQ ID NO: 50. As illustrated in FIG. 1, in the LIC433, LIC434, and LIC435 fusion proteins, the Fab fragment was located at the N-terminus, the Fc fragment was located at the C-terminus, and the Fab fragment and the Fc was linked to IL-12P70 via a cleavable linker. One molecule of LIC433 contained two molecules of IL-12P70. The elution peak of protein A affinity chromatography of the prepared LIC433 fusion protein was illustrated in FIG. 39. An ultraviolet absorbance peak exceeding 500 mAU can be observed, indicating that the target protein was eluted by the elution buffer.

### Example 30: Activity Assay of LIC433 and Control Fusion Proteins

The *in vitro* activities of the LIC433, single-chain cleavable linker-formatted LIC434, and LIC435 fusion proteins prepared in Example 29 were determined using the same method as in Example 5, with LIC401 used as a positive control. The results were illustrated in FIG. 40. Compared with the positive control LIC401, the LIC433 fusion protein exhibited weaker stimulatory activity on spleen splenocytes. In addition, its activity was partially recovered after cleavage by urokinase, but lower than that of monovalent IL12.

### Example 31: Preparation of LIC436 Fusion Protein

The LIC436 fusion protein was prepared using the same method as in Example 1. The DNA fragments encoding amino acid sequences as set forth in SEQ ID NO: 9, SEQ ID NO: 12, and SEQ ID NO: 54 were prepared by chemical gene synthesis and PCR technology, followed by plasmid construction. As illustrated in FIG. 1, the PDL1 mAb was directly linked to IL-12P70 via a non-cleavable linker, with the P40 subunit of IL-12P70 on the outside. The elution peak of protein A affinity chromatography of the prepared fusion protein was illustrated in FIG. 41. An ultraviolet absorbance peak exceeding 1000 mAU can be observed, indicating that the target protein was eluted by the elution buffer.

### Example 32: Preparation of LIC444 Fusion Protein

A novel fusion protein LIC444 was formed by modifying the N-terminus-P35 subunit-P40 subunit-C-terminus in IL-12P70 in LIC436 to the N-terminus-P40 subunit-P35 subunit-C-terminus. The DNA fragments encoding amino acid sequences as set forth in SEQ ID NO: 9, SEQ ID NO: 12, and SEQ ID NO: 69 were prepared by chemical gene synthesis and PCR technology, followed by plasmid construction. As illustrated in FIG. 1, the PDL1 mAb was directly linked to IL-12P70 via a non-cleavable linker, with the P35 subunit of IL-12P70 on the outside. The elution peak of protein A affinity chromatography of the prepared fusion protein was illustrated in FIG. 42. An ultraviolet absorbance peak exceeding 500 mAU can be observed, indicating that the target protein was eluted by the elution buffer.

### Example 33: Activity Assay of LIC436 and LIC444 Fusion Proteins

The *in vitro* activities of the LIC436 fusion protein prepared in Example 31 and the LIC444 fusion protein prepared in Example 32 were determined using the same method as in Example 5. The results were illustrated in FIG. 43. Compared with the positive control LIC401, the proteins LIC436 and LIC444, which were fused with the PDL1 mAb, exhibited slightly weaker stimulatory activities on splenocytes, but the activities of LIC436 and LIC444 were comparable.

### Example 34: Preparation and Activity Assay of LIC438 and LIC441 Fusion Proteins

1. The LIC438 and LIC441 fusion proteins were prepared using the same method as in Example 1. DNA fragments were prepared using chemical gene synthesis and PCR technology, followed by plasmid construction. The fusion proteins were obtained after expression and purification. The amino acid sequences corresponding to the LIC438 fusion protein were as set forth in SEQ ID NO: 56 and SEQ ID NO: 57, and the amino acid sequences corresponding to the LIC441 fusion protein were as set forth in SEQ ID NO: 59 and SEQ ID NO: 61. As illustrated in FIG. 1, in the structures of the LIC438 and LIC441 fusion proteins, in one chain, the PDL1 single-chain antibody was directly linked to the Fc via a non-cleavable linker, and in the other chain, the PDL1 (i.e., LIC438) or PD1 (i.e., LIC441) single-chain antibody and Fc were linked via IL-12P70. IL-12P70 was linked to the PDL1 single-chain antibody and the Fc via a non-cleavable linker, with IL-12P70 in the middle.
2. The *in vitro* activities of the LIC438 and LIC441 fusion proteins were determined using the same method as in Example 5. The results were illustrated in FIG. 44. Compared with the previously measured positive control LIC401, the fusion proteins LIC438 and LIC441 exhibited weakened stimulatory activities on splenocytes. The masking effect of Fc at the N-terminus on a single molecule IL12-P70 was stronger than that of ScFv with two identical targets at the N-terminus on a single molecule IL12-P70. This molecular format can attenuate the activity of IL12-P70.

### Example 35: Preparation of EGFR-double-cleavable-I tandem Fusion Protein (LIC445)

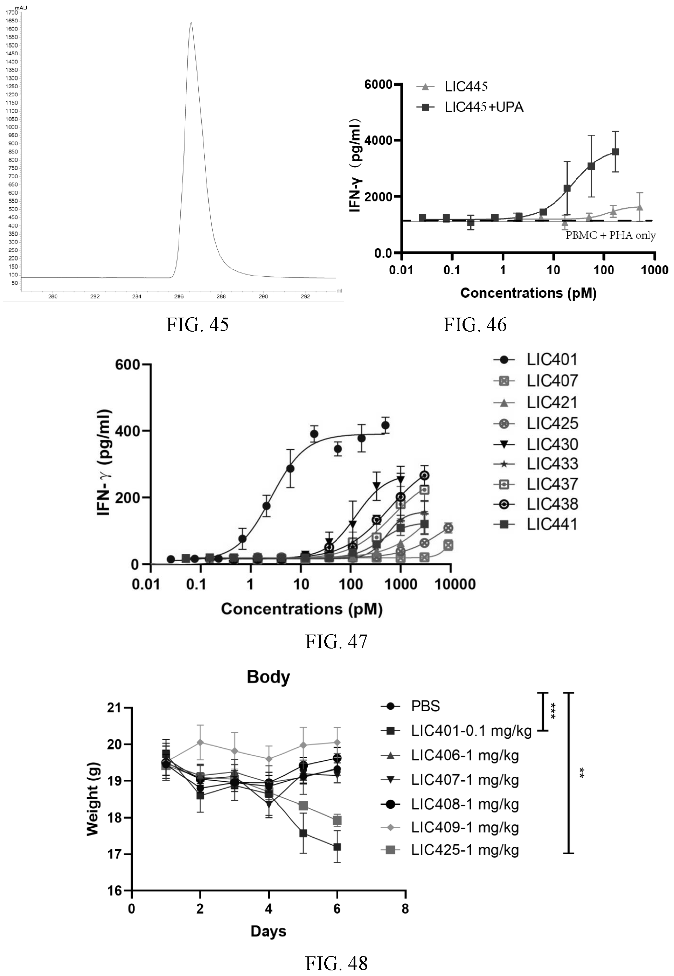

The LIC445 fusion protein was prepared using the same method as in Example 1. The DNA fragments encoding amino acid sequences as set forth in SEQ ID NO: 74 and SEQ ID NO: 73 were prepared by chemical gene synthesis and PCR technology, followed by plasmid construction. As illustrated in FIG. 1, the heavy chain of the human EGFR mAb was directly linked to human IL-12P70 via a cleavable linker. The elution peak of protein A affinity chromatography of the prepared fusion protein was illustrated in FIG. 45. An ultraviolet absorbance peak exceeding 1000 mAU can be observed, indicating that the target protein was eluted by the elution buffer.

### Example 36: Activity Assay of LIC445 Fusion Protein

The *in vitro* activity of the LIC445 fusion protein was determined using the same method as in Example 5. Human whole blood PBMCs were separated by density gradient centrifugation, and the cells were subsequently treated with ACK lysis buffer and washed repeatedly with PBS. The cells were resuspended in culture medium (RPMI 1640 + 10% FBS + 1% Pen/Strep + 4mm Glutamax + 50mm β-mercaptoethanol).

1 × 10⁵ freshly treated PBMCs were seeded into each well of a 96-well plate. PHA was added to a final concentration of 5 mg/ml for activation. Then, LIC445 and cleaved LIC445 prepared in Example 37 serially diluted at different concentrations were added and incubated in a 5% CO₂ incubator at 37°C for 72 hours. The cell supernatant was collected, and IFNγ production was measured using a human IFNγ ELISA kit. The results, as illustrated in FIG. 46, indicated that when the human IL-12 was fused to the C-terminus of the EGFR mAb, the activity differed before and after enzymatic cleavage, suggesting that fusing IL-12 to the C-terminus of the heavy chain of the EGFR antibody can effectively mask its activity, which can serve as a strategy for cytokine attenuation or for prodrug preparation.

### Example 37: Comparison of Activities of LIC407, LIC421, LIC425, LIC430, LIC433, LIC437, LIC438, and LIC441 Fusion Proteins

1. The LIC437 fusion protein was prepared using the same method as in Example 1. The corresponding amino acid sequences of the LIC437 fusion protein prepared by chemical gene synthesis and PCR technology were as set forth in SEQ ID NO: 9, SEQ ID NO: 52, and SEQ ID NO: 55. As illustrated in FIG. 1, in the LIC437 fusion protein, the Fab fragment was located at the N-terminus, and the Fc fragment was located at the C-terminus. In one chain, the Fab fragment of PDL1 was directly linked to the Fc fragment via a non-cleavable linker, in the other chain, the Fab fragment of PDL1 was linked to the Fc fragment via IL-12P70. IL-12P70 was linked to the Fab fragment and the Fc fragment of PDL1 via a non-cleavable linker, with IL-12P70 in the middle.
2. The *in vitro* activities of LIC407 prepared in Example 9, LIC421 prepared in Example 16, LIC425 prepared in Example 19, LIC430 prepared in Example 27, LIC433 prepared in Example 29, LIC437 prepared in Example 39, and LIC438 and LIC441 prepared in Example 34 were simultaneously determined using the same method as in Example 5, and with LIC401 as a positive control, the activity effects of masking structures of different molecular on the activity of IL-12 are compared. The results were illustrated in FIG. 47 and Table 1. The LIC407 structure, i.e., the structure in which the mAb was located at the N-termini of the IL12-P35 and IL12-P40 subunits, can exert a strong masking effect on the activity of IL-12, with a masking fold of greater than 4000. The LIC425 structure, i.e., the structure in which the disulfide bonds between VL and VH in a single Fab and the disulfide bond between Fc-Hinge form an upper and lower "lock" structure, exhibited a stronger masking effect than that of LIC421, which is a structure wherein two Fabs were masked by Fc. In addition, the masking effects of LIC425 and LIC421 were stronger than LIC433, indicating that the "sandwich" type steric hindrance exerted a better masking effect on a single IL-12 arranged in parallel than on two or more IL-12 arranged in series. The masking effect was LIC441 > LIC437 > LIC438, indicating that the steric hindrance of Fc > Fab > ScFv.

**Table 1**

| Molecule | EC₅₀(pM) | Masking Fold |
|---|---|---|
| **LIC401** | 2.6 | / |
| **LIC407** | 11050.0 | 4250.0 |
| **LIC421** | 4802.0 | 1846.9 |
| **LIC425** | 6830.0 | 2626.9 |
| **LIC430** | 127.1 | 48.9 |
| **LIC433** | 603.9 | 232.3 |
| **LIC437** | 525.6 | 202.2 |
| **LIC438** | 414.2 | 159.3 |
| **LIC441** | 691.4 | 265.9 |

### Example 38: In Vivo Safety Evaluation of LIC406, LIC407, LIC408, LIC409, and LIC425 Fusion Proteins

3. The safety in vivo of the LIC407 fusion protein prepared in Example 9, the LIC406, LIC408, and LIC409 fusion proteins prepared in Example 10, and the LIC425 fusion protein prepared in Example 19 were simultaneously evaluated using the same method as in Example 25, with LIC401 as a positive control. The results were illustrated in FIG. 48. The results showed that LIC401 was administered twice at a dose of 0.1 mg/kg, resulting in severe weight loss, slowed movement, and frizzy hair. In contrast, LIC425 at a dose of 1 mg/kg induced weight loss in mice, with a smaller magnitude of weight loss compared with LIC401 fusion protein at a dose of 0.1 mg/kg. In addition, LIC406 to LIC409 all exerted no effect on mouse weight at a dose of 1 mg/kg.

The amino acid sequences or nucleotide sequences involved in the present disclosure are as follows:
Amino acid sequence of mouse IL-12-P35 subunit, SEQ ID NO: 1: 
RVIPVSGPARCLSQSRNLLKTTDDMVKTAREKLKHYSCTAEDIDHEDITRD
QTSTLKTCLPLELHKNESCLATRETSSTTRGSCLPPQKTSLMMTLCLGSIYEDLKMYQ
TEFQAINAALQNHNHQQIILDKGMLVAIDELMQSLNHNGETLRQKPPVGEADPYRVK
MKLCILLHAFSTRVVTINRVMGYLSSA;
Amino acid sequence OF mouse IL-12-P40 subunit, SEQ ID No: 2: 
MWELEKDVYVVEVDWTPDAPGETVNLTCDTPEEDDITWTSDQRHGVIGS
GKTLTITVKEFLDAGQYTCHKGGETLSHSHLLLHKKENGIWSTEILKNFKNKTFLKCE
APNYSGRFTCSWLVQRNMDLKFNIKSSSSSPDSRAVTCGMASLSAEKVTLDQRDYEK
YSVSCQEDVTCPTAEETLPIELALEARQQNKYENYSTSFFIRDIIKPDPPKNLQMKPLK
NSQVEVSWEYPDSWSTPHSYFSLKFFVRIQRKKEKMKETEEGCNQKGAFLVEKTSTE
VQCKGGNVCVQAQDRYYNSSCSKWACVPCRVRS;
Amino acid sequence of human IgG1-Fc-Knob-Mut, SEQ ID NO: 3: 
EPKSCDKTHTCPPCPAPELLGGPSVFLFPPKPKDTLMISRTPEVTCVVVDVS
HEDPEVKFNWYVDGVEVHNAKTKPREEQYASTYRVVSVLTVLHQDWLNGKEYKCK
VSNKALPAPIEKTISKAKGQPREPQVYTLPPCRDELTKNQVSLWCLVKGFYPSDIAVE
WESNGQPENNYKTTPPVLDSDGSFFLYSKLTVDKSRWQQGNVFSCSVMHEALHNHY
TQKSLSLS;
Amino acid sequence of human IgG1-Fc-Hole-Mut, SEQ ID NO: 4: 
EPKSCDKTHTCPPCPAPELLGGPSVFLFPPKPKDTLMISRTPEVTCVVVDVS
HEDPEVKFNWYVDGVEVHNAKTKPREEQYASTYRVVSVLTVLHQDWLNGKEYKCK
VSNKALPAPIEKTISKAKGQPREPQVCTLPPSRDELTKNQVSLSCAVKGFYPSDIAVEW
 
ESNGQPENNYKTTPPVLDSDGSFFLVSKLTVDKSRWQQGNVFSCSVMHEALHNHYT
QKSLSLS;
Amino acid sequence of non-cleavable linker 1 at the N-terminus of IL12 subunit, SEQ ID NO: 5:
   GSSGGSGGSGGSG;
Amino acid sequence of non-cleavable linker 2 at the C-terminus of IL12 subunit, SEQ ID NO: 6:
   GGGGSGGGGS;
Amino acid sequence of cleavable UPA substrate linker 3, SEQ ID NO: 7:
   GSSGGSGGSGGSGLSGRSDNHGSSGGSGGSGGSG;
Amino acid sequence of cleavable MMP substrate linker 4, SEQ ID NO: 8:
   GSSGGSGGSGGSGQLLGFLTAGSSGGSGGSGGSG;
Amino acid sequence of light chain of human PDL1 monoclonal antibody, SEQ ID NO: 9: 
DIQMTQSPSSLSASVGDRVTITCRASQDVSTAVAWYQQKPGKAPKLLIYSA
SFLYSGVPSRFSGSGSGTDFTLTISSLQPEDFATYYCQQYLYHPATFGQGTKVEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC;
Amino acid sequence of heavy chain (Mut) of human PDL1-IgG1 monoclonal antibody, SEQ ID NO: 10: 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYV
DGVEVHNAKTKPREEQYASTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLS;
Amino acid sequence of heavy chain (Knob-Mut) of human PDL1-IgG1 monoclonal antibody, SEQ ID No: 11: 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYV
DGVEVHNAKTKPREEQYASTYRVVSVLTVLHQDWLNGKEYKCKVSNKALPAPIEKTI
SKAKGQPREPQVYTLPPCRDELTKNQVSLWCLVKGFYPSDIAVEWESNGQPENNYKT
TPPVLDSDGSFFLYSKLTVDKSRWQQGNVFSCSVMHEALHNHYTQKSLSLS;
Amino acid sequence of heavy chain (Hole-Mut) of human PDL1-IgG1 monoclonal antibody, SEQ ID No: 12: 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYV
DGVEVHNAKTKPREEQYASTYRVVSVLTVLHQDWLNGKEYKCKVSNKALPAPIEKTI
SKAKGQPREPQVCTLPPSRDELTKNQVSLSCAVKGFYPSDIAVEWESNGQPENNYKTT
PPVLDSDGSFFLVSKLTVDKSRWQQGNVFSCSVMHEALHNHYTQKSLSLS;
Amino acid sequence of light chain of human PD1 monoclonal antibody, SEQ ID No: 13: 
DVVMTQSPLSLPVTLGQPASISCRSSQSLLDSDGGTYLYWFQQRPGQSPRR
LIYLVSTLGSGVPDRFSGSGSGTDFTLKISRVEAEDVGVYYCMQLTHWPYTFGQGTKL
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC;
Amino acid sequence of heavy chain of human PD1-IgG4 monoclonal antibody, SEQ ID No: 14: 
QVQLVQSGAEVKKPGSSVKVSCKASGFTFTTYYISWVRQAPGQGLEYLGY
INMGSGGTNYNEKFKGRVTITADKSTSTAYMELSSLRSEDTAVYYCAIIGYFDYWGQG
TMVTVSSASTKGPSVFPLAPCSRSTSESTAALGCLVKDYFPEPVTVSWNSGALTSGVH
TFPAVLQSSGLYSLSSVVTVPSSSLGTKTYTCNVDHKPSNTKVDKRVESKYGPPCPPCP
 
APEFLGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPR
EPQVYTLPPSQEEMTKNQVSLTCLVKGFYPSDIAVEWESNGQPENNYKTTPPVLDSDG
SFFLYSRLTVDKSRWQEGNVFSCSVMHEALHNHYTQKSLSLSLGK;
Amino acid sequence of heavy chain (Knob) of human PD1-IgG4 monoclonal antibody, SEQ ID No: 15: 
QVQLVQSGAEVKKPGSSVKVSCKASGFTFTTYYISWVRQAPGQGLEYLGY
INMGSGGTNYNEKFKGRVTITADKSTSTAYMELSSLRSEDTAVYYCAIIGYFDYWGQG
TMVTVSSASTKGPSVFPLAPCSRSTSESTAALGCLVKDYFPEPVTVSWNSGALTSGVH
TFPAVLQSSGLYSLSSVVTVPSSSLGTKTYTCNVDHKPSNTKVDKRVESKYGPPCPPCP
APEFLGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPR
EPQVYTLPPCQEEMTKNQVSLWCLVKGFYPSDIAVEWESNGQPENNYKTTPPVLDSD
GSFFLYSRLTVDKSRWQEGNVFSCSVMHEALHNHYTQKSLSLSLGK;
Amino acid sequence of heavy chain (Hole) of human PD1-IgG4 monoclonal antibody, SEQ ID No: 16: 
QVQLVQSGAEVKKPGSSVKVSCKASGFTFTTYYISWVRQAPGQGLEYLGY
INMGSGGTNYNEKFKGRVTITADKSTSTAYMELSSLRSEDTAVYYCAIIGYFDYWGQG
TMVTVSSASTKGPSVFPLAPCSRSTSESTAALGCLVKDYFPEPVTVSWNSGALTSGVH
TFPAVLQSSGLYSLSSVVTVPSSSLGTKTYTCNVDHKPSNTKVDKRVESKYGPPCPPCP
APEFLGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPR
EPQVCTLPPSQEEMTKNQVSLSCAVKGFYPSDIAVEWESNGQPENNYKTTPPVLDSDG
SFFLVSRLTVDKSRWQEGNVFSCSVMHEALHNHYTQKSLSLSLGK;
Amino acid sequence of human PDL1-ScFv, SEQ ID No: 17: 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSGSSGGSGGSGGSGDIQMTQSPSSLSASVGDRVTITCRASQDVSTAV
AWYQQKPGKAPKLLIYSASFLYSGVPSRFSGSGSGTDFTLTISSLQPEDFATYYCQQYL
YHPATFGQGTKVEIK;
Amino acid sequence of human PD1-ScFv, SEQ ID No: 18: 
QVQLVQSGAEVKKPGSSVKVSCKASGFTFTTYYISWVRQAPGQGLEYLGY
INMGSGGTNYNEKFKGRVTITADKSTSTAYMELSSLRSEDTAVYYCAIIGYFDYWGQG
TMVTVSSGSSGGSGGSGGSGDVVMTQSPLSLPVTLGQPASISCRSSQSLLDSDGGTYL
YWFQQRPGQSPRRLIYLVSTLGSGVPDRFSGSGSGTDFTLKISRVEAEDVGVYYCMQL
THWPYTFGQGTKLEIK;
Amino acid sequence of human PDL1-VH-CH1, SEQ ID No: 19: 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKV;
Amino acid sequence of human PD1-VH-CH1, SEQ ID No: 20: 
QVQLVQSGAEVKKPGSSVKVSCKASGFTFTTYYISWVRQAPGQGLEYLGY
INMGSGGTNYNEKFKGRVTITADKSTSTAYMELSSLRSEDTAVYYCAIIGYFDYWGQG
TMVTVSSASTKGPSVFPLAPCSRSTSESTAALGCLVKDYFPEPVTVSWNSGALTSGVH
TFPAVLQSSGLYSLSSVVTVPSSSLGTKTYTCNVDHKPSNTKVDKRV;
IF fusion protein LIC401: the amino acid sequence of chain 1 is SEQ ID No: 21 (SEQ ID No: 1 + SEQ ID No: 6 + SEQ ID No: 3), and the amino acid sequence of chain 2 is SEQ ID No: 22 (SEQ ID No: 2 + SEQ ID No: 6 + SEQ ID No: 4): 
RVIPVSGPARCLSQSRNLLKTTDDMVKTAREKLKHYSCTAEDIDHEDITRD
QTSTLKTCLPLELHKNESCLATRETSSTTRGSCLPPQKTSLMMTLCLGSIYEDLKMYQ
TEFQAINAALQNHNHQQIILDKGMLVAIDELMQSLNHNGETLRQKPPVGEADPYRVK
MKLCILLHAFSTRVVTINRVMGYLSSAGGGGSGGGGSEPKSCDKTHTCPPCPAPELLG
GPSVFLFPPKPKDTLMISRTPEVTCVVVDVSHEDPEVKFNWYVDGVEVHNAKTKPRE
EQYASTYRVVSVLTVLHQDWLNGKEYKCKVSNKALPAPIEKTISKAKGQPREPQVYT
LPPCRDELTKNQVSLWCLVKGFYPSDIAVEWESNGQPENNYKTTPPVLDSDGSFFLYS
KLTVDKSRWQQGNVFSCSVMHEALHNHYTQKSLSLSPGK (SEQ ID No: 21);
 
MWELEKDVYVVEVDWTPDAPGETVNLTCDTPEEDDITWTSDQRHGVIGS
GKTLTITVKEFLDAGQYTCHKGGETLSHSHLLLHKKENGIWSTEILKNFKNKTFLKCE
APNYSGRFTCSWLVQRNMDLKFNIKSSSSSPDSRAVTCGMASLSAEKVTLDQRDYEK
 
YSVSCQEDVTCPTAEETLPIELALEARQQNKYENYSTSFFIRDIIKPDPPKNLQMKPLK
NSQVEVSWEYPDSWSTPHSYFSLKFFVRIQRKKEKMKETEEGCNQKGAFLVEKTSTE
VQCKGGNVCVQAQDRYYNSSCSKWACVPCRVRSGGGGSGGGGSEPKSCDKTHTCPP
CPAPELLGGPSVFLFPPKPKDTLMISRTPEVTCVVVDVSHEDPEVKFNWYVDGVEVH
NAKTKPREEQYASTYRVVSVLTVLHQDWLNGKEYKCKVSNKALPAPIEKTISKAKGQ
PREPQVCTLPPSRDELTKNQVSLSCAVKGFYPSDIAVEWESNGQPENNYKTTPPVLDS
DGSFFLVSKLTVDKSRWQQGNVFSCSVMHEALHNHYTQKSLSLSPGK (SEQ ID No:
22);
FI Fusion Protein LIC402: the amino acid sequence of chain 1 is SEQ ID No: 23 (SEQ ID No: 3 + SEQ ID No: 5 + SEQ ID No: 1), and the amino acid sequence of chain 2 is SEQ ID No: 24 (SEQ ID No: 4 + SEQ ID No: 5 + SEQ ID No: 2): 
EPKSCDKTHTCPPCPAPELLGGPSVFLFPPKPKDTLMISRTPEVTCVVVDVS
HEDPEVKFNWYVDGVEVHNAKTKPREEQYASTYRVVSVLTVLHQDWLNGKEYKCK
VSNKALPAPIEKTISKAKGQPREPQVYTLPPCRDELTKNQVSLWCLVKGFYPSDIAVE
WESNGQPENNYKTTPPVLDSDGSFFLYSKLTVDKSRWQQGNVFSCSVMHEALHNHY
TQKSLSLSPGKGSSGGSGGSGGSGRVIPVSGPARCLSQSRNLLKTTDDMVKTAREKLK
HYSCTAEDIDHEDITRDQTSTLKTCLPLELHKNESCLATRETSSTTRGSCLPPQKTSLM
MTLCLGSIYEDLKMYQTEFQAINAALQNHNHQQIILDKGMLVAIDELMQSLNHNGET
LRQKPPVGEADPYRVKMKLCILLHAFSTRVVTINRVMGYLSSA (SEQ ID No: 23);
 
EPKSCDKTHTCPPCPAPELLGGPSVFLFPPKPKDTLMISRTPEVTCVVVDVS
HEDPEVKFNWYVDGVEVHNAKTKPREEQYASTYRVVSVLTVLHQDWLNGKEYKCK
VSNKALPAPIEKTISKAKGQPREPQVCTLPPSRDELTKNQVSLSCAVKGFYPSDIAVEW
ESNGQPENNYKTTPPVLDSDGSFFLVSKLTVDKSRWQQGNVFSCSVMHEALHNHYT
QKSLSLSPGKGSSGGSGGSGGSGMWELEKDVYVVEVDWTPDAPGETVNLTCDTPEE
DDITWTSDQRHGVIGSGKTLTITVKEFLDAGQYTCHKGGETLSHSHLLLHKKENGIW
STEILKNFKNKTFLKCEAPNYSGRFTCSWLVQRNMDLKFNIKSSSSSPDSRAVTCGMA
SLSAEKVTLDQRDYEKYSVSCQEDVTCPTAEETLPIELALEARQQNKYENYSTSFFIR
DIIKPDPPKNLQMKPLKNSQVEVSWEYPDSWSTPHSYFSLKFFVRIQRKKEKMKETEE
GCNQKGAFLVEKTSTEVQCKGGNVCVQAQDRYYNSSCSKWACVPCRVRS (SEQ ID
No: 24);
F-double-cleavable-I fusion protein LIC403: the amino acid sequence of chain 1 is SEQ ID No: 25 (SEQ ID No: 3 + SEQ ID No: 7 + SEQ ID No: 1), and the amino acid sequence of chain 2 is SEQ ID No: 26 (SEQ ID No: 4 + SEQ ID No: 7 + SEQ ID No: 2): 
EPKSCDKTHTCPPCPAPELLGGPSVFLFPPKPKDTLMISRTPEVTCVVVDVS
HEDPEVKFNWYVDGVEVHNAKTKPREEQYASTYRVVSVLTVLHQDWLNGKEYKCK
VSNKALPAPIEKTISKAKGQPREPQVYTLPPCRDELTKNQVSLWCLVKGFYPSDIAVE
WESNGQPENNYKTTPPVLDSDGSFFLYSKLTVDKSRWQQGNVFSCSVMHEALHNHY
TQKSLSLSPGKGSSGGSGGSGGSGLSGRSDNHGSSGGSGGSGGSGRVIPVSGPARCLS
QSRNLLKTTDDMVKTAREKLKHYSCTAEDIDHEDITRDQTSTLKTCLPLELHKNESCL
ATRETSSTTRGSCLPPQKTSLMMTLCLGSIYEDLKMYQTEFQAINAALQNHNHQQIIL
DKGMLVAIDELMQSLNHNGETLRQKPPVGEADPYRVKMKLCILLHAFSTRVVTINRV
MGYLSSA (SEQ ID No: 25);
 
EPKSCDKTHTCPPCPAPELLGGPSVFLFPPKPKDTLMISRTPEVTCVVVDVS
HEDPEVKFNWYVDGVEVHNAKTKPREEQYASTYRVVSVLTVLHQDWLNGKEYKCK
VSNKALPAPIEKTISKAKGQPREPQVCTLPPSRDELTKNQVSLSCAVKGFYPSDIAVEW
ESNGQPENNYKTTPPVLDSDGSFFLVSKLTVDKSRWQQGNVFSCSVMHEALHNHYT
QKSLSLSPGKGSSGGSGGSGGSGLSGRSDNHGSSGGSGGSGGSGMWELEKDVYVVE
VDWTPDAPGETVNLTCDTPEEDDITWTSDQRHGVIGSGKTLTITVKEFLDAGQYTCH
KGGETLSHSHLLLHKKENGIWSTEILKNFKNKTFLKCEAPNYSGRFTCSWLVQRNMD
LKFNIKSSSSSPDSRAVTCGMASLSAEKVTLDQRDYEKYSVSCQEDVTCPTAEETLPIE
LALEARQQNKYENYSTSFFIRDIIKPDPPKNLQMKPLKNSQVEVSWEYPDSWSTPHSY
FSLKFFVRIQRKKEKMKETEEGCNQKGAFLVEKTSTEVQCKGGNVCVQAQDRYYNS
SCSKWACVPCRVRS (SEQ ID No: 26);
F-right-cleavable-I fusion protein LIC404: the amino acid sequence of chain 1 is SEQ ID No: 23, and the amino acid sequence of chain 2 is SEQ ID No: 26;
F-left-cleavable-I fusion protein LIC405: the amino acid sequence of chain 1 is SEQ ID No: 25, and the amino acid sequence of chain 2 is SEQ ID No: 24;
PI fusion protein LIC406: the amino acid sequence of chain 1 is SEQ ID No: 27 (SEQ ID No: 11 + SEQ ID No: 5 + SEQ ID No: 1), the amino acid sequence of chain 2 is SEQ ID No: 28 (SEQ ID No: 12 + SEQ ID No: 5 + SEQ ID No: 2), and the amino acid sequence of chain 3 is SEQ ID No: 9: 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYV
DGVEVHNAKTKPREEQYASTYRVVSVLTVLHQDWLNGKEYKCKVSNKALPAPIEKTI
SKAKGQPREPQVYTLPPCRDELTKNQVSLWCLVKGFYPSDIAVEWESNGQPENNYKT
TPPVLDSDGSFFLYSKLTVDKSRWQQGNVFSCSVMHEALHNHYTQKSLSLSPGKGSS
GGSGGSGGSGRVIPVSGPARCLSQSRNLLKTTDDMVKTAREKLKHYSCTAEDIDHEDI
TRDQTSTLKTCLPLELHKNESCLATRETSSTTRGSCLPPQKTSLMMTLCLGSIYEDLK
MYQTEFQAINAALQNHNHQQIILDKGMLVAIDELMQSLNHNGETLRQKPPVGEADPY
RVKMKLCILLHAFSTRVVTINRVMGYLSSA (SEQ ID No: 27);
 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYV
DGVEVHNAKTKPREEQYASTYRVVSVLTVLHQDWLNGKEYKCKVSNKALPAPIEKTI
SKAKGQPREPQVCTLPPSRDELTKNQVSLSCAVKGFYPSDIAVEWESNGQPENNYKTT
PPVLDSDGSFFLVSKLTVDKSRWQQGNVFSCSVMHEALHNHYTQKSLSLSPGKGSSG
GSGGSGGSGMWELEKDVYVVEVDWTPDAPGETVNLTCDTPEEDDITWTSDQRHGVI
GSGKTLTITVKEFLDAGQYTCHKGGETLSHSHLLLHKKENGIWSTEILKNFKNKTFLK
CEAPNYSGRFTCSWLVQRNMDLKFNIKSSSSSPDSRAVTCGMASLSAEKVTLDQRDY
EKYSVSCQEDVTCPTAEETLPIELALEARQQNKYENYSTSFFIRDIIKPDPPKNLQMKP
LKNSQVEVSWEYPDSWSTPHSYFSLKFFVRIQRKKEKMKETEEGCNQKGAFLVEKTS
TEVQCKGGNVCVQAQDRYYNSSCSKWACVPCRVRS (SEQ ID No: 28);
P-double-cleavable-I fusion protein LIC407: the amino acid sequence of chain 1 is SEQ ID No: 29 (SEQ ID No: 11 + SEQ ID No: 7 + SEQ ID No: 1), the amino acid sequence of chain 2 is SEQ ID No: 30 (SEQ ID No: 12 + SEQ ID No: 7 + SEQ ID No: 2), and the amino acid sequence of chain 3 is SEQ ID No: 9: 
VQLVESGGGLVQPGGSLRLSCAASGFTFSDSWIHWVRQAPGKGLEWVAWI
SPYGGSTYYADSVKGRFTISADTSKNTAYLQMNSLRAEDTAVYYCARRHWPGGFDY
WGQGTLVTVSSASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKKVEPKSCDK
THTCPPCPAPELLGGPSVFLFPPKPKDTLMISRTPEVTCVVVDVSHEDPEVKFNWYVD
GVEVHNAKTKPREEQYASTYRVVSVLTVLHQDWLNGKEYKCKVSNKALPAPIEKTIS
KAKGQPREPQVYTLPPCRDELTKNQVSLWCLVKGFYPSDIAVEWESNGQPENNYKTT
PPVLDSDGSFFLYSKLTVDKSRWQQGNVFSCSVMHEALHNHYTQKSLSLSPGKGSSG
GSGGSGGSGLSGRSDNHGSSGGSGGSGGSGRVIPVSGPARCLSQSRNLLKTTDDMVK
TAREKLKHYSCTAEDIDHEDITRDQTSTLKTCLPLELHKNESCLATRETSSTTRGSCLPP
QKTSLMMTLCLGSIYEDLKMYQTEFQAINAALQNHNHQQIILDKGMLVAIDELMQSL
NHNGETLRQKPPVGEADPYRVKMKLCILLHAFSTRVVTINRVMGYLSSA (SEQ ID No:
29);
 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYV
DGVEVHNAKTKPREEQYASTYRVVSVLTVLHQDWLNGKEYKCKVSNKALPAPIEKTI
SKAKGQPREPQVCTLPPSRDELTKNQVSLSCAVKGFYPSDIAVEWESNGQPENNYKTT
PPVLDSDGSFFLVSKLTVDKSRWQQGNVFSCSVMHEALHNHYTQKSLSLSPGKGSSG
GSGGSGGSGLSGRSDNHGSSGGSGGSGGSGMWELEKDVYVVEVDWTPDAPGETVN
LTCDTPEEDDITWTSDQRHGVIGSGKTLTITVKEFLDAGQYTCHKGGETLSHSHLLLH
KKENGIWSTEILKNFKNKTFLKCEAPNYSGRFTCSWLVQRNMDLKFNIKSSSSSPDSR
AVTCGMASLSAEKVTLDQRDYEKYSVSCQEDVTCPTAEETLPIELALEARQQNKYEN
YSTSFFIRDIIKPDPPKNLQMKPLKNSQVEVSWEYPDSWSTPHSYFSLKFFVRIQRKKE
KMKETEEGCNQKGAFLVEKTSTEVQCKGGNVCVQAQDRYYNSSCSKWACVPCRVR
S (SEQ ID No: 30);
P-right-cleavable-I fusion protein LIC408: the amino acid sequence of chain 1 is SEQ ID No: 27, the amino acid sequence of chain 2 is SEQ ID No: 30, and the amino acid sequence of chain 3 is SEQ ID No: 9;
P-left-cleavable-I fusion protein LIC409: the amino acid sequence of chain 1 is SEQ ID No: 29, the amino acid sequence of chain 2 is SEQ ID No: 28, and the amino acid sequence of chain 3 is SEQ ID No: 9;
IF-PDL1-ScFv fusion protein LIC410: the amino acid sequence of chain 1 is SEQ ID No: 31 (SEQ ID No: 21 + SEQ ID No: 17), and the amino acid sequence of chain 2 is SEQ ID No: 32 (SEQ ID No: 22 + SEQ ID No: 17): 
RVIPVSGPARCLSQSRNLLKTTDDMVKTAREKLKHYSCTAEDIDHEDITRD
QTSTLKTCLPLELHKNESCLATRETSSTTRGSCLPPQKTSLMMTLCLGSIYEDLKMYQ
TEFQAINAALQNHNHQQIILDKGMLVAIDELMQSLNHNGETLRQKPPVGEADPYRVK
MKLCILLHAFSTRVVTINRVMGYLSSAGGGGSGGGGSEPKSCDKTHTCPPCPAPELLG
GPSVFLFPPKPKDTLMISRTPEVTCVVVDVSHEDPEVKFNWYVDGVEVHNAKTKPRE
EQYASTYRVVSVLTVLHQDWLNGKEYKCKVSNKALPAPIEKTISKAKGQPREPQVYT
LPPCRDELTKNQVSLWCLVKGFYPSDIAVEWESNGQPENNYKTTPPVLDSDGSFFLYS
KLTVDKSRWQQGNVFSCSVMHEALHNHYTQKSLSLSPGKEVQLVESGGGLVQPGGS
LRLSCAASGFTFSDSWIHWVRQAPGKGLEWVAWISPYGGSTYYADSVKGRFTISADT
SKNTAYLQMNSLRAEDTAVYYCARRHWPGGFDYWGQGTLVTVSSGSSGGSGGSGGS
GDIQMTQSPSSLSASVGDRVTITCRASQDVSTAVAWYQQKPGKAPKLLIYSASFLYSG
VPSRFSGSGSGTDFTLTISSLQPEDFATYYCQQYLYHPATFGQGTKVEIK (SEQ ID No:
31);
 
MWELEKDVYVVEVDWTPDAPGETVNLTCDTPEEDDITWTSDQRHGVIGS
GKTLTITVKEFLDAGQYTCHKGGETLSHSHLLLHKKENGIWSTEILKNFKNKTFLKCE
APNYSGRFTCSWLVQRNMDLKFNIKSSSSSPDSRAVTCGMASLSAEKVTLDQRDYEK
YSVSCQEDVTCPTAEETLPIELALEARQQNKYENYSTSFFIRDIIKPDPPKNLQMKPLK
NSQVEVSWEYPDSWSTPHSYFSLKFFVRIQRKKEKMKETEEGCNQKGAFLVEKTSTE
VQCKGGNVCVQAQDRYYNSSCSKWACVPCRVRSGGGGSGGGGSEPKSCDKTHTCPP
CPAPELLGGPSVFLFPPKPKDTLMISRTPEVTCVVVDVSHEDPEVKFNWYVDGVEVH
NAKTKPREEQYASTYRVVSVLTVLHQDWLNGKEYKCKVSNKALPAPIEKTISKAKGQ
PREPQVCTLPPSRDELTKNQVSLSCAVKGFYPSDIAVEWESNGQPENNYKTTPPVLDS
 
DGSFFLVSKLTVDKSRWQQGNVFSCSVMHEALHNHYTQKSLSLSPGKEVQLVESGGG
LVQPGGSLRLSCAASGFTFSDSWIHWVRQAPGKGLEWVAWISPYGGSTYYADSVKGR
FTISADTSKNTAYLQMNSLRAEDTAVYYCARRHWPGGFDYWGQGTLVTVSSGSSGGS
GGSGGSGDIQMTQSPSSLSASVGDRVTITCRASQDVSTAVAWYQQKPGKAPKLLIYSA
SFLYSGVPSRFSGSGSGTDFTLTISSLQPEDFATYYCQQYLYHPATFGQGTKVEIK (SEQ
ID No: 32);
PDL1-ScFv-FI fusion protein LIC411: the amino acid sequence of chain 1 is SEQ ID No: 33 (SEQ ID No: 17 + SEQ ID No: 23), and the amino acid sequence of chain 2 is SEQ ID No: 34 (SEQ ID No: 17 + SEQ ID No: 24); 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSGSSGGSGGSGGSGDIQMTQSPSSLSASVGDRVTITCRASQDVSTAV
AWYQQKPGKAPKLLIYSASFLYSGVPSRFSGSGSGTDFTLTISSLQPEDFATYYCQQYL
YHPATFGQGTKVEIKEPKSCDKTHTCPPCPAPELLGGPSVFLFPPKPKDTLMISRTPEVT
CVVVDVSHEDPEVKFNWYVDGVEVHNAKTKPREEQYASTYRVVSVLTVLHQDWLN
GKEYKCKVSNKALPAPIEKTISKAKGQPREPQVYTLPPCRDELTKNQVSLWCLVKGFY
PSDIAVEWESNGQPENNYKTTPPVLDSDGSFFLYSKLTVDKSRWQQGNVFSCSVMHE
ALHNHYTQKSLSLSPGKGSSGGSGGSGGSGRVIPVSGPARCLSQSRNLLKTTDDMVK
TAREKLKHYSCTAEDIDHEDITRDQTSTLKTCLPLELHKNESCLATRETSSTTRGSCLPP
QKTSLMMTLCLGSIYEDLKMYQTEFQAINAALQNHNHQQIILDKGMLVAIDELMQSL
NHNGETLRQKPPVGEADPYRVKMKLCILLHAFSTRVVTINRVMGYLSSA (SEQ ID No:
33);
 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSGSSGGSGGSGGSGDIQMTQSPSSLSASVGDRVTITCRASQDVSTAV
AWYQQKPGKAPKLLIYSASFLYSGVPSRFSGSGSGTDFTLTISSLQPEDFATYYCQQYL
YHPATFGQGTKVEIKEPKSCDKTHTCPPCPAPELLGGPSVFLFPPKPKDTLMISRTPEVT
CVVVDVSHEDPEVKFNWYVDGVEVHNAKTKPREEQYASTYRVVSVLTVLHQDWLN
GKEYKCKVSNKALPAPIEKTISKAKGQPREPQVCTLPPSRDELTKNQVSLSCAVKGFY
PSDIAVEWESNGQPENNYKTTPPVLDSDGSFFLVSKLTVDKSRWQQGNVFSCSVMHE
 
ALHNHYTQKSLSLSPGKGSSGGSGGSGGSGMWELEKDVYVVEVDWTPDAPGETVN
LTCDTPEEDDITWTSDQRHGVIGSGKTLTITVKEFLDAGQYTCHKGGETLSHSHLLLH
KKENGIWSTEILKNFKNKTFLKCEAPNYSGRFTCSWLVQRNMDLKFNIKSSSSSPDSR
AVTCGMASLSAEKVTLDQRDYEKYSVSCQEDVTCPTAEETLPIELALEARQQNKYEN
YSTSFFIRDIIKPDPPKNLQMKPLKNSQVEVSWEYPDSWSTPHSYFSLKFFVRIQRKKE
KMKETEEGCNQKGAFLVEKTSTEVQCKGGNVCVQAQDRYYNSSCSKWACVPCRVR
S (SEQ ID No: 34);
PDL1-ScFv-F-double-cleavable-I fusion protein LIC412: the amino acid sequence of chain 1 is SEQ ID No: 35 (SEQ ID No: 17 + SEQ ID No: 25), and the amino acid sequence of chain 2 is SEQ ID No: 36 (SEQ ID No: 17 + SEQ ID No: 26); 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSGSSGGSGGSGGSGDIQMTQSPSSLSASVGDRVTITCRASQDVSTAV
AWYQQKPGKAPKLLIYSASFLYSGVPSRFSGSGSGTDFTLTISSLQPEDFATYYCQQYL
YHPATFGQGTKVEIKEPKSCDKTHTCPPCPAPELLGGPSVFLFPPKPKDTLMISRTPEVT
CVVVDVSHEDPEVKFNWYVDGVEVHNAKTKPREEQYASTYRVVSVLTVLHQDWLN
GKEYKCKVSNKALPAPIEKTISKAKGQPREPQVYTLPPCRDELTKNQVSLWCLVKGFY
PSDIAVEWESNGQPENNYKTTPPVLDSDGSFFLYSKLTVDKSRWQQGNVFSCSVMHE
ALHNHYTQKSLSLSPGKGSSGGSGGSGGSGLSGRSDNHGSSGGSGGSGGSGRVIPVSG
PARCLSQSRNLLKTTDDMVKTAREKLKHYSCTAEDIDHEDITRDQTSTLKTCLPLELH
KNESCLATRETSSTTRGSCLPPQKTSLMMTLCLGSIYEDLKMYQTEFQAINAALQNHN
HQQIILDKGMLVAIDELMQSLNHNGETLRQKPPVGEADPYRVKMKLCILLHAFSTRVV
TINRVMGYLSSA (SEQ ID No: 35);
 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSGSSGGSGGSGGSGDIQMTQSPSSLSASVGDRVTITCRASQDVSTAV
AWYQQKPGKAPKLLIYSASFLYSGVPSRFSGSGSGTDFTLTISSLQPEDFATYYCQQYL
YHPATFGQGTKVEIKEPKSCDKTHTCPPCPAPELLGGPSVFLFPPKPKDTLMISRTPEVT
CVVVDVSHEDPEVKFNWYVDGVEVHNAKTKPREEQYASTYRVVSVLTVLHQDWLN
GKEYKCKVSNKALPAPIEKTISKAKGQPREPQVCTLPPSRDELTKNQVSLSCAVKGFY
 
PSDIAVEWESNGQPENNYKTTPPVLDSDGSFFLVSKLTVDKSRWQQGNVFSCSVMHE
ALHNHYTQKSLSLSPGKGSSGGSGGSGGSGLSGRSDNHGSSGGSGGSGGSGMWELE
KDVYVVEVDWTPDAPGETVNLTCDTPEEDDITWTSDQRHGVIGSGKTLTITVKEFLD
AGQYTCHKGGETLSHSHLLLHKKENGIWSTEILKNFKNKTFLKCEAPNYSGRFTCSW
LVQRNMDLKFNIKSSSSSPDSRAVTCGMASLSAEKVTLDQRDYEKYSVSCQEDVTCP
TAEETLPIELALEARQQNKYENYSTSFFIRDIIKPDPPKNLQMKPLKNSQVEVSWEYPD
SWSTPHSYFSLKFFVRIQRKKEKMKETEEGCNQKGAFLVEKTSTEVQCKGGNVCVQ
AQDRYYNSSCSKWACVPCRVRS (SEQ ID No: 36);
PDL1-ScFv-F-right-cleavable-I fusion protein LIC413: the amino acid sequence of chain 1 is SEQ ID No: 33, and the amino acid sequence of chain 2 is SEQ ID No: 36;
PDL1-ScFv-F-left-cleavable-I fusion protein LIC414: the amino acid sequence of chain 1 is SEQ ID No: 35, and the amino acid sequence of chain 2 is SEQ ID No: 34;
IF-PDL1-ScFv-PD1-ScFv fusion protein LIC415: the amino acid sequence of chain 1 is SEQ ID No: 31, and the amino acid sequence of chain 2 is SEQ ID No: 37 (SEQ ID No: 22 + SEQ ID No: 18): 
MWELEKDVYVVEVDWTPDAPGETVNLTCDTPEEDDITWTSDQRHGVIGS
GKTLTITVKEFLDAGQYTCHKGGETLSHSHLLLHKKENGIWSTEILKNFKNKTFLKCE
APNYSGRFTCSWLVQRNMDLKFNIKSSSSSPDSRAVTCGMASLSAEKVTLDQRDYEK
YSVSCQEDVTCPTAEETLPIELALEARQQNKYENYSTSFFIRDIIKPDPPKNLQMKPLK
NSQVEVSWEYPDSWSTPHSYFSLKFFVRIQRKKEKMKETEEGCNQKGAFLVEKTSTE
VQCKGGNVCVQAQDRYYNSSCSKWACVPCRVRSGGGGSGGGGSEPKSCDKTHTCPP
CPAPELLGGPSVFLFPPKPKDTLMISRTPEVTCVVVDVSHEDPEVKFNWYVDGVEVH
NAKTKPREEQYASTYRVVSVLTVLHQDWLNGKEYKCKVSNKALPAPIEKTISKAKGQ
PREPQVCTLPPSRDELTKNQVSLSCAVKGFYPSDIAVEWESNGQPENNYKTTPPVLDS
DGSFFLVSKLTVDKSRWQQGNVFSCSVMHEALHNHYTQKSLSLSPGKQVQLVQSGA
EVKKPGSSVKVSCKASGFTFTTYYISWVRQAPGQGLEYLGYINMGSGGTNYNEKFK
GRVTITADKSTSTAYMELSSLRSEDTAVYYCAIIGYFDYWGQGTMVTVSSGSSGGSGG
SGGSGDVVMTQSPLSLPVTLGQPASISCRSSQSLLDSDGGTYLYWFQQRPGQSPRRLI
YLVSTLGSGVPDRFSGSGSGTDFTLKISRVEAEDVGVYYCMQLTHWPYTFGQGTKLEI
K (SEQ ID No: 37);
PDL1-ScFv-PD1-ScFv-FI fusion protein LIC416: the amino acid sequence of chain 1 is SEQ ID No: 33, and the amino acid sequence of chain 2 is SEQ ID No: 38 (SEQ ID No: 18 + SEQ ID No: 24): 
QVQLVQSGAEVKKPGSSVKVSCKASGFTFTTYYISWVRQAPGQGLEYLGY
INMGSGGTNYNEKFKGRVTITADKSTSTAYMELSSLRSEDTAVYYCAIIGYFDYWGQG
TMVTVSSGSSGGSGGSGGSGDVVMTQSPLSLPVTLGQPASISCRSSQSLLDSDGGTYL
YWFQQRPGQSPRRLIYLVSTLGSGVPDRFSGSGSGTDFTLKISRVEAEDVGVYYCMQL
THWPYTFGQGTKLEIKEPKSCDKTHTCPPCPAPELLGGPSVFLFPPKPKDTLMISRTPE
VTCVVVDVSHEDPEVKFNWYVDGVEVHNAKTKPREEQYASTYRVVSVLTVLHQDW
LNGKEYKCKVSNKALPAPIEKTISKAKGQPREPQVCTLPPSRDELTKNQVSLSCAVKG
FYPSDIAVEWESNGQPENNYKTTPPVLDSDGSFFLVSKLTVDKSRWQQGNVFSCSVM
HEALHNHYTQKSLSLSPGKGSSGGSGGSGGSGMWELEKDVYVVEVDWTPDAPGET
VNLTCDTPEEDDITWTSDQRHGVIGSGKTLTITVKEFLDAGQYTCHKGGETLSHSHLL
LHKKENGIWSTEILKNFKNKTFLKCEAPNYSGRFTCSWLVQRNMDLKFNIKSSSSSPD
SRAVTCGMASLSAEKVTLDQRDYEKYSVSCQEDVTCPTAEETLPIELALEARQQNKY
ENYSTSFFIRDIIKPDPPKNLQMKPLKNSQVEVSWEYPDSWSTPHSYFSLKFFVRIQRK
KEKMKETEEGCNQKGAFLVEKTSTEVQCKGGNVCVQAQDRYYNSSCSKWACVPCR
VRS (SEQ ID No: 38);
PDL1-ScFv-PD1-ScFv-F-double-cleavable-I fusion protein LIC417: the amino acid sequence of chain 1 is SEQ ID No: 35, and the amino acid sequence of chain 2 is SEQ ID No: 39 (SEQ ID No: 18 + SEQ ID No: 26): 
QVQLVQSGAEVKKPGSSVKVSCKASGFTFTTYYISWVRQAPGQGLEYLGY
INMGSGGTNYNEKFKGRVTITADKSTSTAYMELSSLRSEDTAVYYCAIIGYFDYWGQG
TMVTVSSGSSGGSGGSGGSGDVVMTQSPLSLPVTLGQPASISCRSSQSLLDSDGGTYL
YWFQQRPGQSPRRLIYLVSTLGSGVPDRFSGSGSGTDFTLKISRVEAEDVGVYYCMQL
THWPYTFGQGTKLEIKEPKSCDKTHTCPPCPAPELLGGPSVFLFPPKPKDTLMISRTPE
VTCVVVDVSHEDPEVKFNWYVDGVEVHNAKTKPREEQYASTYRVVSVLTVLHQDW
LNGKEYKCKVSNKALPAPIEKTISKAKGQPREPQVCTLPPSRDELTKNQVSLSCAVKG
FYPSDIAVEWESNGQPENNYKTTPPVLDSDGSFFLVSKLTVDKSRWQQGNVFSCSVM
HEALHNHYTQKSLSLSPGKGSSGGSGGSGGSGLSGRSDNHGSSGGSGGSGGSGMWE
 
LEKDVYVVEVDWTPDAPGETVNLTCDTPEEDDITWTSDQRHGVIGSGKTLTITVKEFL
DAGQYTCHKGGETLSHSHLLLHKKENGIWSTEILKNFKNKTFLKCEAPNYSGRFTCS
WLVQRNMDLKFNIKSSSSSPDSRAVTCGMASLSAEKVTLDQRDYEKYSVSCQEDVTC
PTAEETLPIELALEARQQNKYENYSTSFFIRDIIKPDPPKNLQMKPLKNSQVEVSWEYP
DSWSTPHSYFSLKFFVRIQRKKEKMKETEEGCNQKGAFLVEKTSTEVQCKGGNVCV
QAQDRYYNSSCSKWACVPCRVRS (SEQ ID No: 39);
PDL1-ScFv-PD1-ScFv-F-right-cleavable-I fusion protein LIC418: the amino acid sequence of chain 1 is SEQ ID No: 33, and the amino acid sequence of chain 2 is SEQ ID No: 39;
PDL1-ScFv-PD1-ScFv-F-left-cleavable-I fusion protein LIC419: the amino acid sequence of chain 1 is SEQ ID No: 35, and the amino acid sequence of chain 2 is SEQ ID No: 38;
PIF-T fusion protein LIC420: the amino acid sequence of chain 1 is SEQ ID No: 40 (SEQ ID No: 19 + SEQ ID No: 5 + SEQ ID No: 21), the amino acid sequence of chain 2 is SEQ ID No: 41 (SEQ ID No: 19 + SEQ ID No: 5 + SEQ ID No: 22), and the amino acid sequence of chain 3 is SEQ ID No: 9: 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVGSSGGS
GGSGGSGRVIPVSGPARCLSQSRNLLKTTDDMVKTAREKLKHYSCTAEDIDHEDITRD
QTSTLKTCLPLELHKNESCLATRETSSTTRGSCLPPQKTSLMMTLCLGSIYEDLKMYQ
TEFQAINAALQNHNHQQIILDKGMLVAIDELMQSLNHNGETLRQKPPVGEADPYRVK
MKLCILLHAFSTRVVTINRVMGYLSSAGGGGSGGGGSEPKSCDKTHTCPPCPAPELLG
GPSVFLFPPKPKDTLMISRTPEVTCVVVDVSHEDPEVKFNWYVDGVEVHNAKTKPRE
EQYASTYRVVSVLTVLHQDWLNGKEYKCKVSNKALPAPIEKTISKAKGQPREPQVYT
LPPCRDELTKNQVSLWCLVKGFYPSDIAVEWESNGQPENNYKTTPPVLDSDGSFFLYS
KLTVDKSRWQQGNVFSCSVMHEALHNHYTQKSLSLSPGK (SEQ ID No: 40);
 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
 
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVGSSGGS
GGSGGSGMWELEKDVYVVEVDWTPDAPGETVNLTCDTPEEDDITWTSDQRHGVIGS
GKTLTITVKEFLDAGQYTCHKGGETLSHSHLLLHKKENGIWSTEILKNFKNKTFLKCE
APNYSGRFTCSWLVQRNMDLKFNIKSSSSSPDSRAVTCGMASLSAEKVTLDQRDYEK
YSVSCQEDVTCPTAEETLPIELALEARQQNKYENYSTSFFIRDIIKPDPPKNLQMKPLK
NSQVEVSWEYPDSWSTPHSYFSLKFFVRIQRKKEKMKETEEGCNQKGAFLVEKTSTE
VQCKGGNVCVQAQDRYYNSSCSKWACVPCRVRSGGGGSGGGGSEPKSCDKTHTCPP
CPAPELLGGPSVFLFPPKPKDTLMISRTPEVTCVVVDVSHEDPEVKFNWYVDGVEVH
NAKTKPREEQYASTYRVVSVLTVLHQDWLNGKEYKCKVSNKALPAPIEKTISKAKGQ
PREPQVCTLPPSRDELTKNQVSLSCAVKGFYPSDIAVEWESNGQPENNYKTTPPVLDS
DGSFFLVSKLTVDKSRWQQGNVFSCSVMHEALHNHYTQKSLSLSPGK (SEQ ID No:
41);
P-double-cleavable-IF-T fusion protein LIC421: the amino acid sequence of chain 1 is SEQ ID No: 42 (SEQ ID No: 19 + SEQ ID No: 7 + SEQ ID No: 21); the amino acid sequence of chain 2 is SEQ ID No: 43 (SEQ ID No: 19 + SEQ ID No: 7 + SEQ ID No: 22), and the amino acid sequence of chain 3 is SEQ ID No: 9: 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVGSSGGS
GGSGGSGLSGRSDNHGSSGGSGGSGGSGRVIPVSGPARCLSQSRNLLKTTDDMVKTA
REKLKHYSCTAEDIDHEDITRDQTSTLKTCLPLELHKNESCLATRETSSTTRGSCLPPQ
KTSLMMTLCLGSIYEDLKMYQTEFQAINAALQNHNHQQIILDKGMLVAIDELMQSLN
HNGETLRQKPPVGEADPYRVKMKLCILLHAFSTRVVTINRVMGYLSSAGGGGSGGGG
SEPKSCDKTHTCPPCPAPELLGGPSVFLFPPKPKDTLMISRTPEVTCVVVDVSHEDPEV
KFNWYVDGVEVHNAKTKPREEQYASTYRVVSVLTVLHQDWLNGKEYKCKVSNKAL
PAPIEKTISKAKGQPREPQVYTLPPCRDELTKNQVSLWCLVKGFYPSDIAVEWESNGQP
ENNYKTTPPVLDSDGSFFLYSKLTVDKSRWQQGNVFSCSVMHEALHNHYTQKSLSLS
PGK (SEQ ID No: 42);
 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVGSSGGS
GGSGGSGLSGRSDNHGSSGGSGGSGGSGMWELEKDVYVVEVDWTPDAPGETVNLT
CDTPEEDDITWTSDQRHGVIGSGKTLTITVKEFLDAGQYTCHKGGETLSHSHLLLHKK
ENGIWSTEILKNFKNKTFLKCEAPNYSGRFTCSWLVQRNMDLKFNIKSSSSSPDSRAV
TCGMASLSAEKVTLDQRDYEKYSVSCQEDVTCPTAEETLPIELALEARQQNKYENYS
TSFFIRDIIKPDPPKNLQMKPLKNSQVEVSWEYPDSWSTPHSYFSLKFFVRIQRKKEKM
KETEEGCNQKGAFLVEKTSTEVQCKGGNVCVQAQDRYYNSSCSKWACVPCRVRSGG
GGSGGGGSEPKSCDKTHTCPPCPAPELLGGPSVFLFPPKPKDTLMISRTPEVTCVVVD
VSHEDPEVKFNWYVDGVEVHNAKTKPREEQYASTYRVVSVLTVLHQDWLNGKEYK
CKVSNKALPAPIEKTISKAKGQPREPQVCTLPPSRDELTKNQVSLSCAVKGFYPSDIAV
EWESNGQPENNYKTTPPVLDSDGSFFLVSKLTVDKSRWQQGNVFSCSVMHEALHNH
YTQKSLSLSPGK (SEQ ID No: 43);
P-right-cleavable-IF-T fusion protein LIC422: the amino acid sequence of chain 1 is SEQ ID No: 40, the amino acid sequence of chain 2 is SEQ ID No: 43, and the amino acid sequence of chain 3 is SEQ ID No: 9;
P-left-cleavable-IF-T fusion protein LIC423: the amino acid sequence of chain 1 is SEQ ID No: 42, the amino acid sequence of chain 2 is SEQ ID No: 41, and the amino acid sequence of chain 3 is SEQ ID No: 9;
PIF-Y fusion protein LIC424: the amino acid sequence of chain 1 is SEQ ID No: 44 (SEQ ID No: 9 + SEQ ID No: 5 + SEQ ID No: 21), and the amino acid sequence of chain 2 is SEQ ID No: 40: 
DIQMTQSPSSLSASVGDRVTITCRASQDVSTAVAWYQQKPGKAPKLLIYSA
SFLYSGVPSRFSGSGSGTDFTLTISSLQPEDFATYYCQQYLYHPATFGQGTKVEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGECGSSGGSGGSGGS
GRVIPVSGPARCLSQSRNLLKTTDDMVKTAREKLKHYSCTAEDIDHEDITRDQTSTLK
TCLPLELHKNESCLATRETSSTTRGSCLPPQKTSLMMTLCLGSIYEDLKMYQTEFQAIN
 
AALQNHNHQQIILDKGMLVAIDELMQSLNHNGETLRQKPPVGEADPYRVKMKLCILL
HAFSTRVVTINRVMGYLSSAGGGGSGGGGSEPKSCDKTHTCPPCPAPELLGGPSVFLF
PPKPKDTLMISRTPEVTCVVVDVSHEDPEVKFNWYVDGVEVHNAKTKPREEQYAST
YRVVSVLTVLHQDWLNGKEYKCKVSNKALPAPIEKTISKAKGQPREPQVYTLPPCRD
ELTKNQVSLWCLVKGFYPSDIAVEWESNGQPENNYKTTPPVLDSDGSFFLYSKLTVDK
SRWQQGNVFSCSVMHEALHNHYTQKSLSLSPGK (SEQ ID No: 44);
P-double-cleavable-IF-Y fusion protein LIC425: the amino acid sequence of chain 1 is SEQ ID No: 45 (SEQ ID No: 9 + SEQ ID No: 7 + SEQ ID No: 21), and the amino acid sequence of chain 2 is SEQ ID No: 43: 
DIQMTQSPSSLSASVGDRVTITCRASQDVSTAVAWYQQKPGKAPKLLIYSA
SFLYSGVPSRFSGSGSGTDFTLTISSLQPEDFATYYCQQYLYHPATFGQGTKVEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGECGSSGGSGGSGGS
GLSGRSDNHGSSGGSGGSGGSGRVIPVSGPARCLSQSRNLLKTTDDMVKTAREKLKH
YSCTAEDIDHEDITRDQTSTLKTCLPLELHKNESCLATRETSSTTRGSCLPPQKTSLMM
TLCLGSIYEDLKMYQTEFQAINAALQNHNHQQIILDKGMLVAIDELMQSLNHNGETL
RQKPPVGEADPYRVKMKLCILLHAFSTRVVTINRVMGYLSSAGGGGSGGGGSEPKSC
DKTHTCPPCPAPELLGGPSVFLFPPKPKDTLMISRTPEVTCVVVDVSHEDPEVKFNWY
VDGVEVHNAKTKPREEQYASTYRVVSVLTVLHQDWLNGKEYKCKVSNKALPAPIEK
TISKAKGQPREPQVYTLPPCRDELTKNQVSLWCLVKGFYPSDIAVEWESNGQPENNYK
TTPPVLDSDGSFFLYSKLTVDKSRWQQGNVFSCSVMHEALHNHYTQKSLSLSPGK
(SEQ ID No: 45);
P-right-cleavable-IF-Y fusion protein LIC426: the amino acid sequence of chain 1 is SEQ ID No: 44, and the amino acid sequence of chain 2 is SEQ ID No: 43;
P-left-cleavable-IF-Y fusion protein LIC427: the amino acid sequence of chain 1 is SEQ ID No: 45, and the amino acid sequence of chain 2 is SEQ ID No: 40;
PI homodimer fusion protein LIC428: the amino acid sequence of chain 1 is SEQ ID No: 46 (SEQ ID No: 10 + SEQ ID No: 5 + SEQ ID No: 1 + SEQ ID No: 75 + SEQ ID No: 2), and the amino acid sequence of chain 2 is SEQ ID No: 9: EVQLVESGGGLVQPGGSLRLSCAASGFTFSDSWIHWVRQAPGKGLEWVA 
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYV
DGVEVHNAKTKPREEQYASTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGKGSS
GGSGGSGGSGRVIPVSGPARCLSQSRNLLKTTDDMVKTAREKLKHYSCTAEDIDHEDI
TRDQTSTLKTCLPLELHKNESCLATRETSSTTRGSCLPPQKTSLMMTLCLGSIYEDLK
MYQTEFQAINAALQNHNHQQIILDKGMLVAIDELMQSLNHNGETLRQKPPVGEADPY
RVKMKLCILLHAFSTRVVTINRVMGYLSSAGGGGSGGGGSGGGGSGGGGSGGGGSM
WELEKDVYVVEVDWTPDAPGETVNLTCDTPEEDDITWTSDQRHGVIGSGKTLTITVK
EFLDAGQYTCHKGGETLSHSHLLLHKKENGIWSTEILKNFKNKTFLKCEAPNYSGRFT
CSWLVQRNMDLKFNIKSSSSSPDSRAVTCGMASLSAEKVTLDQRDYEKYSVSCQEDV
TCPTAEETLPIELALEARQQNKYENYSTSFFIRDIIKPDPPKNLQMKPLKNSQVEVSWE
YPDSWSTPHSYFSLKFFVRIQRKKEKMKETEEGCNQKGAFLVEKTSTEVQCKGGNVC
VQAQDRYYNSSCSKWACVPCRVRS (SEQ ID No: 46);
PIF-T homodimer fusion protein LIC429: the amino acid sequence of chain 1 is SEQ ID No: 47 (SEQ ID No: 19 + SEQ ID No: 5 + SEQ ID No: 1 + SEQ ID No: 75 + SEQ ID No: 2 + SEQ ID No: 6 + PDL1 heavy chain Fc (Mut)), and the amino acid sequence of chain 2 is SEQ ID No: 9: 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVGSSGGS
GGSGGSGRVIPVSGPARCLSQSRNLLKTTDDMVKTAREKLKHYSCTAEDIDHEDITRD
QTSTLKTCLPLELHKNESCLATRETSSTTRGSCLPPQKTSLMMTLCLGSIYEDLKMYQ
TEFQAINAALQNHNHQQIILDKGMLVAIDELMQSLNHNGETLRQKPPVGEADPYRVK
MKLCILLHAFSTRVVTINRVMGYLSSAGGGGSGGGGSGGGGSGGGGSGGGGSMWEL
EKDVYVVEVDWTPDAPGETVNLTCDTPEEDDITWTSDQRHGVIGSGKTLTITVKEFL
 
DAGQYTCHKGGETLSHSHLLLHKKENGIWSTEILKNFKNKTFLKCEAPNYSGRFTCS
WLVQRNMDLKFNIKSSSSSPDSRAVTCGMASLSAEKVTLDQRDYEKYSVSCQEDVTC
PTAEETLPIELALEARQQNKYENYSTSFFIRDIIKPDPPKNLQMKPLKNSQVEVSWEYP
DSWSTPHSYFSLKFFVRIQRKKEKMKETEEGCNQKGAFLVEKTSTEVQCKGGNVCV
QAQDRYYNSSCSKWACVPCRVRSGGGGSGGGGSEPKSCDKTHTCPPCPAPELLGGPS
VFLFPPKPKDTLMISRTPEVTCVVVDVSHEDPEVKFNWYVDGVEVHNAKTKPREEQY
ASTYRVVSVLTVLHQDWLNGKEYKCKVSNKALPAPIEKTISKAKGQPREPQVYTLPPS
REEMTKNQVSLTCLVKGFYPSDIAVEWESNGQPENNYKTTPPVLDSDGSFFLYSKLTV
DKSRWQQGNVFSCSVMHEALHNHYTQKSLSLSPGK (SEQ ID No: 47);
P-double-cleavable-IF-T homodimer fusion protein LIC430: the amino acid sequence of chain 1 is SEQ ID No: 48 (SEQ ID No: 19 + SEQ ID No: 7 + SEQ ID No: 1 + SEQ ID No: 75 + SEQ ID No: 2 + SEQ ID No: 6 + PDL1 heavy chain Fc (Mut)), and the amino acid sequence of chain 2 is SEQ ID No: 9: 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVGSSGGS
GGSGGSGLSGRSDNHGSSGGSGGSGGSGRVIPVSGPARCLSQSRNLLKTTDDMVKTA
REKLKHYSCTAEDIDHEDITRDQTSTLKTCLPLELHKNESCLATRETSSTTRGSCLPPQ
KTSLMMTLCLGSIYEDLKMYQTEFQAINAALQNHNHQQIILDKGMLVAIDELMQSLN
HNGETLRQKPPVGEADPYRVKMKLCILLHAFSTRVVTINRVMGYLSSAGGGGSGGGG
SGGGGSGGGGSGGGGSMWELEKDVYVVEVDWTPDAPGETVNLTCDTPEEDDITWT
SDQRHGVIGSGKTLTITVKEFLDAGQYTCHKGGETLSHSHLLLHKKENGIWSTEILKN
FKNKTFLKCEAPNYSGRFTCSWLVQRNMDLKFNIKSSSSSPDSRAVTCGMASLSAEK
VTLDQRDYEKYSVSCQEDVTCPTAEETLPIELALEARQQNKYENYSTSFFIRDIIKPDP
PKNLQMKPLKNSQVEVSWEYPDSWSTPHSYFSLKFFVRIQRKKEKMKETEEGCNQK
GAFLVEKTSTEVQCKGGNVCVQAQDRYYNSSCSKWACVPCRVRSGGGGSGGGGSEP
KSCDKTHTCPPCPAPELLGGPSVFLFPPKPKDTLMISRTPEVTCVVVDVSHEDPEVKFN
WYVDGVEVHNAKTKPREEQYASTYRVVSVLTVLHQDWLNGKEYKCKVSNKALPAPI
EKTISKAKGQPREPQVYTLPPSREEMTKNQVSLTCLVKGFYPSDIAVEWESNGQPENN
 
YKTTPPVLDSDGSFFLYSKLTVDKSRWQQGNVFSCSVMHEALHNHYTQKSLSLSPGK
(SEQ ID No: 48);
P-single-cleavable-IF-T homodimer fusion protein LIC431: the amino acid sequence of chain 1 is SEQ ID No: 49 (SEQ ID No: 19 + SEQ ID No: 5 + SEQ ID No: 1 + SEQ ID No: 75 + SEQ ID No: 2 + SEQ ID No: 6 + SEQ ID No: 3), the amino acid sequence of chain 2 is SEQ ID No: 50 (SEQ ID No: 19 + SEQ ID No: 7 + SEQ ID No: 1 + SEQ ID No: 75 + SEQ ID No: 2 + SEQ ID No: 6 + SEQ ID No: 4), and the amino acid sequence of chain 3 is SEQ ID No: 9: 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVGSSGGS
GGSGGSGRVIPVSGPARCLSQSRNLLKTTDDMVKTAREKLKHYSCTAEDIDHEDITRD
QTSTLKTCLPLELHKNESCLATRETSSTTRGSCLPPQKTSLMMTLCLGSIYEDLKMYQ
TEFQAINAALQNHNHQQIILDKGMLVAIDELMQSLNHNGETLRQKPPVGEADPYRVK
MKLCILLHAFSTRVVTINRVMGYLSSAGGGGSGGGGSGGGGSGGGGSGGGGSMWEL
EKDVYVVEVDWTPDAPGETVNLTCDTPEEDDITWTSDQRHGVIGSGKTLTITVKEFL
DAGQYTCHKGGETLSHSHLLLHKKENGIWSTEILKNFKNKTFLKCEAPNYSGRFTCS
WLVQRNMDLKFNIKSSSSSPDSRAVTCGMASLSAEKVTLDQRDYEKYSVSCQEDVTC
PTAEETLPIELALEARQQNKYENYSTSFFIRDIIKPDPPKNLQMKPLKNSQVEVSWEYP
DSWSTPHSYFSLKFFVRIQRKKEKMKETEEGCNQKGAFLVEKTSTEVQCKGGNVCV
QAQDRYYNSSCSKWACVPCRVRSGGGGSGGGGSEPKSCDKTHTCPPCPAPELLGGPS
VFLFPPKPKDTLMISRTPEVTCVVVDVSHEDPEVKFNWYVDGVEVHNAKTKPREEQY
ASTYRVVSVLTVLHQDWLNGKEYKCKVSNKALPAPIEKTISKAKGQPREPQVYTLPP
CRDELTKNQVSLWCLVKGFYPSDIAVEWESNGQPENNYKTTPPVLDSDGSFFLYSKLT
VDKSRWQQGNVFSCSVMHEALHNHYTQKSLSLSPGK (SEQ ID No: 49);
 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVGSSGGS
 
GGSGGSGLSGRSDNHGSSGGSGGSGGSGRVIPVSGPARCLSQSRNLLKTTDDMVKTA
REKLKHYSCTAEDIDHEDITRDQTSTLKTCLPLELHKNESCLATRETSSTTRGSCLPPQ
KTSLMMTLCLGSIYEDLKMYQTEFQAINAALQNHNHQQIILDKGMLVAIDELMQSLN
HNGETLRQKPPVGEADPYRVKMKLCILLHAFSTRVVTINRVMGYLSSAGGGGSGGGG
SGGGGSGGGGSGGGGSMWELEKDVYVVEVDWTPDAPGETVNLTCDTPEEDDITWT
SDQRHGVIGSGKTLTITVKEFLDAGQYTCHKGGETLSHSHLLLHKKENGIWSTEILKN
FKNKTFLKCEAPNYSGRFTCSWLVQRNMDLKFNIKSSSSSPDSRAVTCGMASLSAEK
VTLDQRDYEKYSVSCQEDVTCPTAEETLPIELALEARQQNKYENYSTSFFIRDIIKPDP
PKNLQMKPLKNSQVEVSWEYPDSWSTPHSYFSLKFFVRIQRKKEKMKETEEGCNQK
GAFLVEKTSTEVQCKGGNVCVQAQDRYYNSSCSKWACVPCRVRSGGGGSGGGGSEP
KSCDKTHTCPPCPAPELLGGPSVFLFPPKPKDTLMISRTPEVTCVVVDVSHEDPEVKFN
WYVDGVEVHNAKTKPREEQYASTYRVVSVLTVLHQDWLNGKEYKCKVSNKALPAPI
EKTISKAKGQPREPQVCTLPPSRDELTKNQVSLSCAVKGFYPSDIAVEWESNGQPENN
YKTTPPVLDSDGSFFLVSKLTVDKSRWQQGNVFSCSVMHEALHNHYTQKSLSLSPGK
(SEQ ID No: 50);
PIF-Y homodimer fusion protein LIC432: the amino acid sequence of chain 1 is SEQ ID No: 51 (SEQ ID No: 9 + SEQ ID No: 5 + SEQ ID No: 1 + SEQ ID No: 75 + SEQ ID No: 2 + SEQ ID No: 6 + SEQ ID No: 3), and the amino acid sequence of chain 2 is SEQ ID No: 52 (SEQ ID No: 19 + SEQ ID No: 5 + SEQ ID No: 1 + SEQ ID No: 75 + SEQ ID No: 2 + SEQ ID No: 6 + SEQ ID No: 4): 
DIQMTQSPSSLSASVGDRVTITCRASQDVSTAVAWYQQKPGKAPKLLIYSA
SFLYSGVPSRFSGSGSGTDFTLTISSLQPEDFATYYCQQYLYHPATFGQGTKVEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGECGSSGGSGGSGGS
GRVIPVSGPARCLSQSRNLLKTTDDMVKTAREKLKHYSCTAEDIDHEDITRDQTSTLK
TCLPLELHKNESCLATRETSSTTRGSCLPPQKTSLMMTLCLGSIYEDLKMYQTEFQAIN
AALQNHNHQQIILDKGMLVAIDELMQSLNHNGETLRQKPPVGEADPYRVKMKLCILL
HAFSTRVVTINRVMGYLSSAGGGGSGGGGSGGGGSGGGGSGGGGSMWELEKDVYV
VEVDWTPDAPGETVNLTCDTPEEDDITWTSDQRHGVIGSGKTLTITVKEFLDAGQYT
CHKGGETLSHSHLLLHKKENGIWSTEILKNFKNKTFLKCEAPNYSGRFTCSWLVQRN
 
MDLKFNIKSSSSSPDSRAVTCGMASLSAEKVTLDQRDYEKYSVSCQEDVTCPTAEETL
PIELALEARQQNKYENYSTSFFIRDIIKPDPPKNLQMKPLKNSQVEVSWEYPDSWSTP
HSYFSLKFFVRIQRKKEKMKETEEGCNQKGAFLVEKTSTEVQCKGGNVCVQAQDRY
YNSSCSKWACVPCRVRSGGGGSGGGGSEPKSCDKTHTCPPCPAPELLGGPSVFLFPPK
PKDTLMISRTPEVTCVVVDVSHEDPEVKFNWYVDGVEVHNAKTKPREEQYASTYRV
VSVLTVLHQDWLNGKEYKCKVSNKALPAPIEKTISKAKGQPREPQVYTLPPCRDELTK
NQVSLWCLVKGFYPSDIAVEWESNGQPENNYKTTPPVLDSDGSFFLYSKLTVDKSRW
QQGNVFSCSVMHEALHNHYTQKSLSLSPGK (SEQ ID No: 51);
 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVGSSGGS
GGSGGSGRVIPVSGPARCLSQSRNLLKTTDDMVKTAREKLKHYSCTAEDIDHEDITRD
QTSTLKTCLPLELHKNESCLATRETSSTTRGSCLPPQKTSLMMTLCLGSIYEDLKMYQ
TEFQAINAALQNHNHQQIILDKGMLVAIDELMQSLNHNGETLRQKPPVGEADPYRVK
MKLCILLHAFSTRVVTINRVMGYLSSAGGGGSGGGGSGGGGSGGGGSGGGGSMWEL
EKDVYVVEVDWTPDAPGETVNLTCDTPEEDDITWTSDQRHGVIGSGKTLTITVKEFL
DAGQYTCHKGGETLSHSHLLLHKKENGIWSTEILKNFKNKTFLKCEAPNYSGRFTCS
WLVQRNMDLKFNIKSSSSSPDSRAVTCGMASLSAEKVTLDQRDYEKYSVSCQEDVTC
PTAEETLPIELALEARQQNKYENYSTSFFIRDIIKPDPPKNLQMKPLKNSQVEVSWEYP
DSWSTPHSYFSLKFFVRIQRKKEKMKETEEGCNQKGAFLVEKTSTEVQCKGGNVCV
QAQDRYYNSSCSKWACVPCRVRSGGGGSGGGGSEPKSCDKTHTCPPCPAPELLGGPS
VFLFPPKPKDTLMISRTPEVTCVVVDVSHEDPEVKFNWYVDGVEVHNAKTKPREEQY
ASTYRVVSVLTVLHQDWLNGKEYKCKVSNKALPAPIEKTISKAKGQPREPQVCTLPPS
RDELTKNQVSLSCAVKGFYPSDIAVEWESNGQPENNYKTTPPVLDSDGSFFLVSKLTV
DKSRWQQGNVFSCSVMHEALHNHYTQKSLSLSPGK (SEQ ID No: 52);
P-double-cleavable-IF-Y homodimer fusion protein LIC433: the amino acid sequence of chain 1 is SEQ ID No: 53 (SEQ ID No: 9 + SEQ ID No: 7 + SEQ ID No: 1 + SEQ ID No: 75 + SEQ ID No: 2 + SEQ ID No: 6 + SEQ ID No: 3), and the amino acid sequence of chain 2 is SEQ ID No: 50: 
DIQMTQSPSSLSASVGDRVTITCRASQDVSTAVAWYQQKPGKAPKLLIYSA
SFLYSGVPSRFSGSGSGTDFTLTISSLQPEDFATYYCQQYLYHPATFGQGTKVEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGECGSSGGSGGSGGS
GLSGRSDNHGSSGGSGGSGGSGRVIPVSGPARCLSQSRNLLKTTDDMVKTAREKLKH
YSCTAEDIDHEDITRDQTSTLKTCLPLELHKNESCLATRETSSTTRGSCLPPQKTSLMM
TLCLGSIYEDLKMYQTEFQAINAALQNHNHQQIILDKGMLVAIDELMQSLNHNGETL
RQKPPVGEADPYRVKMKLCILLHAFSTRVVTINRVMGYLSSAGGGGSGGGGSGGGGS
GGGGSGGGGSMWELEKDVYVVEVDWTPDAPGETVNLTCDTPEEDDITWTSDQRHG
VIGSGKTLTITVKEFLDAGQYTCHKGGETLSHSHLLLHKKENGIWSTEILKNFKNKTF
LKCEAPNYSGRFTCSWLVQRNMDLKFNIKSSSSSPDSRAVTCGMASLSAEKVTLDQR
DYEKYSVSCQEDVTCPTAEETLPIELALEARQQNKYENYSTSFFIRDIIKPDPPKNLQM
KPLKNSQVEVSWEYPDSWSTPHSYFSLKFFVRIQRKKEKMKETEEGCNQKGAFLVEK
TSTEVQCKGGNVCVQAQDRYYNSSCSKWACVPCRVRSGGGGSGGGGSEPKSCDKTH
TCPPCPAPELLGGPSVFLFPPKPKDTLMISRTPEVTCVVVDVSHEDPEVKFNWYVDGV
EVHNAKTKPREEQYASTYRVVSVLTVLHQDWLNGKEYKCKVSNKALPAPIEKTISKA
KGQPREPQVYTLPPCRDELTKNQVSLWCLVKGFYPSDIAVEWESNGQPENNYKTTPP
VLDSDGSFFLYSKLTVDKSRWQQGNVFSCSVMHEALHNHYTQKSLSLSPGK (SEQ ID
No: 53);
P-left-cleavable-IF-Y homodimer fusion protein LIC434: the amino acid sequence of chain 1 is SEQ ID No: 53, and the amino acid sequence of chain 2 is SEQ ID No: 52;
P-right-cleavable-IF-Y homodimer fusion protein LIC435: the amino acid sequence of chain 1 is SEQ ID No: 51, and the amino acid sequence of chain 2 is SEQ ID No: 50;
PI tandem single-molecule fusion protein LIC436: the amino acid sequence of chain 1 is SEQ ID No: 54 (SEQ ID No: 11 + SEQ ID No: 5 + SEQ ID No: 1 + SEQ ID No: 75 + SEQ ID No: 2), the amino acid sequence of chain 2 is SEQ ID No: 12, and the amino acid sequence of chain 3 is SEQ ID No: 9: 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
 
LTSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYV
DGVEVHNAKTKPREEQYASTYRVVSVLTVLHQDWLNGKEYKCKVSNKALPAPIEKTI
SKAKGQPREPQVYTLPPCRDELTKNQVSLWCLVKGFYPSDIAVEWESNGQPENNYKT
TPPVLDSDGSFFLYSKLTVDKSRWQQGNVFSCSVMHEALHNHYTQKSLSLSPGKGSS
GGSGGSGGSGRVIPVSGPARCLSQSRNLLKTTDDMVKTAREKLKHYSCTAEDIDHEDI
TRDQTSTLKTCLPLELHKNESCLATRETSSTTRGSCLPPQKTSLMMTLCLGSIYEDLK
MYQTEFQAINAALQNHNHQQIILDKGMLVAIDELMQSLNHNGETLRQKPPVGEADPY
RVKMKLCILLHAFSTRVVTINRVMGYLSSAGGGGSGGGGSGGGGSGGGGSGGGGSM
WELEKDVYVVEVDWTPDAPGETVNLTCDTPEEDDITWTSDQRHGVIGSGKTLTITVK
EFLDAGQYTCHKGGETLSHSHLLLHKKENGIWSTEILKNFKNKTFLKCEAPNYSGRFT
CSWLVQRNMDLKFNIKSSSSSPDSRAVTCGMASLSAEKVTLDQRDYEKYSVSCQEDV
TCPTAEETLPIELALEARQQNKYENYSTSFFIRDIIKPDPPKNLQMKPLKNSQVEVSWE
YPDSWSTPHSYFSLKFFVRIQRKKEKMKETEEGCNQKGAFLVEKTSTEVQCKGGNVC
VQAQDRYYNSSCSKWACVPCRVRS (SEQ ID No: 54);
PIF-T tandem single cytokine fusion protein LIC437: the amino acid sequence of chain 1 is SEQ ID No: 55 (SEQ ID No: 19 + SEQ ID No: 75 + SEQ ID No: 3), the amino acid sequence of chain 2 is SEQ ID No: 52, and the amino acid sequence of chain 3 is SEQ ID No: 9: 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVGGGGS
GGGGSGGGGSGGGGSGGGGSEPKSCDKTHTCPPCPAPELLGGPSVFLFPPKPKDTLMI
SRTPEVTCVVVDVSHEDPEVKFNWYVDGVEVHNAKTKPREEQYASTYRVVSVLTVL
HQDWLNGKEYKCKVSNKALPAPIEKTISKAKGQPREPQVYTLPPCRDELTKNQVSLW
CLVKGFYPSDIAVEWESNGQPENNYKTTPPVLDSDGSFFLYSKLTVDKSRWQQGNVFS
CSVMHEALHNHYTQKSLSLSPGK (SEQ ID No: 55);
PDL1-ScFv-IF tandem single cytokine fusion protein LIC438: the amino acid sequence of chain 1 is SEQ ID No: 56 (SEQ ID No: 17 + SEQ ID No: 75 + SEQ ID No: 3), and the amino acid sequence of chain 2 is SEQ ID No: 57 (SEQ ID No: 17 + SEQ ID No: 5 + SEQ ID No: 1 + SEQ ID No: 75 + SEQ ID No: 2 + SEQ ID No: 6 + SEQ ID No: 4): 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSGSSGGSGGSGGSGDIQMTQSPSSLSASVGDRVTITCRASQDVSTAV
AWYQQKPGKAPKLLIYSASFLYSGVPSRFSGSGSGTDFTLTISSLQPEDFATYYCQQYL
YHPATFGQGTKVEIKGGGGSGGGGSGGGGSGGGGSGGGGSEPKSCDKTHTCPPCPAP
ELLGGPSVFLFPPKPKDTLMISRTPEVTCVVVDVSHEDPEVKFNWYVDGVEVHNAKT
KPREEQYASTYRVVSVLTVLHQDWLNGKEYKCKVSNKALPAPIEKTISKAKGQPREP
QVYTLPPCRDELTKNQVSLWCLVKGFYPSDIAVEWESNGQPENNYKTTPPVLDSDGSF
FLYSKLTVDKSRWQQGNVFSCSVMHEALHNHYTQKSLSLSPGK (SEQ ID No: 56);
 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSGSSGGSGGSGGSGDIQMTQSPSSLSASVGDRVTITCRASQDVSTAV
AWYQQKPGKAPKLLIYSASFLYSGVPSRFSGSGSGTDFTLTISSLQPEDFATYYCQQYL
YHPATFGQGTKVEIKGSSGGSGGSGGSGRVIPVSGPARCLSQSRNLLKTTDDMVKTAR
EKLKHYSCTAEDIDHEDITRDQTSTLKTCLPLELHKNESCLATRETSSTTRGSCLPPQK
TSLMMTLCLGSIYEDLKMYQTEFQAINAALQNHNHQQIILDKGMLVAIDELMQSLNH
NGETLRQKPPVGEADPYRVKMKLCILLHAFSTRVVTINRVMGYLSSAGGGGSGGGGS
GGGGSGGGGSGGGGSMWELEKDVYVVEVDWTPDAPGETVNLTCDTPEEDDITWTS
DQRHGVIGSGKTLTITVKEFLDAGQYTCHKGGETLSHSHLLLHKKENGIWSTEILKNF
KNKTFLKCEAPNYSGRFTCSWLVQRNMDLKFNIKSSSSSPDSRAVTCGMASLSAEKV
TLDQRDYEKYSVSCQEDVTCPTAEETLPIELALEARQQNKYENYSTSFFIRDIIKPDPP
KNLQMKPLKNSQVEVSWEYPDSWSTPHSYFSLKFFVRIQRKKEKMKETEEGCNQKG
AFLVEKTSTEVQCKGGNVCVQAQDRYYNSSCSKWACVPCRVRSGGGGSGGGGSEPK
SCDKTHTCPPCPAPELLGGPSVFLFPPKPKDTLMISRTPEVTCVVVDVSHEDPEVKFN
WYVDGVEVHNAKTKPREEQYASTYRVVSVLTVLHQDWLNGKEYKCKVSNKALPAPI
EKTISKAKGQPREPQVCTLPPSRDELTKNQVSLSCAVKGFYPSDIAVEWESNGQPENN
YKTTPPVLDSDGSFFLVSKLTVDKSRWQQGNVFSCSVMHEALHNHYTQKSLSLSPGK
(SEQ ID No: 57);
ence of chain 1 is SEQ ID No: 56, and the amino acid sequence of chain 2 is SEQ ID No: 58 (SEQ ID No: 18 + SEQ ID No: 5 + SEQ ID No: 1 + SEQ ID No: 75 + SEQ ID No: 2 + SEQ ID No: 6 + SEQ ID No: 4): 
QVQLVQSGAEVKKPGSSVKVSCKASGFTFTTYYISWVRQAPGQGLEYLGY
INMGSGGTNYNEKFKGRVTITADKSTSTAYMELSSLRSEDTAVYYCAIIGYFDYWGQG
TMVTVSSGSSGGSGGSGGSGDVVMTQSPLSLPVTLGQPASISCRSSQSLLDSDGGTYL
YWFQQRPGQSPRRLIYLVSTLGSGVPDRFSGSGSGTDFTLKISRVEAEDVGVYYCMQL
THWPYTFGQGTKLEIKGSSGGSGGSGGSGRVIPVSGPARCLSQSRNLLKTTDDMVKT
AREKLKHYSCTAEDIDHEDITRDQTSTLKTCLPLELHKNESCLATRETSSTTRGSCLPP
QKTSLMMTLCLGSIYEDLKMYQTEFQAINAALQNHNHQQIILDKGMLVAIDELMQSL
NHNGETLRQKPPVGEADPYRVKMKLCILLHAFSTRVVTINRVMGYLSSAGGGGSGGG
GSGGGGSGGGGSGGGGSMWELEKDVYVVEVDWTPDAPGETVNLTCDTPEEDDITW
TSDQRHGVIGSGKTLTITVKEFLDAGQYTCHKGGETLSHSHLLLHKKENGIWSTEILK
NFKNKTFLKCEAPNYSGRFTCSWLVQRNMDLKFNIKSSSSSPDSRAVTCGMASLSAE
KVTLDQRDYEKYSVSCQEDVTCPTAEETLPIELALEARQQNKYENYSTSFFIRDIIKPD
PPKNLQMKPLKNSQVEVSWEYPDSWSTPHSYFSLKFFVRIQRKKEKMKETEEGCNQ
KGAFLVEKTSTEVQCKGGNVCVQAQDRYYNSSCSKWACVPCRVRSGGGGSGGGGSE
PKSCDKTHTCPPCPAPELLGGPSVFLFPPKPKDTLMISRTPEVTCVVVDVSHEDPEVKF
NWYVDGVEVHNAKTKPREEQYASTYRVVSVLTVLHQDWLNGKEYKCKVSNKALPA
PIEKTISKAKGQPREPQVCTLPPSRDELTKNQVSLSCAVKGFYPSDIAVEWESNGQPEN
NYKTTPPVLDSDGSFFLVSKLTVDKSRWQQGNVFSCSVMHEALHNHYTQKSLSLSPG
K (SEQ ID No: 58);
FI-PDL1-ScFv tandem single cytokine fusion protein LIC440: the amino acid sequence of chain 1 is SEQ ID No: 59 (SEQ ID No: 3 + SEQ ID No: 75 + SEQ ID No: 17), and the amino acid sequence of chain 2 is SEQ ID No: 60 (SEQ ID No: 4 + SEQ ID No: 5 + SEQ ID No: 1 + SEQ ID No: 75 + SEQ ID No: 2 + SEQ ID No: 6 + SEQ ID No: 17): 
EPKSCDKTHTCPPCPAPELLGGPSVFLFPPKPKDTLMISRTPEVTCVVVDVS
HEDPEVKFNWYVDGVEVHNAKTKPREEQYASTYRVVSVLTVLHQDWLNGKEYKCK
VSNKALPAPIEKTISKAKGQPREPQVYTLPPCRDELTKNQVSLWCLVKGFYPSDIAVE
 
WESNGQPENNYKTTPPVLDSDGSFFLYSKLTVDKSRWQQGNVFSCSVMHEALHNHY
TQKSLSLSPGKGGGGSGGGGSGGGGSGGGGSGGGGSEVQLVESGGGLVQPGGSLRLS
CAASGFTFSDSWIHWVRQAPGKGLEWVAWISPYGGSTYYADSVKGRFTISADTSKNT
AYLQMNSLRAEDTAVYYCARRHWPGGFDYWGQGTLVTVSSGSSGGSGGSGGSGDIQ
MTQSPSSLSASVGDRVTITCRASQDVSTAVAWYQQKPGKAPKLLIYSASFLYSGVPSRF
SGSGSGTDFTLTISSLQPEDFATYYCQQYLYHPATFGQGTKVEIK (SEQ ID No: 59);
 
EPKSCDKTHTCPPCPAPELLGGPSVFLFPPKPKDTLMISRTPEVTCVVVDVS
HEDPEVKFNWYVDGVEVHNAKTKPREEQYASTYRVVSVLTVLHQDWLNGKEYKCK
VSNKALPAPIEKTISKAKGQPREPQVCTLPPSRDELTKNQVSLSCAVKGFYPSDIAVEW
ESNGQPENNYKTTPPVLDSDGSFFLVSKLTVDKSRWQQGNVFSCSVMHEALHNHYT
QKSLSLSPGKGSSGGSGGSGGSGRVIPVSGPARCLSQSRNLLKTTDDMVKTAREKLKH
YSCTAEDIDHEDITRDQTSTLKTCLPLELHKNESCLATRETSSTTRGSCLPPQKTSLMM
TLCLGSIYEDLKMYQTEFQAINAALQNHNHQQIILDKGMLVAIDELMQSLNHNGETL
RQKPPVGEADPYRVKMKLCILLHAFSTRVVTINRVMGYLSSAGGGGSGGGGSGGGGS
GGGGSGGGGSMWELEKDVYVVEVDWTPDAPGETVNLTCDTPEEDDITWTSDQRHG
VIGSGKTLTITVKEFLDAGQYTCHKGGETLSHSHLLLHKKENGIWSTEILKNFKNKTF
LKCEAPNYSGRFTCSWLVQRNMDLKFNIKSSSSSPDSRAVTCGMASLSAEKVTLDQR
DYEKYSVSCQEDVTCPTAEETLPIELALEARQQNKYENYSTSFFIRDIIKPDPPKNLQM
KPLKNSQVEVSWEYPDSWSTPHSYFSLKFFVRIQRKKEKMKETEEGCNQKGAFLVEK
TSTEVQCKGGNVCVQAQDRYYNSSCSKWACVPCRVRSGGGGSGGGGSEVQLVESGG
GLVQPGGSLRLSCAASGFTFSDSWIHWVRQAPGKGLEWVAWISPYGGSTYYADSVKG
RFTISADTSKNTAYLQMNSLRAEDTAVYYCARRHWPGGFDYWGQGTLVTVSSGSSG
GSGGSGGSGDIQMTQSPSSLSASVGDRVTITCRASQDVSTAVAWYQQKPGKAPKLLIY
SASFLYSGVPSRFSGSGSGTDFTLTISSLQPEDFATYYCQQYLYHPATFGQGTKVEIK
(SEQ ID No: 60);
FI-PDL1-ScFv-PD1-ScFv tandem single cytokine fusion protein LIC441: the amino acid sequence of chain 1 is SEQ ID No: 59, and the amino acid sequence of chain 2 is SEQ ID No: 61 (SEQ ID No: 4 + SEQ ID No: 5 + SEQ ID No: 1 + SEQ ID No: 75 + SEQ ID No: 2 + SEQ ID No: 6 + SEQ ID No: 18): EPKSCDKTHTCPPCPAPELLGGPSVFLFPPKPKDTLMISRTPEVTCVVVDVS 
HEDPEVKFNWYVDGVEVHNAKTKPREEQYASTYRVVSVLTVLHQDWLNGKEYKCK
VSNKALPAPIEKTISKAKGQPREPQVCTLPPSRDELTKNQVSLSCAVKGFYPSDIAVEW
ESNGQPENNYKTTPPVLDSDGSFFLVSKLTVDKSRWQQGNVFSCSVMHEALHNHYT
QKSLSLSPGKGSSGGSGGSGGSGRVIPVSGPARCLSQSRNLLKTTDDMVKTAREKLKH
YSCTAEDIDHEDITRDQTSTLKTCLPLELHKNESCLATRETSSTTRGSCLPPQKTSLMM
TLCLGSIYEDLKMYQTEFQAINAALQNHNHQQIILDKGMLVAIDELMQSLNHNGETL
RQKPPVGEADPYRVKMKLCILLHAFSTRVVTINRVMGYLSSAGGGGSGGGGSGGGGS
GGGGSGGGGSMWELEKDVYVVEVDWTPDAPGETVNLTCDTPEEDDITWTSDQRHG
VIGSGKTLTITVKEFLDAGQYTCHKGGETLSHSHLLLHKKENGIWSTEILKNFKNKTF
LKCEAPNYSGRFTCSWLVQRNMDLKFNIKSSSSSPDSRAVTCGMASLSAEKVTLDQR
DYEKYSVSCQEDVTCPTAEETLPIELALEARQQNKYENYSTSFFIRDIIKPDPPKNLQM
KPLKNSQVEVSWEYPDSWSTPHSYFSLKFFVRIQRKKEKMKETEEGCNQKGAFLVEK
TSTEVQCKGGNVCVQAQDRYYNSSCSKWACVPCRVRSGGGGSGGGGSQVQLVQSG
AEVKKPGSSVKVSCKASGFTFTTYYISWVRQAPGQGLEYLGYINMGSGGTNYNEKF
KGRVTITADKSTSTAYMELSSLRSEDTAVYYCAIIGYFDYWGQGTMVTVSSGSSGGSG
GSGGSGDVVMTQSPLSLPVTLGQPASISCRSSQSLLDSDGGTYLYWFQQRPGQSPRRL
IYLVSTLGSGVPDRFSGSGSGTDFTLKISRVEAEDVGVYYCMQLTHWPYTFGQGTKLE
IK (SEQ ID No: 61);
PDL1-ScFv-FI tandem single cytokine fusion protein LIC442: the amino acid sequence of chain 1 is SEQ ID No: 62 (SEQ ID No: 17 + SEQ ID No: 3 + SEQ ID No: 5 + SEQ ID No: 1 + SEQ ID No: 75 + SEQ ID No: 2), and the amino acid sequence of chain 2 is SEQ ID No: 63 (SEQ ID No: 17 + SEQ ID No: 4): 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSGSSGGSGGSGGSGDIQMTQSPSSLSASVGDRVTITCRASQDVSTAV
AWYQQKPGKAPKLLIYSASFLYSGVPSRFSGSGSGTDFTLTISSLQPEDFATYYCQQYL
YHPATFGQGTKVEIKEPKSCDKTHTCPPCPAPELLGGPSVFLFPPKPKDTLMISRTPEVT
CVVVDVSHEDPEVKFNWYVDGVEVHNAKTKPREEQYASTYRVVSVLTVLHQDWLN
GKEYKCKVSNKALPAPIEKTISKAKGQPREPQVYTLPPCRDELTKNQVSLWCLVKGFY
PSDIAVEWESNGQPENNYKTTPPVLDSDGSFFLYSKLTVDKSRWQQGNVFSCSVMHE
 
ALHNHYTQKSLSLSPGKGSSGGSGGSGGSGDIQMTQSPSSLSASVGDRVTITCRASQD
VSTAVAWYQQKPGKAPKLLIYSASFLYSGVPSRFSGSGSGTDFTLTISSLQPEDFATYYC
QQYLYHPATFGQGTKVEIKGSSGGSGGSGGSGRVIPVSGPARCLSQSRNLLKTTDDMV
KTAREKLKHYSCTAEDIDHEDITRDQTSTLKTCLPLELHKNESCLATRETSSTTRGSCL
PPQKTSLMMTLCLGSIYEDLKMYQTEFQAINAALQNHNHQQIILDKGMLVAIDELMQ
SLNHNGETLRQKPPVGEADPYRVKMKLCILLHAFSTRVVTINRVMGYLSSAGGGGSG
GGGSGGGGSGGGGSGGGGSMWELEKDVYVVEVDWTPDAPGETVNLTCDTPEEDDI
TWTSDQRHGVIGSGKTLTITVKEFLDAGQYTCHKGGETLSHSHLLLHKKENGIWSTEI
LKNFKNKTFLKCEAPNYSGRFTCSWLVQRNMDLKFNIKSSSSSPDSRAVTCGMASLS
AEKVTLDQRDYEKYSVSCQEDVTCPTAEETLPIELALEARQQNKYENYSTSFFIRDIIK
PDPPKNLQMKPLKNSQVEVSWEYPDSWSTPHSYFSLKFFVRIQRKKEKMKETEEGCN
QKGAFLVEKTSTEVQCKGGNVCVQAQDRYYNSSCSKWACVPCRVRS (SEQ ID No:
62);
 
EVQLVESGGGLVQPGGSLRLSCAASGFTFSDSWIHWVRQAPGKGLEWVA
WISPYGGSTYYADSVKGRFTISADTSKNTAYLQMNSLRAEDTAVYYCARRHWPGGFD
YWGQGTLVTVSSGSSGGSGGSGGSGDIQMTQSPSSLSASVGDRVTITCRASQDVSTAV
AWYQQKPGKAPKLLIYSASFLYSGVPSRFSGSGSGTDFTLTISSLQPEDFATYYCQQYL
YHPATFGQGTKVEIKEPKSCDKTHTCPPCPAPELLGGPSVFLFPPKPKDTLMISRTPEVT
CVVVDVSHEDPEVKFNWYVDGVEVHNAKTKPREEQYASTYRVVSVLTVLHQDWLN
GKEYKCKVSNKALPAPIEKTISKAKGQPREPQVCTLPPSRDELTKNQVSLSCAVKGFY
PSDIAVEWESNGQPENNYKTTPPVLDSDGSFFLVSKLTVDKSRWQQGNVFSCSVMHE
ALHNHYTQKSLSLSPGK (SEQ ID No: 63);
PDL1-ScFv-PD1-ScFv-FI tandem single cytokine fusion protein LIC443: the amino acid sequence of chain 1 is SEQ ID No: 62, and the amino acid sequence of chain 2 is SEQ ID No: 64 (SEQ ID No: 18 + SEQ ID No: 4): 
QVQLVQSGAEVKKPGSSVKVSCKASGFTFTTYYISWVRQAPGQGLEYLGY
INMGSGGTNYNEKFKGRVTITADKSTSTAYMELSSLRSEDTAVYYCAIIGYFDYWGQG
TMVTVSSGSSGGSGGSGGSGDVVMTQSPLSLPVTLGQPASISCRSSQSLLDSDGGTYL
YWFQQRPGQSPRRLIYLVSTLGSGVPDRFSGSGSGTDFTLKISRVEAEDVGVYYCMQL
THWPYTFGQGTKLEIKEPKSCDKTHTCPPCPAPELLGGPSVFLFPPKPKDTLMISRTPE
 
VTCVVVDVSHEDPEVKFNWYVDGVEVHNAKTKPREEQYASTYRVVSVLTVLHQDW
LNGKEYKCKVSNKALPAPIEKTISKAKGQPREPQVCTLPPSRDELTKNQVSLSCAVKG
FYPSDIAVEWESNGQPENNYKTTPPVLDSDGSFFLVSKLTVDKSRWQQGNVFSCSVM
HEALHNHYTQKSLSLSPGK (SEQ ID No: 64);
F-double-cleavable-I fusion protein LIC403-1: the amino acid sequence of chain 1 is SEQ ID No: 65 (the cleavable linker contains 16 less amino acids than that of LIC403), and the amino acid sequence of chain 2 is SEQ ID No: 66 (the cleavable linker contains 16 less amino acids than that of LIC403): 
EPKSCDKTHTCPPCPAPELLGGPSVFLFPPKPKDTLMISRTPEVTCVVVDVS
HEDPEVKFNWYVDGVEVHNAKTKPREEQYASTYRVVSVLTVLHQDWLNGKEYKCK
VSNKALPAPIEKTISKAKGQPREPQVYTLPPCRDELTKNQVSLWCLVKGFYPSDIAVE
WESNGQPENNYKTTPPVLDSDGSFFLYSKLTVDKSRWQQGNVFSCSVMHEALHNHY
TQKSLSLSPGKGSSGGLSGRSDNHGSGGSRVIPVSGPARCLSQSRNLLKTTDDMVKTA
REKLKHYSCTAEDIDHEDITRDQTSTLKTCLPLELHKNESCLATRETSSTTRGSCLPPQ
KTSLMMTLCLGSIYEDLKMYQTEFQAINAALQNHNHQQIILDKGMLVAIDELMQSLN
HNGETLRQKPPVGEADPYRVKMKLCILLHAFSTRVVTINRVMGYLSSA (SEQ ID No:
65);
 
EPKSCDKTHTCPPCPAPELLGGPSVFLFPPKPKDTLMISRTPEVTCVVVDVS
HEDPEVKFNWYVDGVEVHNAKTKPREEQYASTYRVVSVLTVLHQDWLNGKEYKCK
VSNKALPAPIEKTISKAKGQPREPQVCTLPPSRDELTKNQVSLSCAVKGFYPSDIAVEW
ESNGQPENNYKTTPPVLDSDGSFFLVSKLTVDKSRWQQGNVFSCSVMHEALHNHYT
QKSLSLSPGKGSSGGLSGRSDNHGSGGSMWELEKDVYVVEVDWTPDAPGETVNLTC
DTPEEDDITWTSDQRHGVIGSGKTLTITVKEFLDAGQYTCHKGGETLSHSHLLLHKKE
NGIWSTEILKNFKNKTFLKCEAPNYSGRFTCSWLVQRNMDLKFNIKSSSSSPDSRAVT
CGMASLSAEKVTLDQRDYEKYSVSCQEDVTCPTAEETLPIELALEARQQNKYENYST
SFFIRDIIKPDPPKNLQMKPLKNSQVEVSWEYPDSWSTPHSYFSLKFFVRIQRKKEKM
KETEEGCNQKGAFLVEKTSTEVQCKGGNVCVQAQDRYYNSSCSKWACVPCRVRS
(SEQ ID No: 66);
PD1(Fab)-double-cleavable-IF-Y fusion protein LIC425-1: the amino acid sequence of chain 1 is SEQ ID No: 67 (SEQ ID No: 13 + SEQ ID No: 8 + SEQ ID No: 21), and the amino acid sequence of chain 2 is SEQ ID No: 68 (SEQ ID No: 20 + SEQ ID No: 8 + SEQ ID No: 22): 
DVVMTQSPLSLPVTLGQPASISCRSSQSLLDSDGGTYLYWFQQRPGQSPRR
LIYLVSTLGSGVPDRFSGSGSGTDFTLKISRVEAEDVGVYYCMQLTHWPYTFGQGTKL
EIKRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGECGSSGGS
GGSGGSGQLLGFLTAGSSGGSGGSGGSGRVIPVSGPARCLSQSRNLLKTTDDMVKTAR
EKLKHYSCTAEDIDHEDITRDQTSTLKTCLPLELHKNESCLATRETSSTTRGSCLPPQK
TSLMMTLCLGSIYEDLKMYQTEFQAINAALQNHNHQQIILDKGMLVAIDELMQSLNH
NGETLRQKPPVGEADPYRVKMKLCILLHAFSTRVVTINRVMGYLSSAGGGGSGGGGS
EPKSCDKTHTCPPCPAPELLGGPSVFLFPPKPKDTLMISRTPEVTCVVVDVSHEDPEVK
FNWYVDGVEVHNAKTKPREEQYASTYRVVSVLTVLHQDWLNGKEYKCKVSNKALP
APIEKTISKAKGQPREPQVYTLPPCRDELTKNQVSLWCLVKGFYPSDIAVEWESNGQP
ENNYKTTPPVLDSDGSFFLYSKLTVDKSRWQQGNVFSCSVMHEALHNHYTQKSLSLS
PGK (SEQ ID No: 67);
 
QVQLVQSGAEVKKPGSSVKVSCKASGFTFTTYYISWVRQAPGQGLEYLGY
INMGSGGTNYNEKFKGRVTITADKSTSTAYMELSSLRSEDTAVYYCAIIGYFDYWGQG
TMVTVSSASTKGPSVFPLAPCSRSTSESTAALGCLVKDYFPEPVTVSWNSGALTSGVH
TFPAVLQSSGLYSLSSVVTVPSSSLGTKTYTCNVDHKPSNTKVDKRVGSSGGSGGSGG
SGQLLGFLTAGSSGGSGGSGGSGMWELEKDVYVVEVDWTPDAPGETVNLTCDTPEE
DDITWTSDQRHGVIGSGKTLTITVKEFLDAGQYTCHKGGETLSHSHLLLHKKENGIW
STEILKNFKNKTFLKCEAPNYSGRFTCSWLVQRNMDLKFNIKSSSSSPDSRAVTCGMA
SLSAEKVTLDQRDYEKYSVSCQEDVTCPTAEETLPIELALEARQQNKYENYSTSFFIR
DIIKPDPPKNLQMKPLKNSQVEVSWEYPDSWSTPHSYFSLKFFVRIQRKKEKMKETEE
GCNQKGAFLVEKTSTEVQCKGGNVCVQAQDRYYNSSCSKWACVPCRVRSGGGGSG
GGGSEPKSCDKTHTCPPCPAPELLGGPSVFLFPPKPKDTLMISRTPEVTCVVVDVSHED
PEVKFNWYVDGVEVHNAKTKPREEQYASTYRVVSVLTVLHQDWLNGKEYKCKVSN
KALPAPIEKTISKAKGQPREPQVCTLPPSRDELTKNQVSLSCAVKGFYPSDIAVEWESN
GQPENNYKTTPPVLDSDGSFFLVSKLTVDKSRWQQGNVFSCSVMHEALHNHYTQKS
LSLSPGK (SEQ ID No: 68);
PI tandem single-molecule fusion protein LIC444: the amino acid sequence of chain 1 is SEQ ID No: 69 (SEQ ID No: 11 + SEQ ID No: 5 + SEQ ID No: 2 + SEQ ID No: 75 + SEQ ID No: 1), the amino acid sequence of chain 2 is SEQ ID No: 12, and the amino acid sequence of chain 3 is SEQ ID No: 9: 
EPKSCDKTHTCPPCPAPELLGGPSVFLFPPKPKDTLMISRTPEVTCVVVDVS
HEDPEVKFNWYVDGVEVHNAKTKPREEQYASTYRVVSVLTVLHQDWLNGKEYKCK
VSNKALPAPIEKTISKAKGQPREPQVYTLPPCRDELTKNQVSLWCLVKGFYPSDIAVE
WESNGQPENNYKTTPPVLDSDGSFFLYSKLTVDKSRWQQGNVFSCSVMHEALHNHY
TQKSLSLSPGKGSSGGSGGSGGSGMWELEKDVYVVEVDWTPDAPGETVNLTCDTPE
EDDITWTSDQRHGVIGSGKTLTITVKEFLDAGQYTCHKGGETLSHSHLLLHKKENGI
WSTEILKNFKNKTFLKCEAPNYSGRFTCSWLVQRNMDLKFNIKSSSSSPDSRAVTCGM
ASLSAEKVTLDQRDYEKYSVSCQEDVTCPTAEETLPIELALEARQQNKYENYSTSFFI
RDIIKPDPPKNLQMKPLKNSQVEVSWEYPDSWSTPHSYFSLKFFVRIQRKKEKMKETE
EGCNQKGAFLVEKTSTEVQCKGGNVCVQAQDRYYNSSCSKWACVPCRVRSGGGGS
GGGGSGGGGSGGGGSGGGGSRVIPVSGPARCLSQSRNLLKTTDDMVKTAREKLKHY
SCTAEDIDHEDITRDQTSTLKTCLPLELHKNESCLATRETSSTTRGSCLPPQKTSLMMT
LCLGSIYEDLKMYQTEFQAINAALQNHNHQQIILDKGMLVAIDELMQSLNHNGETLR
QKPPVGEADPYRVKMKLCILLHAFSTRVVTINRVMGYLSSA (SEQ ID No: 69);
Amino acid sequence of human IL-12-P35 subunit, SEQ ID No: 70: 
RNLPVATPDPGMFPCLHHSQNLLRAVSNMLQKARQTLEFYPCTSEEIDHED
ITKDKTSTVEACLPLELTKNESCLNSRETSFITNGSCLASRKTSFMMALCLSSIYEDLK
MYQVEFKTMNAKLLMDPKRQIFLDQNMLAVIDELMQALNFNSETVPQKSSLEEPDF
YKTKIKLCILLHAFRIRAVTIDRVMSYLNAS;
Amino acid sequence of human IL-12-P40 subunit, SEQ ID No: 71: 
IWELKKDVYVVELDWYPDAPGEMVVLTCDTPEEDGITWTLDQSSEVLGS
GKTLTIQVKEFGDAGQYTCHKGGEVLSHSLLLLHKKEDGIWSTDILKDQKEPKNKTF
LRCEAKNYSGRFTCWWLTTISTDLTFSVKSSRGSSDPQGVTCGAATLSAERVRGDNKE
YEYSVECQEDSACPAAEESLPIEVMVDAVHKLKYENYTSSFFIRDIIKPDPPKNLQLKP
LKNSRQVEVSWEYPDTWSTPHSYFSLTFCVQVQGKSKREKKDRVFTDKTSATVICRK
NASISVRAQDRYYSSSWSEWASVPCS;
Amino acid sequence of heavy chain of human EGFR monoclonal antibody, SEQ ID No: 72: 
QVQLKQSGPGLVQPSQSLSITCTVSGFSLTNYGVHWVRQSPGKGLEWLGVI
WSGGNTDYNTPFTSRLSINKDNSKSQVFFKMNSLQSNDTAIYYCARALTYYDYEFAY
WGQGTLVTVSAASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGK;
Amino acid sequence of light chain of human EGFR monoclonal antibody, SEQ ID No: 73: 
DILLTQSPVILSVSPGERVSFSCRASQSIGTNIHWYQQRTNGSPRLLIKYASES
ISGIPSRFSGSGSGTDFTLSINSVESEDIADYYCQQNNNWPTTFGAGTKLELKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC;
E-double-cleavable-I tandem fusion protein LIC445: the amino acid sequence of chain 1 is SEQ ID No: 74 (SEQ ID No: 72 + SEQ ID No: 8 + SEQ ID No: 70 + SEQ ID No: 75 + SEQ ID No: 71), and the amino acid sequence of chain 2 is SEQ ID No: 73: 
GWSLILLFLVAVATRVHSQVQLKQSGPGLVQPSQSLSITCTVSGFSLTNYGV
HWVRQSPGKGLEWLGVIWSGGNTDYNTPFTSRLSINKDNSKSQVFFKMNSLQSNDT
AIYYCARALTYYDYEFAYWGQGTLVTVSAASTKGPSVFPLAPSSKSTSGGTAALGCLV
KDYFPEPVTVSWNSGALTSGVHTFPAVLQSSGLYSLSSVVTVPSSSLGTQTYICNVNH
KPSNTKVDKKVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWLNGKE
YKCKVSNKALPAPIEKTISKAKGQPREPQVYTLPPSREEMTKNQVSLTCLVKGFYPSDI
AVEWESNGQPENNYKTTPPVLDSDGSFFLYSKLTVDKSRWQQGNVFSCSVMHEALH
NHYTQKSLSLSPGKGSSGGSGGSGGSGQLLGFLTAGSSGGSGGSGGSGRNLPVATPDP
GMFPCLHHSQNLLRAVSNMLQKARQTLEFYPCTSEEIDHEDITKDKTSTVEACLPLEL
TKNESCLNSRETSFITNGSCLASRKTSFMMALCLSSIYEDLKMYQVEFKTMNAKLLM
 
DPKRQIFLDQNMLAVIDELMQALNFNSETVPQKSSLEEPDFYKTKIKLCILLHAFRIRA
VTIDRVMSYLNASGGGGSGGGGSGGGGSGGGGSGGGGSIWELKKDVYVVELDWYP
DAPGEMVVLTCDTPEEDGITWTLDQSSEVLGSGKTLTIQVKEFGDAGQYTCHKGGEV
LSHSLLLLHKKEDGIWSTDILKDQKEPKNKTFLRCEAKNYSGRFTCWWLTTISTDLTF
SVKSSRGSSDPQGVTCGAATLSAERVRGDNKEYEYSVECQEDSACPAAEESLPIEVMV
DAVHKLKYENYTSSFFIRDIIKPDPPKNLQLKPLKNSRQVEVSWEYPDTWSTPHSYFSL
TFCVQVQGKSKREKKDRVFTDKTSATVICRKNASISVRAQDRYYSSSWSEWASVPCS
(SEQ ID No: 74);
Linker peptide between P35 and P40, SEQ ID No: 75:
   GGGGSGGGGSGGGGSGGGGSGGGGS (SEQ ID No: 75);
Mouse P35-linker peptide-P40, SEQ ID No: 76 (SEQ ID No: 1 + SEQ ID No: 75 + SEQ ID No: 2): 
RVIPVSGPARCLSQSRNLLKTTDDMVKTAREKLKHYSCTAEDIDHEDITRD
QTSTLKTCLPLELHKNESCLATRETSSTTRGSCLPPQKTSLMMTLCLGSIYEDLKMYQ
TEFQAINAALQNHNHQQIILDKGMLVAIDELMQSLNHNGETLRQKPPVGEADPYRVK
MKLCILLHAFSTRVVTINRVMGYLSSAGGGGSGGGGSGGGGSGGGGSGGGGSMWEL
EKDVYVVEVDWTPDAPGETVNLTCDTPEEDDITWTSDQRHGVIGSGKTLTITVKEFL
DAGQYTCHKGGETLSHSHLLLHKKENGIWSTEILKNFKNKTFLKCEAPNYSGRFTCS
WLVQRNMDLKFNIKSSSSSPDSRAVTCGMASLSAEKVTLDQRDYEKYSVSCQEDVTC
PTAEETLPIELALEARQQNKYENYSTSFFIRDIIKPDPPKNLQMKPLKNSQVEVSWEYP
DSWSTPHSYFSLKFFVRIQRKKEKMKETEEGCNQKGAFLVEKTSTEVQCKGGNVCV
QAQDRYYNSSCSKWACVPCRVRS;
Human P35-linker peptide-P40, SEQ ID No: 77 (SEQ ID No: 70 + SEQ ID No: 75 + SEQ ID No: 71): 
RNLPVATPDPGMFPCLHHSQNLLRAVSNMLQKARQTLEFYPCTSEEIDHED
ITKDKTSTVEACLPLELTKNESCLNSRETSFITNGSCLASRKTSFMMALCLSSIYEDLK
MYQVEFKTMNAKLLMDPKRQIFLDQNMLAVIDELMQALNFNSETVPQKSSLEEPDF
YKTKIKLCILLHAFRIRAVTIDRVMSYLNASGGGGSGGGGSGGGGSGGGGSGGGGSI
WELKKDVYVVELDWYPDAPGEMVVLTCDTPEEDGITWTLDQSSEVLGSGKTLTIQV
KEFGDAGQYTCHKGGEVLSHSLLLLHKKEDGIWSTDILKDQKEPKNKTFLRCEAKNY
 
SGRFTCWWLTTISTDLTFSVKSSRGSSDPQGVTCGAATLSAERVRGDNKEYEYSVECQ
EDSACPAAEESLPIEVMVDAVHKLKYENYTSSFFIRDIIKPDPPKNLQLKPLKNSRQVE
VSWEYPDTWSTPHSYFSLTFCVQVQGKSKREKKDRVFTDKTSATVICRKNASISVRAQ
DRYYSSSWSEWASVPCS;
Amino acid sequence of cleavable cathepsin substrate linker, SEQ ID No: 78:
   GSSGGSGGSGGSGFFYGSSGGSGGSGGSG;
Amino acid sequence of cleavable protease substrate linker, SEQ ID No: 79:
   GSSGGSGGSGGSGRQARAVGGSSGGSGGSGGSG;
Amino acid sequence of cleavable legumain substrate linker, SEQ ID No: 80:
   GSSGGSGGSGGSGAANLGSSGGSGGSGGSG;
F-double-cleavable-I fusion protein LIC403-2: the amino acid sequence of chain 1 is SEQ ID No: 81 (the cleavable linker contains 14 more amino acids than that of LIC403), and the amino acid sequence of chain 2 is SEQ ID No: 82 (the cleavable linker contains 14 more amino acids than that of LIC403): 
EPKSCDKTHTCPPCPAPELLGGPSVFLFPPKPKDTLMISRTPEVTCVVVDVS
HEDPEVKFNWYVDGVEVHNAKTKPREEQYASTYRVVSVLTVLHQDWLNGKEYKCK
VSNKALPAPIEKTISKAKGQPREPQVYTLPPCRDELTKNQVSLWCLVKGFYPSDIAVE
WESNGQPENNYKTTPPVLDSDGSFFLYSKLTVDKSRWQQGNVFSCSVMHEALHNHY
TQKSLSLSPGKGSSGGSGGSGGSGSSGGSGGLSGRSDNHGSSGGSGGSGGSGSSGGSG
GRVIPVSGPARCLSQSRNLLKTTDDMVKTAREKLKHYSCTAEDIDHEDITRDQTSTLK
TCLPLELHKNESCLATRETSSTTRGSCLPPQKTSLMMTLCLGSIYEDLKMYQTEFQAIN
AALQNHNHQQIILDKGMLVAIDELMQSLNHNGETLRQKPPVGEADPYRVKMKLCILL
HAFSTRVVTINRVMGYLSSA (SEQ ID No: 81);
 
EPKSCDKTHTCPPCPAPELLGGPSVFLFPPKPKDTLMISRTPEVTCVVVDVS
HEDPEVKFNWYVDGVEVHNAKTKPREEQYASTYRVVSVLTVLHQDWLNGKEYKCK
VSNKALPAPIEKTISKAKGQPREPQVCTLPPSRDELTKNQVSLSCAVKGFYPSDIAVEW
ESNGQPENNYKTTPPVLDSDGSFFLVSKLTVDKSRWQQGNVFSCSVMHEALHNHYT
QKSLSLSPGKGSSGGSGGSGGSGSSGGSGGLSGRSDNHGSSGGSGGSGGSGSSGGSG
GMWELEKDVYVVEVDWTPDAPGETVNLTCDTPEEDDITWTSDQRHGVIGSGKTLTIT
VKEFLDAGQYTCHKGGETLSHSHLLLHKKENGIWSTEILKNFKNKTFLKCEAPNYSG
 
RFTCSWLVQRNMDLKFNIKSSSSSPDSRAVTCGMASLSAEKVTLDQRDYEKYSVSCQ
EDVTCPTAEETLPIELALEARQQNKYENYSTSFFIRDIIKPDPPKNLQMKPLKNSQVEV
SWEYPDSWSTPHSYFSLKFFVRIQRKKEKMKETEEGCNQKGAFLVEKTSTEVQCKGG
NVCVQAQDRYYNSSCSKWACVPCRVRS (SEQ ID No: 82);
Amino acid sequence of cleavable UPA substrate linker 3, SEQ ID No: 83:
   GSSGGLSGRSDNHGSGGS;
Amino acid sequence of cleavable UPA substrate linker 3, SEQ ID No: 84:
   GSSGGSGGSGGSGSSGGSGGLSGRSDNHGSSGGSGGSGGSGSSGGSGG;
Amino acid sequence of non-cleavable linker 2 at the N-terminus of IL12 subunit, SEQ ID No: 85:
   GSSGGSGGSGGSGGSSGGSGGSGGSGGSSGGSGG;
FI fusion protein LIC402-1: the amino acid sequence of chain 1 is SEQ ID No: 86 (the non-cleavable linker contains 21 more amino acids than that of LIC402), and the amino acid sequence of chain 2 is SEQ ID No: 87 (the non-cleavable linker contains 21 more amino acids than that of LIC402): 
EPKSCDKTHTCPPCPAPELLGGPSVFLFPPKPKDTLMISRTPEVTCVVVDVS
HEDPEVKFNWYVDGVEVHNAKTKPREEQYASTYRVVSVLTVLHQDWLNGKEYKCK
VSNKALPAPIEKTISKAKGQPREPQVYTLPPCRDELTKNQVSLWCLVKGFYPSDIAVE
WESNGQPENNYKTTPPVLDSDGSFFLYSKLTVDKSRWQQGNVFSCSVMHEALHNHY
TQKSLSLSPGKGSSGGSGGSGGSGGSSGGSGGSGGSGGSSGGSGGRVIPVSGPARCLS
QSRNLLKTTDDMVKTAREKLKHYSCTAEDIDHEDITRDQTSTLKTCLPLELHKNESCL
ATRETSSTTRGSCLPPQKTSLMMTLCLGSIYEDLKMYQTEFQAINAALQNHNHQQIIL
DKGMLVAIDELMQSLNHNGETLRQKPPVGEADPYRVKMKLCILLHAFSTRVVTINRV
MGYLSSA(SEQ ID No: 86);
 
EPKSCDKTHTCPPCPAPELLGGPSVFLFPPKPKDTLMISRTPEVTCVVVDVS
HEDPEVKFNWYVDGVEVHNAKTKPREEQYASTYRVVSVLTVLHQDWLNGKEYKCK
VSNKALPAPIEKTISKAKGQPREPQVCTLPPSRDELTKNQVSLSCAVKGFYPSDIAVEW
ESNGQPENNYKTTPPVLDSDGSFFLVSKLTVDKSRWQQGNVFSCSVMHEALHNHYT
QKSLSLSPGKGSSGGSGGSGGSGGSSGGSGGSGGSGGSSGGSGGMWELEKDVYVVE
VDWTPDAPGETVNLTCDTPEEDDITWTSDQRHGVIGSGKTLTITVKEFLDAGQYTCH
 
KGGETLSHSHLLLHKKENGIWSTEILKNFKNKTFLKCEAPNYSGRFTCSWLVQRNMD
LKFNIKSSSSSPDSRAVTCGMASLSAEKVTLDQRDYEKYSVSCQEDVTCPTAEETLPIE
LALEARQQNKYENYSTSFFIRDIIKPDPPKNLQMKPLKNSQVEVSWEYPDSWSTPHSY
FSLKFFVRIQRKKEKMKETEEGCNQKGAFLVEKTSTEVQCKGGNVCVQAQDRYYNS
SCSKWACVPCRVRS(SEQ ID No: 87);
Amino acid sequence of the signal peptide of human EGFR monoclonal antibody, SEQ ID No: 88:
   MGWSLILLFLVAVATRVHS_{∘}

In the description of the present specification, reference to the terms such as "an embodiment," "some embodiments," "an example," "a specific example," or "some examples" means that a particular feature, structure, material, or characteristic described in connection with the embodiment or example is included in at least one embodiment or example of the present disclosure. The illustrative expressions of the above terms as used in this specification do not necessarily refer to the same embodiment or example. Furthermore, the particular features, structures, materials, or characteristics described may be combined in any suitable manner in one or more embodiments or examples. In addition, unless otherwise conflicting, those skilled in the art will appreciate that the different embodiments or examples described in this specification, as well as the features of different embodiments or examples, may be combined or integrated.

Although the embodiments of the present disclosure have been shown and described above, it will be understood by those of ordinary skill in the art that the above embodiments are exemplary only and should not be construed as limiting the scope of the invention. Alterations, substitutions, and variations can be made to the described embodiments without departing from the scope of the present disclosure.

## Claims

1. A fusion protein, comprising:
a first structural unit selected from a first Fc fragment, or a first antibody or antigen-binding fragment thereof, wherein the first antibody or antigen-binding fragment thereof has a binding activity to a tumor antigen or an immune checkpoint;
a second structural unit selected from a cleavable linker or a non-cleavable linker, wherein the cleavable linker is capable of being cleaved by a protease expressed in a tumor tissue; and
a third structural unit comprising an IL-12 cytokine or functional fragment thereof,
wherein the first structural unit is linked to one or more third structural units, the third structural unit being linked to the C-terminus of the first structural unit via the second structural unit.

2. The fusion protein according to claim 1, saitisfying one or more of the following conditions:
1) when the first structural unit is linked to one third structural unit, the second structural unit is selected from a cleavable linker;
2) when the first structural unit is linked to a plurality of third structural units, the second structural units linked to the plurality of third structural units contain at least one cleavable linker, and preferably, the second structural units linked to the plurality of third structural units are all the cleavable linkers;
3) the fusion protein comprises no antibody of IL-12 cytokine or antigen-binding fragment thereof;
4) the first antibody or antigen-binding fragment thereof is selected from a first full-length antibody, a first Fab fragment, a first ScFv fragment, or a first VHH fragment;
5) the tumor antigen or the immune checkpoint is selected from at least one of Her2, EGFR, VEGF, VEGFR, Claudin. 18.2, B7-H3, Nectin-4, MSLN, Trop2, Siglec-9, Siglec-15, PD-L1, PD-1, TIGIT, LAG3, TIM-3, CTLA-4, BTLA, or VISTA;
6) the IL-12 cytokine or functional fragment thereof comprises at least one of a p35 fragment of IL-12 or functional fragment thereof, and a p40 fragment of IL-12 or functional fragment thereof;
7) the second structural unit is a cleavable linker;
8) the second structural unit is a non-cleavable linker;
9) the fusion protein comprises two monomers, wherein two second structural units are a cleavable linker and a non-cleavable linker, respectively;
10) the protease expressed in the tumor tissue comprises a matrix metalloproteinase, a serine protease, or an asparagine endopeptidase;
11) the cleavable linker comprises a protease substrate sequence and optionally a flexible peptide segment;
12) the flexible peptide segment comprises at least one of (GS)n, (GGS)n, (GGSG)n, (GSSG)n, (GGGS)n, (GGGGS)n, and (GSGGS)n, where n is any integer between 1 and 10;
13) the flexible peptide segment has a length of 1 to 60 amino acids, and preferably 5 to 30 amino acids, 5 to 25 amino acids, or 5 to 20 amino acids;
14) the non-cleavable linker comprises at least one of (GS)n, (GGS)n, (GGSG)n, (GSSG)n, (GGGS)n, (GGGGS)n, and (GSGGS)n, where n is any integer between 1 and 10; and
15) the non-cleavable linker has a length of 1 to 60 amino acids, and preferably 5 to 50 amino acids, 5 to 40 amino acids, 5 to 30 amino acids, or 10 to 20 amino acids.

3. The fusion protein according to claim 2, satisfying one or more of the following conditions:
1) the IL-12 cytokine or functional fragment thereof has an amino acid sequence as set forth in any one of SEQ ID NO: 1, SEQ ID NO: 2, SEQ ID NO: 70, and SEQ ID NO: 71;
2) the IL-12 cytokine or functional fragment thereof comprises two subunit fragments: a P35 fragment of IL-12 or functional fragment thereof, and a P40 fragment of IL-12 orfunctional fragment thereof, wherein the third structural unit further comprises a non-cleavable linker peptide, the two subunit fragments being linked via the non-cleavable linker peptide;
3) the protease substrate sequence is selected from QLLGFLTA, LSGRSDNH, GFFY, RQARAVGG, or AANL;
4) the cleavable linker is a flexible peptide segment-protease substrate sequence, or a flexible peptide segment-protease substrate sequence-flexible peptide segment;
5) the cleavable linker has a length of 15 to 60 amino acids, and preferably 15 to 50 amino acids, 15 to 45 amino acids, or 15 to 40 amino acids; and
6) the non-cleavable linker has an amino acid sequence as set forth in (GGGGS)n, where n is 3, 4, 5, or 6.

4. The fusion protein according to claim 3, saitisfying one or more of the following conditions:
1) the IL-12 cytokine or functional fragment thereof comprises two subunit fragments: a p35 fragment of IL-12 or a functional fragment thereof, and a p40 fragment of IL-12 or a functional fragment thereof, wherein the C-terminus of one of the two subunit fragments is linked to the N-terminus of the linker peptide, and the C-terminus of the linker peptide is linked to the N-terminus of the other of two subunit fragments;
2) the linker peptide comprises at least one of (GS)n, (GGS)n, (GGSG)n, (GSSG)n, (GGGS)n, (GGGGS)n, and (GSGGS)n, where n is any integer between 1 and 10;
3) the linker peptide has a length of 1 to 60 amino acids, and preferably 10 to 40 amino acids, or 15 to 40 amino acids;
4) the cleavable linker has an amino acid sequence as set forth in SEQ ID NO: 7, SEQ ID NO: 8, SEQ ID NO: 78, SEQ ID NO: 79, SEQ ID NO: 80, SEQ ID NO: 83, or SEQ ID NO: 84; and
5) the non-cleavable linker has an amino acid sequence as set forth in SEQ ID NO: 5 or SEQ ID NO: 6.

5. The fusion protein according to claim 3, satisfying one or more of the following conditions:
1) the linker peptide has an amino acid sequence as set forth in (GGGGS)n, where n is 3, 4, 5, or 6; and
2) the third structural unit has an amino acid sequence as set forth in SEQ ID NO: 1, SEQ ID NO: 2, SEQ ID NO: 70, SEQ ID NO: 71, SEQ ID NO: 76, or SEQ ID NO: 77.

6. The fusion protein according to claim 2, satisfying one or more of the following conditions:
1) the first structural unit is a first ScFv fragment, wherein: the C-terminus of the first structural unit is linked to the N-terminus of the second structural unit, and the C-terminus of the second structural unit is linked to the N-terminus of the third structural unit; or the C-terminus of the third structural unit is linked to the N-terminus of the second structural unit, and the C-terminus of the second structural unit is linked to the N-terminus of the first structural unit;
2) the first structural unit is a first Fc fragment or a first VHH fragment, wherein the first Fc fragment or the first VHH fragment is linked to one or two third structural units, each of the one or two third structural units being linked to the first Fc fragment or the first VHH fragment via the second structural unit, wherein: the C-terminus of the first structural unit is linked to the N-terminus of the second structural unit, and the C-terminus of the second structural unit is linked to the N-terminus of the third structural unit; or the C-terminus of the third structural unit is linked to the N-terminus of the second structural unit, and the C-terminus of the second structural unit is linked to the N-terminus of the first structural unit;
3) when the first Fc fragment or the first VHH fragment is linked to one third structural unit, the IL-12 cytokine or functional fragment thereof in the one third structural unit comprises two subunit fragments: a P35 fragment of IL-12 or functional fragment thereof, and a P40 fragment of IL-12 or functional fragment thereof, and preferably the P35 fragment of IL-12 or functional fragment thereof, and the P40 fragment of IL-12 or functional fragment thereof;
4) when the first Fc fragment or the first VHH fragment is linked to two third structural units, each of the two second structural units is independently selected from a cleavable linker or a non-cleavable linker;
5) when the first Fc fragment or the first VHH fragment is linked to two third structural units, the IL-12 cytokines or functional fragments thereof in the two third structural units are the same or different; and
6) when the fusion protein consists of the first structural unit, the second structural unit, and the third structural unit, and when the first structural unit is the first Fc fragment, the IL-12 cytokine or functional fragment thereof in the third structural unit comprises two subunit fragments: a P35 fragment of IL-12 or functional fragment thereof, and a P40 fragment of IL-12 or functional fragment thereof, wherein the two subunit fragments are linked to two fragments of the first Fc fragment, respectively.

7. The fusion protein according to claim 2, satisfying one or more of the following conditions:
a) the first structural unit is a first full-length antibody linked to the one or more third structural units, wherein each of the one or more third structural units is linked to the first full-length antibody via the second structural unit; and
b) the first structural unit is a first Fab fragment linked to one or two third structural units, wherein each of the one or two third structural units is linked to the C-terminus of the first Fab fragment via the second structural unit.

8. The fusion protein according to claim 7, satisfying one or more of the following conditions:
a-1) the first full-length antibody is linked to one third structural unit, wherein the one third structural unit is linked to the C-terminus of a heavy chain in the first full-length antibody via the second structural unit;
a-2) the first full-length antibody is linked to a plurality of third structural units, wherein each of the plurality of third structural units is linked to the C-terminus of a heavy chain in the first full-length antibody via the second structural unit;
a-3) the first full-length antibody is linked to two third structural units, wherein the two third structural units are linked to the C-termini of two heavy chains in the first full-length antibody via the second structural units, respectively;
a-4) when the first full-length antibody is linked to one third structural unit, the IL-12 cytokine or functional fragment thereof in the one third structural unit comprises two subunit fragments: a P35 fragment of IL-12 or functional fragment thereof, and a P40 fragment of IL-12 or functional fragment thereof, and preferably, the P35 fragment of IL-12 or functional fragment thereof, and the P40 fragment of IL-12 or functional fragment thereof;
a-5) when the first full-length antibody is linked to a plurality of third structural units, wherein each of the plurality of second structural units is independently selected from a cleavable linker or a non-cleavable linker, and preferably, each of the plurality of second structural units is a cleavable linker;
a-6) when the first full-length antibody is linked to two third structural units, each of the two second structural units is independently selected from a cleavable linker or a non-cleavable linker;
a-7) when the first full-length antibody is linked to two third structural units, and when the two third structural units are linked to the C-termini of two heavy chains in the first full-length antibody via the second structural units, respectively, the IL-12 cytokines or functional fragments thereof in the two third structural units is the same or different, preferably, the IL-12 cytokines or functional fragments thereof in the two third structural units are a P35 fragment of IL-12 or a functional fragment thereof and a P40 fragment of IL-12 or a functional fragment thereof, respectively;
b-1) the first Fab fragment is linked to one third structural unit, wherein the one third structural unit is linked to the C-terminus of the CH1 fragment or the C-terminus of the CL fragment in the first Fab fragment via the second structural unit;
b-2) the first Fab fragment is linked to two third structural units, wherein the two third structural units are linked to the C-terminus of a CH1 fragment and the C-terminus of a CL fragment in the first Fab fragment via the second structural units, respectively;
b-3) when the first Fab fragment is linked to one third structural unit, the IL-12 cytokine or functional fragment thereof in the third structural unit comprises two subunit fragments: a P35 fragment of IL-12 or functional fragment thereof, and a P40 fragment of IL-12 orfunctional fragment thereof, preferably the P35 fragment of IL-12 or functional fragment thereof, and the P40 fragment of IL-12 or functional fragment thereof;
b-4) when the first Fab fragment is linked to two third structural units, each of the two second structural units is independently selected from a cleavable linker or a non-cleavable linker; and
b-5) when the first Fab fragment is linked to two third structural units, the IL-12 cytokines or functional fragments thereof in the two third structural units are the same or different, and preferably, the IL-12 cytokines or functional fragments thereof in the two third structural units are a P35 fragment of IL-12 or functional fragment thereof, and a P40 fragment of IL-12 or functional fragment thereof, respectively.

9. The fusion protein according to claim 7, comprising two monomers, when the first Fab fragment is linked to one third structural unit, and when the one third structural unit is linked to the C-terminus of a CH1 fragment in the first Fab fragment via the second structural unit,
wherein in the two monomers, the IL-12 cytokine or functional fragment thereof of the third structural unit is a P35 fragment of IL-12 or functional fragment thereof, and a P40 fragment of IL-12 or functional fragment thereof, wherein the P35 fragment of IL-12 or functional fragment thereof is linked to the P40 fragment of IL-12 or functional fragment thereof via a disulfide bond.

10. The fusion protein according to any one of claims 2 to 9, further comprising a fourth structural unit selected from a second Fc fragment, or a second antibody or antigen-binding fragment thereof, wherein the second antibody or antigen-binding fragment thereof has a binding activity to a tumor antigen or an immune checkpoint.

11. The fusion protein according to claim 10, further satisfying one or more of the following conditions:
1) the second antibody or antigen-binding fragment thereof is selected from a second full-length antibody, a second Fab fragment, a second ScFv fragment, or a second VHH fragment;
2) the C-terminus of the fourth structural unit is linked to the N-terminus of the first structural unit, or the C-terminus of the third structural unit is linked to the N-terminus of the fourth structural unit;
3) the first structural unit is the first Fc fragment, and the fourth structural unit is selected from a second Fab fragment, a second ScFv fragment, or a second VHH fragment; or
the first structural unit is selected from a first Fab fragment, a first ScFv fragment, or a first VHH fragment, and the fourth structural unit is the second Fc fragment,
wherein the third structural unit is located between the first structural unit and the fourth structural unit;
4) the fusion protein further comprises a fifth structural unit, wherein the fourth structural unit is linked to the first structural unit or the third structural unit via the fifth structural unit;
5) the fifth structural unit is selected from a cleavable linker or a non-cleavable linker;
6) when the third structural unit is located between the first structural unit and the fourth structural unit, the third structural unit is linked to the first structural unit via the second structural unit, and the third structural unit is linked to the fourth structural unit via the fifth structural unit;
7) when the first structural unit is the first Fc fragment or a first full-length antibody, the fourth structural unit is selected from a second Fab fragment, a second ScFv fragment, or a second VHH fragment, wherein the C-terminus of the fourth structural unit is linked to the N-terminus of the first structural unit, or wherein the C-terminus of the third structural unit is linked to the N-terminus of the fourth structural unit,
preferably, the C-terminus of the fourth structural unit is linked to the N-terminus of the fifth structural unit, and the C-terminus of the fifth structural unit is linked to the N-terminus of the first structural unit; or the C-terminus of the third structural unit is linked to the N-terminus of the fifth structural unit, and the C-terminus of the fifth structural unit is linked to the N-terminus of the fourth structural unit;
7) when the first structural unit is a first Fab fragment, a first ScFv fragment, or a first VHH fragment, the fourth structural unit is selected from the second Fc fragment, wherein the C-terminus of the third structural unit is linked to the N-terminus of the fourth structural unit; preferably, the C-terminus of the third structural unit is linked to the N-terminus of the fifth structural unit, and the C-terminus of the fifth structural unit is linked to the N-terminus of the fourth structural unit; and
8) when the first structural unit is a first Fab fragment, a first ScFv fragment, or a first VHH fragment, the fourth structural unit is selected from the second Fc fragment, and the N-terminus of each fragment in the fourth structural unit is linked to the C-terminus of the third structural unit.

12. The fusion protein according to claim 1, comprising:
a first peptide chain having an amino acid sequence as set forth in SEQ ID NO: 23, and a second peptide chain having an amino acid sequence as set forth in SEQ ID NO: 24;
a first peptide chain having an amino acid sequence as set forth in SEQ ID NO: 25, and a second peptide chain having an amino acid sequence as set forth in SEQ ID NO: 26;
a first peptide chain having an amino acid sequence as set forth in SEQ ID NO: 23, and a second peptide chain having an amino acid sequence as set forth in SEQ ID NO: 26;
a first peptide chain having an amino acid sequence as set forth in SEQ ID NO: 25, and a second peptide chain having an amino acid sequence as set forth in SEQ ID NO: 24;
a first peptide chain having an amino acid sequence as set forth in SEQ ID NO: 9, a second peptide chain having an amino acid sequence as set forth in SEQ ID NO: 27, a third peptide chain having an amino acid sequence as set forth in SEQ ID NO: 28, and a fourth peptide chain having an amino acid sequence as set forth in SEQ ID NO: 9;
a first peptide chain having an amino acid sequence as set forth in SEQ ID NO: 9, a second peptide chain having an amino acid sequence as set forth in SEQ ID NO: 29, and a third peptide chain having an amino acid sequence as set forth in SEQ ID NO: 30, and a fourth peptide chain having an amino acid sequence as set forth in SEQ ID NO: 9;
a first peptide chain having an amino acid sequence as set forth in SEQ ID NO: 9, a second peptide chain having an amino acid sequence as set forth in SEQ ID NO: 27, and a third peptide chain having an amino acid sequence as set forth in SEQ ID NO: 30, and a fourth peptide chain having an amino acid sequence as set forth in SEQ ID NO: 9;
a first peptide chain having an amino acid sequence as set forth in SEQ ID NO: 9, a second peptide chain having an amino acid sequence as set forth in SEQ ID NO: 29, and a third peptide chain having an amino acid sequence as set forth in SEQ ID NO: 28, and a fourth peptide chain having an amino acid sequence as set forth in SEQ ID NO: 9;
a first peptide chain having an amino acid sequence as set forth in SEQ ID NO: 33 and a second peptide chain having an amino acid sequence as set forth in SEQ ID NO: 34;
a first peptide chain having an amino acid sequence as set forth in SEQ ID NO: 35 and a second peptide chain having an amino acid sequence as set forth in SEQ ID NO: 36;
a first peptide chain having an amino acid sequence as set forth in SEQ ID NO: 33 and a second peptide chain having an amino acid sequence as set forth in SEQ ID NO: 36;
a first peptide chain having an amino acid sequence as set forth in SEQ ID NO: 35 and a second peptide chain having an amino acid sequence as set forth in SEQ ID NO: 34;
a first peptide chain having an amino acid sequence as set forth in SEQ ID NO: 33 and a second peptide chain having an amino acid sequence as set forth in SEQ ID NO: 38;
a first peptide chain having an amino acid sequence as set forth in SEQ ID NO: 35 and a second peptide chain having an amino acid sequence as set forth in SEQ ID NO: 39;
a first peptide chain having an amino acid sequence as set forth in SEQ ID NO: 33 and a second peptide chain having an amino acid sequence as set forth in SEQ ID NO: 39;
a first peptide chain having an amino acid sequence as set forth in SEQ ID NO: 35 and a second peptide chain having an amino acid sequence as set forth in SEQ ID NO: 38;
a first peptide chain having an amino acid sequence as set forth in SEQ ID NO: 9, a second peptide chain having an amino acid sequence as set forth in SEQ ID NO: 40, a third peptide chain as set forth in SEQ ID NO: 41, and a fourth peptide chain as set forth in SEQ ID NO: 9;
a first peptide chain having an amino acid sequence as set forth in SEQ ID NO: 9, and a second peptide chain having an amino acid sequence as set forth in SEQ ID NO: 42, a third peptide chain having an amino acid sequence as set forth in SEQ ID NO: 43, and a fourth peptide chain having an amino acid sequence as set forth in SEQ ID NO: 9;
a first peptide chain having an amino acid sequence as set forth in SEQ ID NO: 9, a second peptide chain having an amino acid sequence as set forth in SEQ ID NO: 40, a third peptide chain having an amino acid sequence as set forth in SEQ ID NO: 43, and a fourth peptide chain having an amino acid sequence as set forth in SEQ ID NO: 9;
a first peptide chain having an amino acid sequence as set forth in SEQ ID NO: 9, a second peptide chain having an amino acid sequence as set forth in SEQ ID NO: 41, a third peptide chain having an amino acid sequence as set forth in SEQ ID NO: 42, and a fourth peptide chain having an amino acid sequence as set forth in SEQ ID NO: 9;
a first peptide chain having an amino acid sequence as set forth in SEQ ID NO: 44, and a second peptide chain having an amino acid sequence as set forth in SEQ ID NO: 40;
a first peptide chain having an amino acid sequence as set forth in SEQ ID NO: 45, and a second peptide chain having an amino acid sequence as set forth in SEQ ID NO: 43;
a first peptide chain having an amino acid sequence as set forth in SEQ ID NO: 44, and a second peptide chain having an amino acid sequence as set forth in SEQ ID NO: 43;
a first peptide chain having an amino acid sequence as set forth in SEQ ID NO: 45 and a second peptide chain having an amino acid sequence as set forth in SEQ ID NO: 40;
a first peptide chain having an amino acid sequence as set forth in SEQ ID NO: 46 and a second peptide chain having an amino acid sequence as set forth in SEQ ID NO: 9;
a first peptide chain having an amino acid sequence as set forth in SEQ ID NO: 47 and a second peptide chain having an amino acid sequence as set forth in SEQ ID NO: 9;
a first peptide chain having an amino acid sequence as set forth in SEQ ID NO: 48 and a second peptide chain having an amino acid sequence as set forth in SEQ ID NO: 9;
a first peptide chain having an amino acid sequence as set forth in SEQ ID NO: 9, a second peptide chain having an amino acid sequence as set forth in SEQ ID NO: 49, a third peptide chain having an amino acid sequence as set forth in SEQ ID NO: 50, and a fourth peptide chain having an amino acid sequence as set forth in SEQ ID NO: 9;
a first peptide chain having an amino acid sequence as set forth in SEQ ID NO: 51 and a second peptide chain having an amino acid sequence as set forth in SEQ ID NO: 52;
a first peptide chain having an amino acid sequence as set forth in SEQ ID NO: 53 and a second peptide chain having an amino acid sequence as set forth in SEQ ID NO: 50;
a first peptide chain having an amino acid sequence as set forth in SEQ ID NO: 53 and a second peptide chain having an amino acid sequence as set forth in SEQ ID NO: 52;
a first peptide chain having an amino acid sequence as set forth in SEQ ID NO: 51 and a second peptide chain having an amino acid sequence as set forth in SEQ ID NO: 50;
a first peptide chain having an amino acid sequence as set forth in SEQ ID NO: 9, a second peptide chain having an amino acid sequence as set forth in SEQ ID NO: 54, a third peptide chain having an amino acid sequence as set forth in SEQ ID NO: 12, and a fourth peptide chain having an amino acid sequence as set forth in SEQ ID NO: 9;
a first peptide chain having an amino acid sequence as set forth in SEQ ID NO: 9, a second peptide chain having an amino acid sequence as set forth in SEQ ID NO: 55, a third peptide chain having an amino acid sequence as set forth in SEQ ID NO: 52, and a fourth peptide chain having an amino acid sequence as set forth in SEQ ID NO: 9;
a first peptide chain having an amino acid sequence as set forth in SEQ ID NO: 56 and a second peptide chain having an amino acid sequence as set forth in SEQ ID NO: 57;
a first peptide chain having an amino acid sequence as set forth in SEQ ID NO: 56 and a second peptide chain having an amino acid sequence as set forth in SEQ ID NO: 58;
a first peptide chain having an amino acid sequence as set forth in SEQ ID NO: 59 and a second peptide chain having an amino acid sequence as set forth in SEQ ID NO: 60;
a first peptide chain having an amino acid sequence as set forth in SEQ ID NO: 59 and a second peptide chain having an amino acid sequence as set forth in SEQ ID NO: 61;
a first peptide chain having an amino acid sequence as set forth in SEQ ID NO: 62 and a second peptide chain having an amino acid sequence as set forth in SEQ ID NO: 63;
a first peptide chain having an amino acid sequence as set forth in SEQ ID NO: 62 and a second peptide chain having an amino acid sequence as set forth in SEQ ID NO: 64;
a first peptide chain having an amino acid sequence as set forth in SEQ ID NO: 66 and a second peptide chain having an amino acid sequence as set forth in SEQ ID NO: 65;
a first peptide chain having an amino acid sequence as set forth in SEQ ID NO: 81 and a second peptide chain having an amino acid sequence as set forth in SEQ ID NO: 82;
a first peptide chain having an amino acid sequence as set forth in SEQ ID NO: 67 and a second peptide chain having an amino acid sequence as set forth in SEQ ID NO: 68;
a first peptide chain having an amino acid sequence as set forth in SEQ ID NO: 9, a second peptide chain having an amino acid sequence as set forth in SEQ ID NO: 69, a third peptide chain having an amino acid sequence as set forth in SEQ ID NO: 12, and a fourth peptide chain having an amino acid sequence as set forth in SEQ ID NO: 9; or
a first peptide chain having an amino acid sequence as set forth in SEQ ID NO: 74 and a second peptide chain having an amino acid sequence as set forth in SEQ ID NO: 73.

13. A nucleic acid molecule, an expression vector, and a recombinant cell, wherein:
the nucleic acid molecule encodes the fusion protein according to any one of claims 1 to 12;
the expression vector carries the nucleic acid molecule; and
the recombinant cell carries the nucleic acid or the expression vector, or the recombinant cell expresses the fusion protein according to any one of claims 1 to 12.

14. A fusion protein complex, comprising the fusion protein according to any one of claims 1 to 12.

15. An immunotherapy cell, expressing the fusion protein according to any one of claims 1 to 12.

16. A pharmaceutical composition, comprising:
the fusion protein according to any one of claims 1 to 12, the nucleic acid molecule, the expression vector, and the recombinant cell according to claim 11, the fusion protein complex according to claim 14, or the immunotherapy cell according to claim 15; and
optionally, a pharmaceutically acceptable excipient.

17. The fusion protein according to any one of claims 1 to 12, the fusion protein complex according to claim 14, the immunotherapy cell according to claim 15, or the pharmaceutical composition according to claim 16, for use in the treatment of cancer, an infectious disease, or an autoimmune disease.

18. Use of the fusion protein according to any one of claims 1 to 12, the fusion protein complex according to claim 14, the immunotherapy cell according to claim 15, or the pharmaceutical composition according to claim 16 in:
the treatment of cancer, an infectious disease, or an autoimmune disease; and/or
the manufacture of a medicament for treating cancer, an infectious disease, or an autoimmune disease.

19. A method for treating cancer, an infectious disease, or an autoimmune disease, the method comprising:
administering to a subject a pharmaceutically acceptable amount of the fusion protein according to any one of claims 1 to 12, the fusion protein complex according to claim 14, the immunotherapy cell according to claim 15, or the pharmaceutical composition according to claim 16.
